# EUROPEAN PATENT SPECIFICATION

(11) **EP 3 445 783 B1**
(45) Date of publication and mention of the grant of the patent: **04.11.2026**
(21) Application number: 17720927.7
(22) Date of filing: 18.04.2017
(51) Int. Cl.: C07K 16/28

(54) **AGONISTIC ANTIBODIES THAT BIND HUMAN CD40 AND USES THEREOF**
AGONISTISCHE ANTIKÖRPER ZUR BINDUNG VON HUMANEM CD40 UND VERWENDUNGEN DAVON
ANTICORPS AGONISTES SE LIANT AU CD40 HUMAIN ET LEURS UTILISATIONS

(30) Priority: 18.04.2016 US 201662324170 P
(43) Date of publication of application: 27.02.2019
(73) Proprietor: Celldex Therapeutics, Inc., Needham, MA 02494-2725 (US)
(72) Inventor: KELER, Tibor, Pipersville, PA 18947-1613 (US); GOLDSTEIN, Joel, Hopewell, NJ 08525 (US); VITALE, Laura, A., Doylestown, PA 18902 (US); HE, Lizhen, Allentown, PA 18104 (US); O'NEILL, Tom, Washington, NJ 07882 (US); CROCKER, Andrea, New Hope, PA 18938 (US); SUNDARAPANDIYAN, Karuna, Bridgewater, NJ 08807-3480 (US); THOMAS, Lawrence, J., Easton, MA 02375 (US); WIDGER, Jenifer, Alpha, NJ 08865 (US)
(74) Representative: J A Kemp LLP
(86) International application number: PCT/US2017/028162
(87) International publication number: WO 2017/184619

(56) References cited:
- WO-A1-2010/032061
- WO-A1-2014/070934
- WO-A2-2006/068953
- LAWRENCE J THOMAS ET AL: "CDX-1140, a Novel Agonist CD40 Antibody with Potent Anti-Lymphoma Activity", 2 December 2016 (2016-12-02), XP055706472, Retrieved from the Internet <URL:https://celldex.com/docs/CDX1140_2017SITC.pdf> [retrieved on 20200618]
- HE LI-ZHEN ET AL: "CDX-1140, a Novel Agonist CD40 Antibody with Potent Anti-Lymphoma Activity", BLOOD, vol. 128, no. 22, 2 December 2016 (2016-12-02), US, pages 1848 - 1848, XP093027943, ISSN: 0006-4971, Retrieved from the Internet <URL:https://ashpublications.org/blood/article/128/22/1848/100381/CDX1140-a-Novel-Agonist-CD40-Antibody-with-Potent> DOI: 10.1182/blood.V128.22.1848.1848
- R. H. VONDERHEIDE ET AL: "Agonistic CD40 Antibodies and Cancer Therapy", CLINICAL CANCER RESEARCH, vol. 19, no. 5, 1 March 2013 (2013-03-01), pages 1035 - 1043, XP055218372, ISSN: 1078-0432, DOI: 10.1158/1078-0432.CCR-12-2064
- SARA M MANGSBO ET AL: "ADC-1013, an agonistic CD40 antibody optimized for local immunotherapy of cancer", JOURNAL FOR IMMUNOTHERAPY OF CANCER, BIOMED CENTRAL LTD, LONDON, UK, vol. 1, no. Suppl 1, 7 November 2013 (2013-11-07), pages P42, XP021167252, ISSN: 2051-1426, DOI: 10.1186/2051-1426-1-S1-P42
- L. C. SANDIN ET AL: "Locally Delivered CD40 Agonist Antibody Accumulates in Secondary Lymphoid Organs and Eradicates Experimental Disseminated Bladder Cancer", CANCER IMMUNOLOGY RESEARCH, vol. 2, no. 1, 1 January 2014 (2014-01-01), pages 80 - 90, XP055218221, ISSN: 2326-6066, DOI: 10.1158/2326-6066.CIR-13-0067
- R. S. DRONCA ET AL: "Immunomodulatory Antibody Therapy of Cancer: The Closer, the Better", CLINICAL CANCER RESEARCH, vol. 21, no. 5, 28 October 2014 (2014-10-28), US, pages 944 - 946, XP055397745, ISSN: 1078-0432, DOI: 10.1158/1078-0432.CCR-14-2111

## Description

### Background of the Invention

Interactions between T cells and antigen-presenting cells involve a variety of accessory molecules that facilitate the generation of an immune response. One such molecule is CD40, a member of the tumor necrosis factor receptor (TNF-R) superfamily which binds to CD40L (Ranheim EA, et al., Blood. 1995 June 15;85(12):3556-65). CD40 is a transmembrane 43-48 kDa glycoprotein composed of 277 amino acid residues (Braesch-Andersen et al., 1989). CD40 is expressed by antigen-presenting cells (APC) and engagement of its natural ligand (CD40L) on T cells activates APC including dendritic cells and B cells (Khalil and Vonderhide (2007) Update Cancer Ther, 2(2): 61-65), thus enhancing immune responses. CD40 is also expressed on many tumor cells and its ligation in this setting mediates a direct cytotoxic effect, e.g., engagement of CD40 on tumor cells results in apoptosis *in vitro* and impaired tumor growth *in vivo* (Tai et al. (2004) Cancer Res, 64(8):2846-52).

Monoclonal antibodies against CD40 provide a variety of potential therapeutic purposes including the treatment of cancers. For example, agonistic CD40 antibodies have been shown to substitute for T cell help provided by CD4+ lymphocytes in murine models of T cell-mediated immunity, and in tumor-bearing hosts CD40 agonists trigger effective immune responses against tumor-associated antigens (Bennett et al. (1998) Nature, 393(6684):478-80). In addition, CD40 antibodies hold great promise for use in vaccines (Fransen et al. (2014) Vaccine 32:1654-1660). However, there are potential adverse effects associated with agents that strongly modulate the immune system (Sandin et al. (2014) Cancer Immunol Res, 2:80-90). Accordingly, there is a need for further insight into the specific properties and mechanisms that make CD40 antibodies therapeutically effective, as well as improved therapeutic antibodies against CD40 that can be used to treat and/or preventing diseases.

Vonderheide and Glennie Clin Cancer Res 2013; 19: 1035-1043 provides a review of preclinical and clinical data relating to agonistic CD40 antibodies in cancer therapy.

WO 2014/070934 describes anti-CD40 monoclonal antibodies and related compositions which may be used in a variety of therapeutic methods for the treatment of cancer.

### Summary of the Invention

The present invention provides isolated anti-CD40 antibodies having particular functional properties which can be linked with advantageous and desirable therapeutic effects. Specifically, agonistic anti-CD40 monoclonal antibodies capable of increasing an immune response to an antigen (*e.g.,* an antigen expressed on a cell) have been generated and characterized. As used herein, the term "antibody" refers to full-length antibodies and antigen binding portions thereof.

Any references in the description to methods of treatment refer to the compounds, pharmaceutical compositions and medicaments of the present invention for use in a method for treatment of the human (or animal) body by therapy (or for diagnosis).

The invention is defined by the claims, which in one embodiment, provides an isolated antibody which binds to human CD40, and comprises heavy chain CDR1, CDR2, and CDR3 sequences comprising SEQ ID NOs: 19, 21, 23, respectively, and light chain CDR1, CDR2, and CDR3 sequences comprising SEQ ID NOs: 25, 27, 29, respectively.

In one embodiment, the antibody comprises heavy and light chain variable regions having amino acid sequences at least 95% identical to SEQ ID Nos: 17 and 18.

In one embodiment, the antibody comprises heavy and light chain variable regions having amino acid sequences at least 98% identical to SEQ ID Nos: 17 and 18.

In one embodiment, the antibody comprises heavy and light chain variable regions having the amino acid sequences as set forth in SEQ ID Nos: 17 and 18, respectively.

In one embodiment, the antibody is a human antibody; and/or the antibody comprises a human constant region.

In one embodiment, the antibody is an antigen binding fragment, a Fab, Fab', (Fab')2, Fv, or scFv fragment.

In one embodiment, the invention provides a molecular conjugate comprising the antibody of the invention, linked to an antigen.

In one embodiment, the invention provides a bispecific molecule comprising the antibody of the invention linked to a second molecule having a binding specificity which is different from the antibody of the invention.

In one embodiment, the invention provides a composition comprising the antibody, molecular conjugate, or bispecific molecule of the invention and a carrier, optionally further comprising an adjuvant, and/or one or more other antibodies. The one or more other antibodies may bind to CTLA-4, PD-1, PD-L1, LAG-3, TIM-3, Galectin 9, CEACAM-1, BTLA, CD69, Galectin 1, TIGIT, CD113, GPR56, VISTA, B7-H3, B7-H4, 2B4, CD48, GARP, PD1H, LAIR1, TIM-1, TIM-4, B7-1, B7-2, CD28, 4-1BB (CD137), 4-1BBL, ICOS, ICOS-L, OX40, OX40L, CD70, CD27, DR3 or CD28H.

In one embodiment, the invention provides the antibody, molecular conjugate, bispecific antibody or composition of the invention for use in a method for inducing or enhancing an immune response against an antigen in a subject, optionally wherein the method further comprises administering the antigen simultaneously, separately or sequentially from the antibody, molecular conjugate, composition, or bispecific molecule.

In one embodiment, the invention provides an ex vivo method of inhibiting growth of CD40 expressing cells comprising contacting the cells with the antibody, composition or bispecific molecule of the invention, in an amount effective to inhibit growth of CD40 expressing cells.

In one embodiment, the invention provides the antibody, molecular conjugate, bispecific antibody or composition of the invention for use in a method of treating a subject by therapy.

In one embodiment, the invention provides the antibody, molecular conjugate, bispecific antibody or composition of the invention, for use in a method of treating a cancer selected from the group consisting of chronic lymphocytic leukemia, mantle cell lymphoma, primary central nervous system lymphoma, Burkitt's lymphoma and marginal zone B cell lymphoma. The method may further comprise administering one or more therapeutic agents to the subject, optionally wherein the therapeutic agent is another antibody, such as an anti-PD-1 antibody, an anti-PD-L1, and/or an anti-CTLA-4 antibody, and/or wherein the first and second antibodies are administered concurrently, or sequentially.

### Brief Description of the Drawings

**FIG. 1** provides the values for the equilibrium dissociation constants (K_{D}) and the kinetic association rate constants (kₒₙ) and dissociation rate constants (k_{off}) for antibodies 3C3, 3G5, 1B4, 3B6, and 6H6 as determined by bio-layer interferometry (BLI) using an Octet^{™} QK^{e} instrument (Pall ForteBio, Menlo Park, CA) according to the manufacturer's guidelines.
**FIG. 2** is a graph showing the binding of human CD40 antibodies (including 3C3, 3G5, 1B4, 3B6, and 6H6) to recombinant purified human CD40 coated microtiter plates using absorbance (OD₄₅₀) in an ELISA as a function of antibody concentration.
**FIG. 3** are graphs showing the binding as mean fluorescence intensity (MFI) by flow cytometry as a function of human CD40 antibody concentration (3C3, 3G5, 1B4, 3B6, and 6H6) to purified human PBMCs (left) and cynomolgus macaque PBMCs (right).
**FIGs. 4A** **and** **4B** **are** graphs showing the effect of human CD40 antibodies on the binding of soluble CD40 ligand (sCD40L) to CD40 protein by ELISA.
**FIG. 5** is a flow cytometric analysis of human CD40 antibodies (3C3, 3G5, 1B4, 3B6, and 6H6) binding to CD40 on Raji cells expressing human CD40 on their surface.
**FIG. 6** is a flow cytometric analysis of human CD40 antibodies (3C3, 3G5, 1B4, 3B6, and 6H6) binding to CD40 on Ramos cells expressing human CD40 on their surface.
**FIGs. 7A and 7B** are graphs showing the induction of CD95 on Ramos cells by human CD40 antibodies.
**FIGs. 8A and** **8B** are graphs showing dendritic cell (DC) activation by human CD40 antibodies (3C3 and 3G5) based on the change in level of expression of the following markers: CD54, HLA-DR, CD86, CD83, and % CD83 + cells as indicated.
**FIGs. 9A and 9B** are graphs showing the induction of IL-12p40 by human CD40 antibodies (3C3 and 3G5).
**FIGs. 10A** **and** **10B** are graphs showing B cell activation by human CD40 antibodies (3C3 and 3G5) based on the change in level of expression of the following markers: CD54, HLA-DR, CD23, % CD23 + cells, CD69, CD86, CD38, and CD71 as indicated.
**FIGs. 11A** **and** **11B** are graphs depicting NFkB activation by human CD40 antibodies using a luciferase reporter cell line expressing CD40.
**FIG. 12** are graphs showing the results of tumor growth and survival in a SCID mouse tumor model (Raji cells) following treatment with CD40 human antibody clones 3C3 and 3G5 via intraperitoneal administration, 0.3 mg per dose.
**FIG. 13** are graphs showing the results of tumor growth and survival in a SCID mouse tumor model (Ramos cells) following treatment with CD40 human antibody clones 3C3 and 3G5 via intraperitoneal administration, 0.3 mg per dose.
**FIGs. 14A** **and** **14B** are graphs showing T-cell proliferation of labeled PBMCs incubated with CD40 antibodies as indicated or the isotype control (IgG2).
**FIG. 15** is a graph showing binding to CD40 independent of Fc receptor interaction using CD40 antibodies 3C3 and 3G5.
**FIG. 16** is a graph showing NFκb activation using CD40 anitbodies 3C3 and 3G5.
**FIG. 17** is a graph showing CD95 induction on Ramos cells using CD40 anitbodies 3C3 and 3G5.
**FIG. 18** shows a schematic representation of an example of an anti-CD40/antigen fusion APC targeted vaccine construct.
**FIG. 19** is a graph showing the synergestic effect of CD40 antibody 3C3 with soluble CD40L on CD95 expression in Ramon cells.
**FIG. 20** is a schematic of soluble CD40 cDNA encoding the full length extracellular domain (ECD) spanning amino acid residues 1-173 with an N-terminal human kappa light chain and a C-terminal Flag tag.
**FIG. 21** shows an alignment of human CD40 ECD amino acid sequence with monkey CD40 ECD amino acid sequence (top) and mouse CD40 ECD amino acid sequence (bottom). Fragments generated are indicated.
**FIG. 22** provides graphs showing binding of CD40 antibody 3C3 to human CD40 ECD fragment A (amino acid residues 1-5; top) or fragment D (amino acid residues 33-36; bottom) with various point mutations or combinations thereof.
**FIGs. 23A-23C** are graphs showing levels of asparate aminotransferase (AST; **23A),** alanine aminotransferase (ALT; **23B)** and creatinine kinase **(23C)** measured in monkeys before and after treatment with CD40 antibodies 3C3 or 3G5 at indicated time points.
**FIG. 24** is a graph showing levels of IL-12 (pg/mL) measured in blood from monkeys treated with CD40 antibodies 3C3 or 3G5 at indicated time points.
**FIGs. 25A-25C** are graphs showing amounts of white blood cells **(25A),** neutrophils **(25B)** and lymphocytes **(25C)** measured in monkeys before and after treatment with CD40 antibodies 3C3 or 3G5 at indicated time points.
**FIG. 26** is a graph showing the percentage change from baseline of amount of B cells in monkeys treated with CD40 antibodies 3C3 or 3G5 over time (days).
**FIG. 27** provides graphs showing HLA-DR expression on B cells relative to baseline following 2 mg (left) or 0.2 mg (right) of CD40 antibody 3C3 (square), 3G5 (diamonds) or saline (circles).
**FIG. 28** provides a graph showing B-cell proliferation when cells were cultured in the presence of either the anti-CD40 mAb 3C3.
**FIGs. 29** **and** **30** provide graphs showing synergistic effects of the combination of the anti-CD40 mAb 3C3 and CD40L in B-cells.
**FIG. 31** provides a table showing cytokine responses in whole blood when this was incubated with the anti-CD40 mAb 3C3.

### Detailed Description

Anti-CD40 antibodies may exhibit particular functional properties correlating with significant therapeutic benefits involving upregulation of immune function (*e.g.* T cell mediated immune responses as in vaccine therapies, NK activation in cancer therapies), inhibition of cell growth (*e.g.,* in cancer therapy), and/or enhanced processing and presentation of an antigen by APCs (*e.g.,* in vaccine therapy). These functional features include, for example, an increased immune response to an antigen independent of Fc receptor binding, and/or without induction of antibody-dependent cellular cytotoxicity (ADCC) or complement dependent cellular cytotoxicity (CDC). Additional functional features include, for example, (1) inhibition of (*e.g.,* complete or partial blocking) binding of CD40L (CD154) to CD40 expressing cells by at least 50%, at least 60% or at least 70% (2) blockage of binding of CD40L to human CD40 independent of Fc receptor binding, (3) induction of cellular apoptosis (e.g., as measured by an increase in the expression of CD95), (4) increased T-cell stimulatory activity (e.g., as measured by an increase in the expression of IL-12p40), and / or (5) increased B-cell activation (e.g., as measured by an increase in the expression of at least one cell-surface marker selected from the group consisting of HLA-DR V450, CD54 PE, CD86 APC, and CD83 BV510, CD19 V500, CD54 PE, HLA-DR V450, CD23 PerCP-Cy5.5, CD69 APC, CD86 APC, CD38 PerCP-Cy5.5 and CD71 PE).

In order that the present invention may be more readily understood, certain terms are first defined. Additional definitions are set forth throughout the detailed description.

The term "CD40" (also referred to as "CD40 molecule," "Bp50," "CDW40," "TNFRSF5," "p50," "B cell surface antigen CD40," "B cell-associated molecule," "CD40 antigen," "TNF receptor superfamily member 5," "CD40 type II isoform," "CD40L receptor," "nerve growth factor receptor-related B-lymphocyte activation molecule," or "tumor necrosis factor receptor superfamily member 5") refers to a receptor that is a member of the TNF-receptor superfamily, which binds to ligand CD40L (also referred to as CD154). CD40 is mediates a broad variety of immune and inflammatory responses including T cell-dependent immunoglobulin class switching, and memory B cell development. The term "CD40" includes any variants or isoforms of CD40 which are naturally expressed by cells (*e.g.,* human CD40 deposited with GENBANK^{®} having accession no. P25942). Accordingly, antibodies of the invention may cross-react with CD40 from species other than human. Alternatively, the antibodies may be specific for human CD40 and may not exhibit any cross-reactivity with other species. CD40 or any variants and isoforms thereof, may either be isolated from cells or tissues which naturally express them or be recombinantly produced using well-known techniques in the art and/or those described herein. Preferably the antibodies are targeted to hCD40 which has a normal glycosylation pattern.
Genbank^{®} (Accession No. P25942) reports the amino acid sequence of human CD40 as follows (SEQ ID NO:1):

MVRLPLQCVL WGCLLTAVHP EPPTACREKQ YLINSQCCSL CQPGQKLVSD
CTEFTETECL PCGESEFLDT WNRETHCHQH KYCDPNLGLR VQQKGTSETD
TICTCEEGWH CTSEACESCV LHRSCSPGFG VKQIATGVSD TICEPCPVGF
FSNVSSAFEK CHPWTSCETK DLVVQQAGTN  KTDVVCGPQD RLRALVVIPI
IFGILFAILL VLVFIKKVAK KPTNKAPHPK QEPQEINFPD DLPGSNTAAP
VQETLHGCQP VTQEDGKESR ISVQERQ

The term "CD40L" (also referred to as "CD40 ligand," "CD407L," or "CD154") refers to the ligand for CD40 (see, for example, Schönbeck and Libby (2001) Cell Mol Life Sci, 58(1):4-43). CD40L is primarily expressed on activated T cells and is a member of the TNF superfamily of molecules. It binds to CD40 on antigen-presenting cells (APC), which leads to many effects depending on the target cell type (Parham, Peter (2004). The Immune System (2nd ed.). Garland Science. Pp. 169-173).
Genbank^{®} (Accession No. NP_000065) reports the amino acid sequence of human CD40L as follows (SEQ ID NO: 2):

MIETYNQTSP RSAATGLPIS MKIFMYLLTV FLITQMIGSA LFAVYLHRRL
DKIEDERNLH EDFVFMKTIQ RCNTGERSLS LLNCEEIKSQ FEGFVKDIML
NKEETKKENS FEMQKGDQNP QIAAHVISEA SSKTTSVLQW AEKGYYTMSN
NLVTLENGKQ LTVKRQGLYY IYAQVTFCSN REASSQAPFI ASLCLKSPGR
FERILLRAAN THSSAKPCGQ QSIHLGGVFE LQPGASVFVN VTDPSQVSHG
TGFTSFGLLK

The term "antibody" as referred to herein includes whole antibodies and any antigen binding fragment (*i.e.,* "antigen-binding portion") or single chain thereof. An "antibody" refers, in one preferred embodiment, to a glycoprotein comprising at least two heavy (H) chains and two light (L) chains inter-connected by disulfide bonds, or an antigen binding portion thereof. Each heavy chain is comprised of a heavy chain variable region (abbreviated herein as V_{H}) and a heavy chain constant region. The heavy chain constant region is comprised of three domains, CH1, CH2 and CH3. Each light chain is comprised of a light chain variable region (abbreviated herein as V_{L}) and a light chain constant region. The light chain constant region is comprised of one domain, CL. The V_{H} and V_{L} regions can be further subdivided into regions of hypervariability, termed complementarity determining regions (CDR), interspersed with regions that are more conserved, termed framework regions (FR). Each V_{H} and V_{L} is composed of three CDRs and four FRs, arranged from amino-terminus to carboxy-terminus in the following order: FR1, CDR1, FR2, CDR2, FR3, CDR3, FR4. The variable regions of the heavy and light chains contain a binding domain that interacts with an antigen. The constant regions of the antibodies may mediate the binding of the immunoglobulin to host tissues or factors, including various cells of the immune system *(e.g.,* effector cells) and the first component (Clq) of the classical complement system.

The term "antigen-binding portion" of an antibody (or simply "antibody portion"), as used herein, refers to one or more fragments of an antibody that retain the ability to specifically bind to an antigen (*e.g.*, human CD40). Such "fragments" are, for example between about 8 and about 1500 amino acids in length, suitably between about 8 and about 745 amino acids in length, suitably about 8 to about 300, for example about 8 to about 200 amino acids, or about 10 to about 50 or 100 amino acids in length. It has been shown that the antigen-binding function of an antibody can be performed by fragments of a full-length antibody. Examples of binding fragments encompassed within the term "antigen-binding portion" of an antibody include (i) a Fab fragment, a monovalent fragment consisting of the V_{L}, V_{H}, CL and CH1 domains; (ii) a F(ab')₂ fragment, a bivalent fragment comprising two Fab fragments linked by a disulfide bridge at the hinge region; (iii) a Fd fragment consisting of the V_{H} and CH1 domains; (iv) a Fv fragment consisting of the V_{L} and V_{H} domains of a single arm of an antibody, (v) a dAb fragment (Ward et al., (1989) Nature 341:544-546), which consists of a V_{H} domain; and (vi) an isolated complementarity determining region (CDR) or (vii) a combination of two or more isolated CDRs which may optionally be joined by a synthetic linker. Furthermore, although the two domains of the Fv fragment, V_{L} and V_{H}, are coded for by separate genes, they can be joined, using recombinant methods, by a synthetic linker that enables them to be made as a single protein chain in which the V_{L} and V_{H} regions pair to form monovalent molecules (known as single chain Fv (scFv); see *e.g.,* Bird et al. (1988) Science 242:423-426; and Huston et al. (1988) Proc. Natl. Acad. Sci. USA 85:5879-5883). Such single chain antibodies are also intended to be encompassed within the term "antigen-binding portion" of an antibody. These antibody fragments are obtained using conventional techniques known to those with skill in the art, and the fragments are screened for utility in the same manner as are intact antibodies. Antigen-binding portions can be produced by recombinant DNA techniques, or by enzymatic or chemical cleavage of intact immunoglobulins.

A "bispecific" or "bifunctional antibody" is an artificial hybrid antibody having two different heavy/light chain pairs and two different binding sites. Bispecific antibodies can be produced by a variety of methods including fusion of hybridomas or linking of Fab' fragments. See, *e.g.,* Songsivilai & Lachmann, Clin. Exp. Immunol. 79:315-321 (1990); Kostelny et al., J. Immunol. 148, 1547-1553 (1992).

The term "monoclonal antibody," as used herein, refers to an antibody which displays a single binding specificity and affinity for a particular epitope. Accordingly, the term "human monoclonal antibody" refers to an antibody which displays a single binding specificity and which has variable and optional constant regions derived from human germline immunoglobulin sequences. In one embodiment, human monoclonal antibodies are produced by a hybridoma which includes a B cell obtained from a transgenic non-human animal, *e.g.,* a transgenic mouse, having a genome comprising a human heavy chain transgene and a light chain transgene fused to an immortalized cell.

The term "recombinant human antibody," as used herein, includes all human antibodies that are prepared, expressed, created or isolated by recombinant means, such as (a) antibodies isolated from an animal (*e.g.,* a mouse) that is transgenic or transchromosomal for human immunoglobulin genes or a hybridoma prepared therefrom, (b) antibodies isolated from a host cell transformed to express the antibody, *e.g.*, from a transfectoma, (c) antibodies isolated from a recombinant, combinatorial human antibody library, and (d) antibodies prepared, expressed, created or isolated by any other means that involve splicing of human immunoglobulin gene sequences to other DNA sequences. Such recombinant human antibodies comprise variable and constant regions that utilize particular human germline immunoglobulin sequences are encoded by the germline genes, but include subsequent rearrangements and mutations which occur, for example, during antibody maturation. As known in the art (see, *e.g.,* Lonberg (2005) Nature Biotech. 23(9):1117-1125), the variable region contains the antigen binding domain, which is encoded by various genes that rearrange to form an antibody specific for a foreign antigen. In addition to rearrangement, the variable region can be further modified by multiple single amino acid changes (referred to as somatic mutation or hypermutation) to increase the affinity of the antibody to the foreign antigen. The constant region will change in further response to an antigen *(i.e.,* isotype switch). Therefore, the rearranged and somatically mutated nucleic acid molecules that encode the light chain and heavy chain immunoglobulin polypeptides in response to an antigen may not have sequence identity with the original nucleic acid molecules, but instead will be substantially identical or similar (*i.e.,* have at least 80% identity).

The term "human antibody" includes antibodies having variable and constant regions (if present) of human germline immunoglobulin sequences. Human antibodies of the invention can include amino acid residues not encoded by human germline immunoglobulin sequences (*e.g.,* mutations introduced by random or site-specific mutagenesis *in vitro* or by somatic mutation *in vivo) (see,* Lonberg, N. et al. (1994) Nature 368(6474): 856-859); Lonberg, N. (1994) Handbook of Experimental Pharmacology 113:49-101; Lonberg, N. and Huszar, D. (1995) Intern. Rev. Immunol. Vol. 13: 65-93, and Harding, F. and Lonberg, N. (1995) Ann. N.Y. Acad. Sci 764:536-546). However, the term "human antibody" does not include antibodies in which CDR sequences derived from the germline of another mammalian species, such as a mouse, have been grafted onto human framework sequences (*i.e.,* humanized antibodies).

As used herein, a "heterologous antibody" is defined in relation to the transgenic non-human organism producing such an antibody. This term refers to an antibody having an amino acid sequence or an encoding nucleic acid sequence corresponding to that found in an organism not consisting of the transgenic non-human animal, and generally from a species other than that of the transgenic non-human animal.

An "isolated antibody," as used herein, is intended to refer to an antibody which is substantially free of other antibodies having different antigenic specificities (*e.g.*, an isolated antibody that specifically binds to human CD40 is substantially free of antibodies that specifically bind antigens other than human CD40). An isolated antibody that specifically binds to an epitope of may, however, have cross-reactivity to other CD40 proteins from different species. However, the antibody preferably always binds to human CD40. In addition, an isolated antibody is typically substantially free of other cellular material and/or chemicals. A combination of "isolated" antibodies having different CD40 specificities may be combined in a well defined composition.

The term "epitope" or "antigenic determinant" refers to a site on an antigen to which an immunoglobulin or antibody specifically binds. Epitopes can be formed both from contiguous amino acids or noncontiguous amino acids juxtaposed by tertiary folding of a protein. Epitopes formed from contiguous amino acids are typically retained on exposure to denaturing solvents, whereas epitopes formed by tertiary folding are typically lost on treatment with denaturing solvents. An epitope typically includes at least 3, 4, 5, 6, 7, 8, 9, 10, 11, 12, 13, 14 or 15 amino acids in a unique spatial conformation. Methods for determining what epitopes are bound by a given antibody (*i.e.,* epitope mapping) are well known in the art and include, for example, immunoblotting and immunoprecipitation assays, wherein overlapping or contiguous peptides from CD40 are tested for reactivity with the given anti-CD40 antibody. Methods of determining spatial conformation of epitopes include techniques in the art and those described herein, for example, x-ray crystallography and 2-dimensional nuclear magnetic resonance (see, *e.g.,* Epitope Mapping Protocols in Methods in Molecular Biology, Vol. 66, G. E. Morris, Ed. (1996)).

Accordingly, antibodies that bind to the same epitope, or an epitope on CD40 which comprises all or a portion of an epitope recognized by the particular antibodies described herein (*e.g.,* the same or an overlapping region or a region between or spanning the region) also are provided by the invention. Antibodies that bind to the same epitope, or an epitope which comprises all or a portion of an epitope recognized by particular antibody can be identified using routine techniques. Such techniques include, for example, epitope mapping methods, such as, x-ray analyses of crystals of antigen: antibody complexes which provides atomic resolution of the epitope. Other methods monitor the binding of the antibody to antigen fragments or mutated variations of the antigen where loss of binding due to a modification of an amino acid residue within the antigen sequence is often considered an indication of an epitope component. In addition, computational combinatorial methods for epitope mapping can also be used. These methods rely on the ability of the antibody of interest to affinity isolate specific short peptides from combinatorial phage display peptide libraries. The peptides are then regarded as leads for the definition of the epitope corresponding to the antibody used to screen the peptide library. For epitope mapping, computational algorithms have also been developed which have been shown to map conformational discontinuous epitopes.

Also provided are antibodies that compete for binding to human CD40 with the antibodies described herein. Antibodies that compete for binding can be identified using routine techniques. Such techniques include, for example, an immunoassay, which shows the ability of one antibody to block the binding of another antibody to a target antigen, *i.e., a* competitive binding assay. Competitive binding is determined in an assay in which the immunoglobulin under test inhibits specific binding of a reference antibody to a common antigen, such as CD40. Numerous types of competitive binding assays are known, for example: solid phase direct or indirect radioimmunoassay (RIA), solid phase direct or indirect enzyme immunoassay (EIA), sandwich competition assay (see Stahli et al., Methods in Enzymology 9:242 (1983)); solid phase direct biotin-avidin EIA (see Kirkland et al., J. Immunol. 137:3614 (1986)); solid phase direct labeled assay, solid phase direct labeled sandwich assay (see Harlow and Lane, Antibodies: A Laboratory Manual, Cold Spring Harbor Press (1988)); solid phase direct label RIA using I-125 label (see Morel et al., Mol. Immunol. 25(1):7 (1988)); solid phase direct biotin-avidin EIA (Cheung et al., Virology 176:546 (1990)); and direct labeled RIA. (Moldenhauer et al., Scand. J. Immunol. 32:77 (1990)). Typically, such an assay involves the use of purified antigen bound to a solid surface or cells bearing either of these, an unlabeled test immunoglobulin and a labeled reference immunoglobulin. Competitive inhibition is measured by determining the amount of label bound to the solid surface or cells in the presence of the test immunoglobulin. Usually the test immunoglobulin is present in excess. Usually, when a competing antibody is present in excess, it will inhibit specific binding of a reference antibody to a common antigen by at least 50-55%, 55-60%, 60-65%, 65-70% 70-75% or more.

As used herein, the terms "specific binding," "selective binding," "selectively binds," and "specifically binds," refer to antibody binding to an epitope on a predetermined antigen. Typically, the antibody binds with an equilibrium dissociation constant (K_{D}) of approximately less than 10⁻⁷ M, such as approximately less than 10 ⁻⁸ M, 10⁻⁹ M or 10⁻¹⁰ M or even lower when determined by bio-layer interferometry (BLI) using an Octet^{™} QK^{e} instrument or by surface plasmon resonance (SPR) technology in a BIACORE 2000 instrument using recombinant human CD40 as the analyte and the antibody as the ligand and binds to the predetermined antigen with an affinity that is at least two-fold greater than its affinity for binding to a non-specific antigen (*e.g.*, BSA, casein) other than the predetermined antigen or a closely-related antigen. The phrases "an antibody recognizing an antigen" and "an antibody specific for an antigen" are used interchangeably herein with the term "an antibody which binds specifically to an antigen."

Antibodies that bind to human CD40 may be capable of increasing an immune response independent of Fc receptor binding. For example, such antibodies exhibit potent agonistic features without cross-linking with an Fc receptor, such as FcγR. These agonistic features include, for example, an increase in T-cell activity and/or an increase in B cell activation as measured, e.g., by an increase in the expression of cell surface markers.

The term "K_{D}," as used herein, is intended to refer to the dissociation equilibrium constant of a particular antibody-antigen interaction. Typically, the human antibodies of the invention bind to CD40 with a dissociation equilibrium constant (K_{D}) of approximately 10⁻⁸ M or less, such as less than 10⁻⁹ M, 10⁻¹⁰ M, 10⁻¹¹ M, or 10⁻¹² M, or even lower when determined by bio-layer interferometry (BLI) using an Octet^{™} QK^{e} instrument or by surface plasmon resonance (SPR) technology in a BIACORE 2000 instrument using recombinant human CD40 as the analyte and the antibody as the ligand.

The term "kd" as used herein, is intended to refer to the off rate constant for the dissociation of an antibody from the antibody/antigen complex.

The term "ka" as used herein, is intended to refer to the on rate constant for the association of an antibody with the antigen.

The term "EC50," as used herein, refers to the concentration of an antibody or an antigen-binding portion thereof, which induces a response, either in an *in vitro* or an *in vivo* assay, which is 50% of the maximal response, *i.e.,* halfway between the maximal response and the baseline.

As used herein, "isotype" refers to the antibody class (*e.g.,* IgM or IgGl) that is encoded by heavy chain constant region genes. In one embodiment, a human monoclonal antibody of the invention is of the IgG1 isotype. In another embodiment, a human monoclonal antibody of the invention is of the IgG2 isotype.

The term "binds to immobilized CD40," refers to the ability of a human antibody of the invention to bind to CD40, for example, expressed on the surface of a cell or which is attached to a solid support.

The term "cross-reacts," as used herein, refers to the ability of an antibody of the invention to bind to CD40 from a different species. For example, an antibody of the present invention which binds human CD40 may also bind another species of CD40. As used herein, cross-reactivity is measured by detecting a specific reactivity with purified antigen in binding assays (*e.g.,* SPR, ELISA) or binding to, or otherwise functionally interacting with, cells physiologically expressing CD40. Methods for determining cross-reactivity include standard binding assays as described herein, for example, by bio-layer interferometry (BLI) using an Octet^{™} QK^{e} instrument or by Biacore^{™} surface plasmon resonance (SPR) analysis using a Biacore^{™} 2000 SPR instrument (Biacore AB, Uppsala, Sweden), or flow cytometric techniques.

As used herein, "isotype switching" refers to the phenomenon by which the class, or isotype, of an antibody changes from one Ig class to one of the other Ig classes.

As used herein, "nonswitched isotype" refers to the isotypic class of heavy chain that is produced when no isotype switching has taken place; the CH gene encoding the nonswitched isotype is typically the first CH gene immediately downstream from the functionally rearranged VDJ gene. Isotype switching has been classified as classical or non-classical isotype switching. Classical isotype switching occurs by recombination events which involve at least one switch sequence region in the transgene. Non-classical isotype switching may occur by, for example, homologous recombination between human σ_{µ} and human ∑_{µ} (δ-associated deletion). Alternative non-classical switching mechanisms, such as intertransgene and/or interchromosomal recombination, among others, may occur and effectuate isotype switching.

As used herein, the term "switch sequence" refers to those DNA sequences responsible for switch recombination. A "switch donor" sequence, typically a µ switch region, will be 5' (*i.e.,* upstream) of the construct region to be deleted during the switch recombination. The "switch acceptor" region will be between the construct region to be deleted and the replacement constant region (*e.g.,* γ, ε, etc.). As there is no specific site where recombination always occurs, the final gene sequence will typically not be predictable from the construct.

As used herein, "glycosylation pattern" is defined as the pattern of carbohydrate units that are covalently attached to a protein, more specifically to an immunoglobulin protein. A glycosylation pattern of a heterologous antibody can be characterized as being substantially similar to glycosylation patterns which occur naturally on antibodies produced by the species of the nonhuman transgenic animal, when one of ordinary skill in the art would recognize the glycosylation pattern of the heterologous antibody as being more similar to said pattern of glycosylation in the species of the nonhuman transgenic animal than to the species from which the CH genes of the transgene were derived.

The term "naturally-occurring" as used herein as applied to an object refers to the fact that an object can be found in nature. For example, a polypeptide or polynucleotide sequence that is present in an organism (including viruses) that can be isolated from a source in nature and which has not been intentionally modified by man in the laboratory is naturally-occurring.

The term "rearranged" as used herein refers to a configuration of a heavy chain or light chain immunoglobulin locus wherein a V segment is positioned immediately adjacent to a D-J or J segment in a conformation encoding essentially a complete V_{H} or V_{L} domain, respectively. A rearranged immunoglobulin gene locus can be identified by comparison to germline DNA; a rearranged locus will have at least one recombined heptamer/nonamer homology element.

The term "unrearranged" or "germline configuration" as used herein in reference to a V segment refers to the configuration wherein the V segment is not recombined so as to be immediately adjacent to a D or J segment.

The term "nucleic acid molecule," as used herein, is intended to include DNA molecules and RNA molecules. A nucleic acid molecule may be single-stranded or double-stranded, but preferably is double-stranded DNA.

The term "isolated nucleic acid molecule," as used herein in reference to nucleic acids encoding antibodies or antibody portions (*e.g.,* V_{H}, V_{L}, CDR3) that bind to CD40, is intended to refer to a nucleic acid molecule in which the nucleotide sequences encoding the antibody or antibody portion are free of other nucleotide sequences encoding antibodies or antibody portions that bind antigens other than CD40, which other sequences may naturally flank the nucleic acid in human genomic DNA.

Conservative sequence modifications of the sequences set forth in SEQ ID Nos: 3-132, *i.e.,* nucleotide and amino acid sequence modifications, are modifications which do not abrogate the binding of the antibody encoded by the nucleotide sequence or containing the amino acid sequence, to the antigen. Such conservative sequence modifications include conservative nucleotide and amino acid substitutions, as well as, nucleotide and amino acid additions and deletions. For example, modifications can be introduced into SEQ ID Nos: 3-148 by standard techniques known in the art, such as site-directed mutagenesis and PCR-mediated mutagenesis. Conservative amino acid substitutions include ones in which the amino acid residue is replaced with an amino acid residue having a similar side chain. Families of amino acid residues having similar side chains have been defined in the art. These families include amino acids with basic side chains (*e.g.,* lysine, arginine, histidine), acidic side chains (*e.g.,* aspartic acid, glutamic acid), uncharged polar side chains (*e.g.,* glycine, asparagine, glutamine, serine, threonine, tyrosine, cysteine, tryptophan), nonpolar side chains (*e.g.,* alanine, valine, leucine, isoleucine, proline, phenylalanine, methionine), beta-branched side chains (*e.g.,* threonine, valine, isoleucine) and aromatic side chains (*e.g.,* tyrosine, phenylalanine, tryptophan, histidine). Thus, a predicted nonessential amino acid residue in a human anti-CD40 antibody is preferably replaced with another amino acid residue from the same side chain family. Methods of identifying nucleotide and amino acid conservative substitutions which do not eliminate antigen binding are well-known in the art (see, *e.g.,* Brummell et al., Biochem. 32:1180-1187 (1993); Kobayashi et al. Protein Eng. 12(10):879-884 (1999); and Burks et al. Proc. Natl. Acad. Sci. USA 94:412-417 (1997)).

Conservative substitutions maybe made, for example, according to the Table below. For example, amino acids in the same block in the second column and preferably in the same line in the third column may be substituted for each other.

| | | |
|---|---|---|
| Aliphatic | Non-Polar | GAP |
| | | ILV |
| | Polar-uncharged | CSTM |
| | | NQ |
| | Polar-charged | DE |
| | | KR |
| Aromatic | | HFWY |

Alternatively, mutations can be introduced randomly along all or part of an anti-CD40 antibody coding sequence, such as by saturation mutagenesis, and the resulting modified anti-CD40 antibodies can be screened for binding activity.

For nucleic acids, the term "substantial homology" indicates that two nucleic acids, or designated sequences thereof, when optimally aligned and compared, are identical, with appropriate nucleotide insertions or deletions, in at least about 80% of the nucleotides, usually at least about 90% to 95%, and more preferably at least about 98% to 99.5% of the nucleotides. Alternatively, substantial homology exists when the segments will hybridize under selective hybridization conditions, to the complement of the strand.

The percent identity between two sequences is a function of the number of identical positions shared by the sequences (*i.e.,* % homology = # of identical positions/total # of positions x 100), taking into account the number of gaps, and the length of each gap, which need to be introduced for optimal alignment of the two sequences. The comparison of sequences and determination of percent identity between two sequences can be accomplished using a mathematical algorithm, as described in the non-limiting examples below.

The percent identity between two nucleotide sequences can be determined using the GAP program in the GCG software package (available at http://www.gcg.com), using a NWSgapdna.CMP matrix and a gap weight of 40, 50, 60, 70, or 80 and a length weight of 1, 2, 3, 4, 5, or 6. The percent identity between two nucleotide or amino acid sequences can also be determined using the algorithm of E. Meyers and W. Miller (CABIOS, 4:11-17 (1989)) which has been incorporated into the ALIGN program (version 2.0), using a PAM120 weight residue table, a gap length penalty of 12 and a gap penalty of 4. In addition, the percent identity between two amino acid sequences can be determined using the Needleman and Wunsch (J. Mol. Biol. (48):444-453 (1970)) algorithm which has been incorporated into the GAP program in the GCG software package (available at http://www.gcg.com), using either a Blossum 62 matrix or a PAM250 matrix, and a gap weight of 16, 14, 12, 10, 8, 6, or 4 and a length weight of 1, 2, 3, 4, 5, or 6.

Nucleic acid and protein sequences can further be used as a "query sequence" to perform a search against public databases to, for example, identify related sequences. Such searches can be performed using the NBLAST and XBLAST programs (version 2.0) of Altschul, et al. (1990) J. Mol. Biol. 215:403-10. BLAST nucleotide searches can be performed with the NBLAST program, score = 100, wordlength = 12 to obtain nucleotide sequences homologous to the nucleic acid molecules of the invention. BLAST protein searches can be performed with the XBLAST program, score = 50, wordlength = 3 to obtain amino acid sequences homologous to the protein molecules of the invention. To obtain gapped alignments for comparison purposes, Gapped BLAST can be utilized as described in Altschul et al., (1997) Nucleic Acids Res. 25(17):3389-3402. When utilizing BLAST and Gapped BLAST programs, the default parameters of the respective programs (*e.g.,* XBLAST and NBLAST) can be used. See http://www.ncbi.nlm.nih.gov.

The nucleic acids may be present in whole cells, in a cell lysate, or in a partially purified or substantially pure form. A nucleic acid is "isolated" or "rendered substantially pure" when purified away from other cellular components or other contaminants, *e.g.,* other cellular nucleic acids or proteins, by standard techniques, including alkaline/SDS treatment, CsCl banding, column chromatography, agarose gel electrophoresis and others well known in the art. *See,* F. Ausubel, et al., ed. Current Protocols in Molecular Biology, Greene Publishing and Wiley Interscience, New York (1987).

The nucleic acid compositions, while often in a native sequence (except for modified restriction sites and the like), from either cDNA, genomic or mixtures thereof may be mutated, in accordance with standard techniques to provide gene sequences. For coding sequences, these mutations, may affect amino acid sequence as desired. In particular, DNA sequences substantially homologous to or derived from native V, D, J, constant, switches and other such sequences described herein are contemplated (where "derived" indicates that a sequence is identical or modified from another sequence).

A nucleic acid is "operably linked" when it is placed into a functional relationship with another nucleic acid sequence. For instance, a promoter or enhancer is operably linked to a coding sequence if it affects the transcription of the sequence. With respect to transcription regulatory sequences, operably linked means that the DNA sequences being linked are contiguous and, where necessary to join two protein coding regions, contiguous and in reading frame. For switch sequences, operably linked indicates that the sequences are capable of effecting switch recombination.

The term "vector," as used herein, is intended to refer to a nucleic acid molecule capable of transporting another nucleic acid to which it has been linked. One type of vector is a "plasmid," which refers to a circular double stranded DNA loop into which additional DNA segments may be ligated. Another type of vector is a viral vector, wherein additional DNA segments may be ligated into the viral genome. Certain vectors are capable of autonomous replication in a host cell into which they are introduced (*e.g.,* bacterial vectors having a bacterial origin of replication and episomal mammalian vectors). Other vectors (*e.g.,* non-episomal mammalian vectors) can be integrated into the genome of a host cell upon introduction into the host cell, and thereby are replicated along with the host genome. Moreover, certain vectors are capable of directing the expression of genes to which they are operatively linked. Such vectors are referred to herein as "recombinant expression vectors"(or simply, "expression vectors") In general, expression vectors of utility in recombinant DNA techniques are often in the form of plasmids. In the present specification, "plasmid" and "vector" may be used interchangeably as the plasmid is the most commonly used form of vector. However, other forms of expression vectors, such as viral vectors (*e.g.,* replication defective retroviruses, adenoviruses and adeno-associated viruses), serve equivalent functions.

The term "recombinant host cell" (or simply "host cell"), as used herein, is intended to refer to a cell into which a recombinant expression vector has been introduced. It should be understood that such terms are intended to refer not only to the particular subject cell but to the progeny of such a cell. Because certain modifications may occur in succeeding generations due to either mutation or environmental influences, such progeny may not, in fact, be identical to the parent cell, but are still included within the scope of the term "host cell" as used herein.

As used herein, the term "antigen" refers to any natural or synthetic immunogenic substance, such as a protein, peptide, or hapten. Suitable antigens for use in the present invention (*e.g.,* in a vaccine in combination with an anti-CD40 antibody of the invention) include, for example, infectious disease antigens and tumor antigens, against which protective or therapeutic immune responses are desired, *e.g.,* antigens expressed by a tumor cell or a pathogenic organism or infectious disease antigens. For example, suitable antigens include tumor-associated antigens for the prevention or treatment of cancers. Examples of tumor-associated antigens include, but are not limited to, sequences comprising all or part of the sequences of βhCG, gp100 or Pmel17, HER2/neu, WT1, mesothelin, CEA, gp100, MART1, TRP-2, melan-A, NY-ESO-1, NY-BR-1, NY-CO-58, MN (gp250), idiotype, MAGE-1, MAGE-3, MAGE-A3, Tyrosinase, Telomerase, SSX2 and MUC-1 antigens, and germ cell derived tumor antigens. Tumor associated antigens also include the blood group antigens, for example, Le^{a}, Le^{b}, LeX, LeY, H-2, B-1, B-2 antigens. Alternatively, more than one antigen can be included within antigen-antibody constructs. For example, a MAGE antigen can be combined with other antigens such as melanin A, tyrosinase, and gp100 along with adjuvants such as GM-CSF or IL-12, and linked to an anti-APC antibody.

Other suitable antigens include viral antigens for the prevention or treatment of viral diseases. Examples of viral antigens include, but are not limited to, HIV-1 gag, HIV-1 env, HIV-1 nef, HBV (surface or core antigens), HPV, FAS, HSV-1, HSV-2, p17, ORF2 and ORF3 antigens. Examples of bacterial antigens include, but are not limited to, *Toxoplasma gondii* or *Treponema pallidum.* The antibody-bacterial antigen conjugates of the invention can be in the treatment or prevention of various bacterial diseases such as Anthrax, Botulism, Tetanus, Chlamydia, Cholera, Diphtheria, Lyme Disease, Syphilis and Tuberculosis. Other suitable antigens from infectious disease pathogens, such as viruses, bacteria, parasites and fungi are disclosed below.

Sequences of the foregoing antigens are well known in the art. For example, an example of a MAGE-3 cDNA sequence is provided in US 6,235,525 (Ludwig Institute for Cancer Research); examples of NY-ESO-1 nucleic acid and protein sequences are provided in US 5,804,381 and US 6,069,233 (Ludwig Institute for Cancer Research); examples of Melan-A nucleic acid and protein sequences are provided in US 5,620,886 and US 5,854,203 (Ludwig Institute for Cancer Research); examples of NY-BR-1 nucleic acid and protein sequences are provided in US 6,774,226 and US 6,911,529 (Ludwig Institute for Cancer Research) and examples of NY-CO-58 nucleic acid and protein sequences are provided in WO 02090986 (Ludwig Institute for Cancer Research); an example of an amino acid sequence for the HER-2/neu protein is available at GENBANK^{®} Accession No. AAA58637; and a nucleotide sequence (mRNA) for human carcinoembryonic antigen-like 1 (CEA-1) is available at GENBANK^{®} Accession No. NM_020219.

An HPV antigen that may be used in the compositions and the methods of the invention may include, for example an HPV-16 antigen, an HPV- 18 antigen, an HPV-31 antigen, an HPV-33 antigen and/or an HPV-35 antigen; and is suitably an HPV-16 antigen and/or HPV-18 antigen. A genome of HPV-16 is described in Virology, 145:181- 185 (1985) and DNA sequences encoding HPV-18 are described in US Patent No. 5,840,306. HPV-16 antigens (e.g., seroreactive regions of the El and/or E2 proteins of HPV-16) are described in US Patent No. 6,531,127, and HPV-18 antigens (e.g., seroreactive regions of the Ll and/or L2 proteins of HPV-18) are described in US Patent No. 5,840,306. Similarly, a complete genome for HBV is available at GENBANK^{®} Accession No. NC_003977. The genome of HCV is described in European Patent Application No. 318 216. PCT/US90/01348 discloses sequence information of clones of the HCV genome, amino acid sequences of HCV viral proteins and methods of making and using such compositions for HCV vaccines comprising HCV proteins and peptides derived there from.

Antigenic peptides of proteins (*i.e.,* those containing T cell epitopes) can be identified in a variety of manners well known in the art. For example, T cell epitopes can be predicted by analyzing the sequence of the protein using web-based predictive algorithms (BIMAS & SYFPEITHI) to generate potential MHC class I and II- binding peptides that match an internal database of 10,000 well characterized MHC binding peptides previously defined by CTLs. High scoring peptides can be ranked and selected as "interesting" on the basis of high affinity to a given MHC molecule.

Another method for identifying antigenic peptides containing T cell epitopes is by dividing the protein into non-overlapping peptides of desired length or overlapping peptides of desired lengths which can be produced recombinantly, synthetically, or in certain limited situations, by chemical cleavage of the protein and tested for immunogenic properties, *e.g.,* eliciting a T cell response (*i.e.,* proliferation or lymphokine secretion).

In order to determine precise T cell epitopes of the protein by, for example, fine mapping techniques, a peptide having T cell stimulating activity and thus comprising at least one T cell epitope, as determined by T cell biology techniques, can be modified by addition or deletion of amino acid residues at either the amino or carboxy terminus of the peptide and tested to determine a change in T cell reactivity to the modified peptide. If two or more peptides which share an area of overlap in the native protein sequence are found to have human T cell stimulating activity, as determined by T cell biology techniques, additional peptides can be produced comprising all or a portion of such peptides and these additional peptides can be tested by a similar procedure. Following this technique, peptides are selected and produced recombinantly or synthetically. Peptides are selected based on various factors, including the strength of the T cell response to the peptide (*e.g.,* stimulation index). The physical and chemical properties of these selected peptides (*e.g.,* solubility, stability) can then be examined to determine whether the peptides are suitable for use in therapeutic compositions or whether the peptides require modification.

The term "antigen presenting cell" or "APC" is a cell that displays foreign antigen complexed with MHC on its surface. T-cells recognize this complex using T-cell receptor (TCR). Examples of APCs include, but are not limited to, dendritic cells (DCs), peripheral blood mononuclear cells (PBMC), monocytes (such as THP-1), B lymphoblastoid cells (such as C1R.A2, 1518 B-LCL) and monocyte-derived dendritic cells (DCs). Some APCs internalize antigens either by phagocytosis or by receptor-mediated endocytosis. Examples of APC receptors include, but are not limited to C-type lectins, such as, the human Dendritic and Epithelial Cell 205 receptor (DEC-205), and the human macrophage mannose receptor.

The term "antigen presentation" refers to the process by which APCs capture antigens and enables their recognition by T-cells, *e.g.,* as a component of an MHC-I and/or MHC-II conjugate.

"MHC molecules" include two types of molecules, MHC class I and MHC class II. MHC class I molecules present antigen to specific CD8+ T cells and MHC class II molecules present antigen to specific CD4+ T cells. Antigens delivered exogenously to APCs are processed primarily for association with MHC class II. In contrast, antigens delivered endogenously to APCs are processed primarily for association with MHC class I.

As used herein, the term "immunostimulatory agent" includes but is not limited to compounds capable of stimulating APCs, such as DCs and macrophages. For example, suitable immunostimulatory agents for use in the present invention are capable of stimulating APCs, so that the maturation process of the APCs is accelerated, the proliferation of APCs is increased, and/or the recruitment or release of co-stimulatory molecules (*e.g.,* CD80, CD86, ICAM-1, MHC molecules and CCR7) and pro-inflammatory cytokines (*e.g.,* IL-1β, IL-6, IL-12, IL-15, and IFN-γ) is upregulated. Suitable immunostimulatory agents are also capable of increasing T cell proliferation. Such immunostimulatory agents include, but are not be limited to, CD27 ligand; FLT 3 ligand; cytokines, such as IFN-α, IFN-β, IFN-γ and IL-2; colony-stimulating factors, such as G-CSF (granulocyte colony-stimulating factor) and GM-CSF (granulocyte-macrophage colony-stimulating factor); an anti-CTLA-4 antibody, anti-PD1 antibody, anti-41BB antibody, or anti-OX-40 antibody; LPS (endotoxin); ssRNA; dsRNA; Bacille Calmette-Guerin (BCG); Levamisole hydrochloride; and intravenous immune globulins. An immunostimulatory agent may be a Toll-like Receptor (TLR) agonist. For example the immunostimulatory agent may be a TLR3 agonist such as double-stranded inosine:cytosine polynucleotide (Poly I:C, for example available as AmpligenTM from Hemispherx Bipharma, PA, US or Poly IC:LC from Oncovir) or Poly A:U; a TLR4 agonist such as monophosphoryl lipid A (MPL) or RC-529 (for example as available from GSK, UK); a TLR5 agonist such as flagellin; a TLR7 or TLR8 agonist such as an imidazoquinoline TLR7 or TLR 8 agonist, for example imiquimod (e.g., AldaraTM) or resiquimod and related imidazoquinoline agents (for example as available from 3M Corporation); or a TLR 9 agonist such as a deoxynucleotide with unmethylated CpG motifs (so-called "CpGs", for example as available from Coley Pharmaceutical). Such immunostimulatory agents may be administered simultaneously, separately or sequentially with the antibodies and constructs of the present invention and may also be physically linked to the antibodies and constructs.

As used herein, the term "linked" refers to the association of two or more molecules. The linkage can be covalent or non-covalent. The linkage also can be genetic *(i.e.,* recombinantly fused). Such linkages can be achieved using a wide variety of art recognized techniques, such as chemical conjugation and recombinant protein production.

As used herein, the term antigen "cross-presentation" refers to presentation of exogenous protein antigens to T cells via MHC class I and class II molecules on APCs.

As used herein, the term "T cell-mediated response" refers to any response mediated by T cells, including effector T cells (*e.g.,* CD8⁺ cells) and helper T cells (*e.g.,* CD4⁺ cells). T cell mediated responses include, for example, T cell cytotoxicity and proliferation.

As used herein, the term "cytotoxic T lymphocyte (CTL) response" refers to an immune response induced by cytotoxic T cells. CTL responses are mediated primarily by CD8⁺ T cells.

As used herein, the terms "inhibits" or "blocks" (e.g., referring to inhibition/blocking of binding of CD40L to CD40 on cells) are used interchangeably and encompass both partial and complete inhibition/blocking. The inhibition/blocking of CD40L preferably reduces or alters the normal level or type of activity that occurs when CD40L binding occurs without inhibition or blocking. Inhibition and blocking are also intended to include any measurable decrease in the binding affinity of CD40L when in contact with an anti-CD40 antibody as compared to CD40L not in contact with an anti-CD40 antibody, *e.g*., inhibits binding of CD40L by at least about 10%, 15%, 20%, 25%, 30%, 35%, 40%, 45%, 50%, 55%, 60%, 65%, 70%, 75% , 80%, 85%, 90%, 95%, 96%, 97%, 98%, 99%, or 100%. In a particular embodiment, the anti-CD40 antibody inhibits binding of CD40L by at least about 70% as measured, e.g., by a BLI or SPR (Biacore) assay. In another embodiment, the anti-CD40 antibody inhibits binding of CD40L by at least about 80%.

As used herein, the term "inhibits growth" (*e.g.*, referring to cells) is intended to include any measurable decrease in the growth of a cell, *e.g.,* the inhibition of growth of a cell by at least about 10%, 20%, 30%, 40%, 50%, 60%, 70%, 80%, 90%, 99%, or 100%.

The terms "inducing an immune response," "increasing an immune response," and "enhancing an immune response" are used interchangeably and refer the stimulation of an immune response (*i.e.,* either passive or adaptive) to a particular antigen.

The terms "induce" and "increase" as used with respect to inducing CDC or ADCC refer to the stimulation of particular direct cell killing mechanisms. For example, in one embodiment, the antibody induces at least about 20, 25, 30, 35, 40, 45, 50, 55, or 60 % lysis via CDC of CD40 expressing cells at a concentration of 10µg/ml. In a preferred embodiment, the antibody induces at least about 40 % lysis via CDC of CD40 expressing cells at a concentration of 10µg/ml. In another embodiment, the antibody induces at least about 20, 25, 30, 35, 40, 45, 50, 55, 60, 65, 70, 75, 80, or 85% lysis via ADCC (*i.e.,* specific lysis) of CD40 expressing cells at a concentration of 10µg/ml. In one embodiment, the antibody induces at least about 40 % lysis via ADCC of CD40 expressing cells at a concentration of 10µg/ml.

The terms "treat," "treating," and "treatment," as used herein, refer to therapeutic or preventative measures described herein. The methods of "treatment" employ administration to a subject, in need of such treatment, a human antibody of the present invention, for example, a subject in need of an enhanced immune response against a particular antigen or a subject who ultimately may acquire such a disorder, in order to prevent, cure, delay, reduce the severity of, or ameliorate one or more symptoms of the disorder or recurring disorder, or in order to prolong the survival of a subject beyond that expected in the absence of such treatment.

The term "effective dose" or "effective dosage" is defined as an amount sufficient to achieve or at least partially achieve the desired effect. The term "therapeutically effective dose" is defined as an amount sufficient to cure or at least partially arrest the disease and its complications in a patient already suffering from the disease. Amounts effective for this use will depend upon the severity of the disorder being treated and the general state of the patient's own immune system.

As used herein, the term "synergistic" means that administration of two drugs produce a greater effect when used in combination than would be expected from adding the individual effects of the two components, for example greater than two times, greater than three times, greater than five times or greater than ten times what would be expected from adding the individual effects of the two components. For example, drug interactions can be analyzed using the commercial software package Calcusyn, which is based on the median effect model of Chou and Talalay (Chou, T.C. & Talalay, P. (1984) Adv. Enzyme Regul. 22, 27-55. Quantatative analysis of dose-effect relationships: the combined effects of multiple drugs or enzyme inhibitors). A Combination Index (C.I.) of 1 indicated an additive drug interaction, whereas a C.I. greater than 1 was antagonistic and a score lower than 1 was synergistic. The CI value definitions are as follows: 1.45-1.2 is moderately antagonistic, 1.2-1.1 is slightly antagonistic, 1.1-0.9 is additive, 0.9-0.85 is slightly synergistic, 0.85-0.7 is moderately synergistic and 0.7-0.3 is synergistic.

The term "patient" includes human and other mammalian subjects that receive either prophylactic or therapeutic treatment.

As used herein, the term "subject" includes any human or non-human animal. For example, the methods and compositions of the present invention can be used to treat a subject with an immune disorder. The term "non-human animal" includes all vertebrates, *e.g.*, mammals and non-mammals, such as non-human primates, sheep, dog, cow, chickens, amphibians, reptiles, *etc.*

### I. Production of Antibodies to CD40

Anti-CD40 antibodies of the invention can be produced using a variety of known techniques, such as the standard somatic cell hybridization technique described by Kohler and Milstein, Nature 256: 495 (1975). Although somatic cell hybridization procedures can be used, in principle, other techniques for producing monoclonal antibodies also can be employed, *e.g*., viral or oncogenic transformation of B lymphocytes, phage display technique using libraries of human antibody genes.

A mouse (e.g., an H2L2 strain of Harbour^{®} transgenic mice) or other appropriate host animal may be immunized with a suitable antigen in order to elicit lymphocytes that produce or are capable of producing antibodies that will specifically bind to the antigen used for immunization. Alternatively, lymphocytes may be immunized *in vitro.* Lymphocytes can then be fused with myeloma cells using a suitable fusing agent, such as polyethylene glycol, to form a hybridoma cell (Goding, Monoclonal Antibodies: Principles and Practice, pp.59-103 (Academic Press, 1986)). Culture medium in which hybridoma cells are growing is assayed for production of monoclonal antibodies directed against the antigen. After hybridoma cells are identified that produce antibodies of the desired specificity, affinity, and/or activity, the clones may be subcloned by limiting dilution procedures and grown by standard methods (Goding, Monoclonal Antibodies: Principles and Practice, pp. 59-103 (Academic Press, 1986)). Suitable culture media for this purpose include, for example, D-MEM or RPMI-1640 medium. In addition, the hybridoma cells may be grown *in vivo* as ascites tumors in an animal. The monoclonal antibodies secreted by the subclones can be separated from the culture medium, ascites fluid, or serum by conventional immunoglobulin purification procedures such as, for example, protein A-Sepharose, hydroxylapatite chromatography, gel electrophoresis, dialysis, or affinity chromatography.

Antibodies directed against CD40 may be generated using transgenic or transchromosomal mice carrying parts of the human immune system rather than the mouse system. Transgenic mice may be employed, referred to herein as "HuMAb mice" which contain a human immunoglobulin gene miniloci that encodes unrearranged human heavy (µ and γ) and κ light chain immunoglobulin sequences, together with targeted mutations that inactivate the endogenous µ and κ chain loci (Lonberg, N. et al. (1994) Nature 368(6474): 856-859). Accordingly, the mice exhibit reduced expression of mouse IgM or κ, and in response to immunization, the introduced human heavy and light chain transgenes undergo class switching and somatic mutation to generate high affinity human IgGκ monoclonal antibodies (Lonberg, N. *et al.* (1994), *supra;* reviewed in Lonberg, N. (1994) Handbook of Experimental Pharmacology 113:49-101; Lonberg, N. and Huszar, D. (1995) Intern. Rev. Immunol. Vol. 13: 65-93, and Harding, F. and Lonberg, N. (1995) Ann. N.Y. Acad. Sci 764:536-546). The preparation of HuMAb mice is described in detail in Section II below and in Taylor, L. et al. (1992) Nucleic Acids Research 20:6287-6295; Chen, J. et al. (1993) International Immunology 5: 647-656; Tuaillon et al. (1993) Proc. Natl. Acad. Sci USA 90:3720-3724; Choi et al. (1993) Nature Genetics 4:117-123; Chen, J. et al. (1993) EMBO J. 12: 821-830; Tuaillon et al. (1994) J. Immunol. 152:2912-2920; Lonberg et al., (1994) Nature 368(6474): 856-859; Lonberg, N. (1994) Handbook of Experimental Pharmacology 113:49-101; Taylor, L. et al. (1994) International Immunology 6: 579-591; Lonberg, N. and Huszar, D. (1995) Intern. Rev. Immunol. Vol. 13: 65-93; Harding, F. and Lonberg, N. (1995) Ann. N.Y. Acad. Sci 764:536-546; Fishwild, D. et al. (1996) Nature Biotechnology 14: 845-851. See further, U.S. Patent Nos. 5,545,806; 5,569,825; 5,625,126; 5,633,425; 5,789,650; 5,877,397; 5,661,016; 5,814,318; 5,874,299; and 5,770,429; all to Lonberg and Kay, and GenPharm International; U.S. Patent No. 5,545,807 to Surani et al.; International Publication Nos. WO 98/24884, published on June 11, 1998; WO 94/25585, published November 10, 1994; WO 93/1227, published June 24, 1993; WO 92/22645, published December 23, 1992; WO 92/03918, published March 19, 1992.

Antibodies that bind human CD40 can be isolated from antibody phage libraries generated using the techniques described in, for example, McCafferty et al., Nature, 348:552-554 (1990). Clackson et al., Nature, 352:624-628 (1991), Marks et al., J. Mol. Biol., 222:581-597 (1991) and Hoet et al (2005) Nature Biotechnology 23, 344-348 ; U.S. Patent Nos. 5,223,409; 5,403,484; and 5,571,698 to Ladner et al.; U.S. Patent Nos. 5,427,908 and 5,580,717 to Dower et al.*;* U.S. Patent Nos. 5,969,108 and 6,172,197 to McCafferty et al.*;* and U.S. Patent Nos. 5,885,793; 6,521,404; 6,544,731; 6,555,313; 6,582,915 and 6,593,081 to Griffiths et al.*.* Additionally, production of high affinity (nM range) human antibodies by chain shuffling (Marks et al., Bio/Technology, 10:779-783 (1992)), as well as combinatorial infection and *in vivo* recombination as a strategy for constructing very large phage libraries (Waterhouse et al., Nuc. Acids. Res., 21:2265-2266 (1993)) may also be used.

Antibodies that binds human CD40 may be produced using the phage display technique described by Hoet *et al., supra.* This technique involves the generation of a human Fab library having a unique combination of immunoglobulin sequences isolated from human donors and having synthetic diversity in the heavy-chain CDRs is generated. The library is then screened for Fabs that bind to human CD40.

The preferred animal system for generating hybridomas which produce antibodies of the invention is the murine system. Hybridoma production in the mouse is well known in the art, including immunization protocols and techniques for isolating and fusing immunized splenocytes.

### Generation of Transfectomas Producing Monoclonal Antibodies to CD40

Antibodies can be produced in a host cell transfectoma using, for example, a combination of recombinant DNA techniques and gene transfection methods as is well known in the art (Morrison, S. (1985) Science 229:1202).

For example, the gene(s) of interest, *e.g*., human antibody genes, can be ligated into an expression vector such as a eukaryotic expression plasmid such as used by GS gene expression system disclosed in WO 87/04462, WO 89/01036 and EP 338 841 or other expression systems well known in the art. The purified plasmid with the cloned antibody genes can be introduced in eukaryotic host cells such as CHO-cells or NSO-cells or alternatively other eukaryotic cells like a plant derived cells, fungi or yeast cells. The method used to introduce these genes could be methods described in the art such as electroporation, lipofectine, lipofectamine or other. After introducing these antibody genes in the host cells, cells expressing the antibody can be identified and selected. These cells represent the transfectomas which can then be amplified for their expression level and upscaled to produce antibodies. Recombinant antibodies can be isolated and purified from these culture supernatants and/or cells.

Alternatively, these cloned antibody genes can be expressed in other expression systems such as *E. coli* or in complete organisms or can be synthetically expressed.

### Use of Partial Antibody Sequences to Express Intact Antibodies

Antibodies interact with target antigens predominantly through amino acid residues that are located in the six heavy and light chain complementarity determining regions (CDRs). For this reason, the amino acid sequences within CDRs are more diverse between individual antibodies than sequences outside of CDRs. Because CDR sequences are responsible for most antibody-antigen interactions, it is possible to express recombinant antibodies that mimic the properties of specific naturally occurring antibodies by constructing expression vectors that include CDR sequences from the specific naturally occurring antibody grafted onto framework sequences from a different antibody with different properties (see, *e.g.,* Riechmann, L. et al., 1998, Nature 332:323-327; Jones, P. et al., 1986, Nature 321:522-525; and Queen, C. et al., 1989, Proc. Natl. Acad. See. U.S.A. 86:10029-10033). Such framework sequences can be obtained from public DNA databases that include germline antibody gene sequences. These germline sequences will differ from mature antibody gene sequences because they will not include completely assembled variable genes, which are formed by V(D)J joining during B cell maturation. Germline gene sequences will also differ from the sequences of a high affinity secondary repertoire antibody at individual evenly across the variable region. For example, somatic mutations are relatively infrequent in the amino-terminal portion of framework region. For example, somatic mutations are relatively infrequent in the amino terminal portion of framework region 1 and in the carboxy-terminal portion of framework region 4. Furthermore, many somatic mutations do not significantly alter the binding properties of the antibody. For this reason, it is not necessary to obtain the entire DNA sequence of a particular antibody in order to recreate an intact recombinant antibody having binding properties similar to those of the original antibody (see PCT/US99/05535 filed on March 12, 1999). Partial heavy and light chain sequence spanning the CDR regions is typically sufficient for this purpose. The partial sequence is used to determine which germline variable and joining gene segments contributed to the recombined antibody variable genes. The germline sequence is then used to fill in missing portions of the variable regions. Heavy and light chain leader sequences are cleaved during protein maturation and do not contribute to the properties of the final antibody. To add missing sequences, cloned cDNA sequences can be combined with synthetic oligonucleotides by ligation or PCR amplification. Alternatively, the entire variable region can be synthesized as a set of short, overlapping, oligonucleotides and combined by PCR amplification to create an entirely synthetic variable region clone. This process has certain advantages such as elimination or inclusion or particular restriction sites, or optimization of particular codons.

The nucleotide sequences of heavy and light chain transcripts from a hybridoma are used to design an overlapping set of synthetic oligonucleotides to create synthetic V sequences with identical amino acid coding capacities as the natural sequences. The synthetic heavy and kappa chain sequences can differ from the natural sequences in three ways: strings of repeated nucleotide bases are interrupted to facilitate oligonucleotide synthesis and PCR amplification; optimal translation initiation sites are incorporated according to Kozak's rules (Kozak, 1991, J. Biol. Chem. 266:19867-19870); and, HindIII sites are engineered upstream of the translation initiation sites.

For both the heavy and light chain variable regions, the optimized coding, and corresponding non-coding, strand sequences are broken down into 30 - 50 nucleotide approximately the midpoint of the corresponding non-coding oligonucleotide. Thus, for each chain, the oligonucleotides can be assembled into overlapping double stranded sets that span segments of 150 - 400 nucleotides. The pools are then used as templates to produce PCR amplification products of 150 - 400 nucleotides. Typically, a single variable region oligonucleotide set will be broken down into two pools which are separately amplified to generate two overlapping PCR products. These overlapping products are then combined by PCR amplification to form the complete variable region. It may also be desirable to include an overlapping fragment of the heavy or light chain constant region (including the BbsI site of the kappa light chain, or the AgeI site if the gamma heavy chain) in the PCR amplification to generate fragments that can easily be cloned into the expression vector constructs.

The reconstructed heavy and light chain variable regions are then combined with cloned promoter, leader sequence, translation initiation, leader sequence, constant region, 3' untranslated, polyadenylation, and transcription termination, sequences to form expression vector constructs. The heavy and light chain expression constructs can be combined into a single vector, co-transfected, serially transfected, or separately transfected into host cells which are then fused to form a host cell expressing both chains.

Plasmids for use in construction of expression vectors were constructed so that PCR amplified V heavy and V kappa light chain cDNA sequences could be used to reconstruct complete heavy and light chain minigenes. These plasmids can be used to express completely human IgG₁κ or IgG₄κ antibodies. Fully human and chimeric antibodies of the present invention also include IgG2, IgG3, IgE, IgA, IgM, and IgD antibodies. Similar plasmids can be constructed for expression of other heavy chain isotypes, or for expression of antibodies comprising lambda light chains.

Structural features of anti-CD40 antibodies may be used to create structurally related anti-CD40 antibodies that retain at least one functional property, such as, for example,
(a) inducing or enhancing an immune response to an antigen independent of Fc receptor binding;
(b) inducing or enhancing an immune response to an antigen without inducing antibody-dependent cellular cytotoxicity (ADCC) of CD40 expressing cells;
(c) inducing or enhancingan immune response to an antigen without inducing complement dependent cellular cytotoxicity (CDC) of CD40 expressing cells; and/or
(d) capable of synergising with CD40L; and/or
Additional features may include, for example:
(d) no inhibiting or no blocking binding of CD40L;
(e) inhibiting or blocking binding of CD40L;
(f) inhibiting or blocking binding of CD40L to human CD40 independent of Fc receptor binding;
(g) inducing or enhancing cellular apoptosis of a tumor cell;
(h) inducing or enhancing T-cell stimulatory activity of a cell (e.g., as measured by an increase in the expression of IL-12p40); and/or
(i) inducing or enhancing B-cell activation (e.g., as measured by an increase in the expression of at least one cell-surface marker selected from the group consisting of HLA-DR V450, CD54 PE, CD86 APC, and CD83 BV510, CD19 V500, CD54 PE, HLA-DR V450, CD23 PerCP-Cy5.5, CD69 APC, CD86 APC, CD38 PerCP-Cy5.5 and CD71 PE).

One or more CDR regions of antibodies may be combined recombinantly with known framework regions and CDRs to create additional, recombinantly-engineered, anti-CD40 antibodies. The heavy and light chain variable framework regions can be derived from the same or different antibody sequences. The antibody sequences can be the sequences of naturally occurring antibodies or can be consensus sequences of several antibodies. See Kettleborough et al., Protein Engineering 4:773 (1991); Kolbinger et al., Protein Engineering 6:971 (1993) and Carter et al., WO 92/22653.

A method for preparing an anti-CD40 antibody may include: preparing an antibody including (1) heavy chain framework regions and heavy chain CDRs, where at least one of the heavy chain CDRs includes an amino acid sequence selected from the amino acid sequences of CDRs shown in SEQ ID NOs:5, 6, 7, 8, 9, 10, 19, 20, 21, 22, 23, 24, 33, 34, 35, 36, 37, 38, 47, 48, 49, 51, 52, 61, 62, 63, 64, 65, 66, 75, 76, 77, 78, 79, 80, 89, 90, 91, 92, 93, 94, 103, 104, 105, 106, 107, 108; and (2) light chain framework regions and light chain CDRs, where at least one of the light chain CDRs includes an amino acid sequence selected from the amino acid sequences of CDRs shown in SEQ ID NOs:11, 12, 13, 14, 15, 16, 25, 26, 27, 28, 29, 30, 39, 40, 41, 42, 43, 44, 53, 54, 55, 56, 57, 58, 67, 68, 69, 70, 71, 72, 81, 82, 83, 84, 85, 86, 95, 96, 97, 98, 99, 100, 109, 110, 111, 112, 113, 114; where the antibody retains the ability to bind to CD40. The ability of the antibody to bind CD40 can be determined using standard binding assays, such as those set forth in the Examples (*e.g.*, an ELISA or a FLISA).

It is well known in the art that antibody heavy and light chain CDR3 domains play a particularly important role in the binding specificity/affinity of an antibody for an antigen (*see,* Hall et al., J. Imunol., 149:1605-1612 (1992); Polymenis et al., J. Immunol., 152:5318-5329 (1994); Jahn et al., Immunobiol., 193:400-419 (1995); Klimka et al., Brit. J. Cancer, 83:252-260 (2000); Beiboer et al., J. Mol. Biol, 296:833-849 (2000); Rader et al., Proc. Natl. Acad. Sci. USA, 95:8910-8915 (1998); Barbas et al., J. Am. Chem. Soc., 116:2161-2162 (1994); Ditzel et al., J. Immunol., 157:739-749 (1996)). Accordingly, the recombinant antibodies prepared as set forth above may comprise the heavy and/or light chain CDR3s of antibodies 3C3, 3G5, 1B4, 3B6, 6H6, 2E1.2, 1B5-NK, and 3B6-NS. The antibodies further can comprise the CDR2s of antibodies 3C3, 3G5, 1B4, 3B6, 6H6, 2E1.2, 1B5-NK, and 3B6-NS. The antibodies further can comprise the CDR1s of antibodies 3C3, 3G5, 1B4, 3B6, 6H6, 2E1.2, 1B5-NK, and 3B6-NS. The antibodies can further comprise any combinations of the CDRs.

Accordingly, anti-CD40 antibodies may comprise: (1) heavy chain framework regions, a heavy chain CDR1 region, a heavy chain CDR2 region, and a heavy chain CDR3 region, wherein the heavy chain CDR3 region is selected from the CDR3s of 3C3, 3G5, 1B4, 3B6, 6H6, 2E1.2, 1B5-NK, or 3B6-NS, and (2) light chain framework regions, a light chain CDR1 region, a light chain CDR2 region, and a light chain CDR3 region, wherein the light chain CDR3 region is selected from the CDR3s of 3C3, 3G5, 1B4, 3B6, 6H6, 2E1.2, 1B5-NK, or 3B6-NS, wherein the antibody binds CD40. The antibody may further include the heavy chain CDR2 and/or the light chain CDR2 of antibodies 3C3, 3G5, 1B4, 3B6, 6H6, 2E1.2, 1B5-NK, or 3B6-NS. The antibody may further comprise the heavy chain CDR1 and/or the light chain CDR1 of antibodies 3C3, 3G5, 1B4, 3B6, 6H6, 2E1.2, 1B5-NK, or 3B6-NS.

### Generation of Antibodies Having Modified Sequences

The variable region sequences, or portions thereof, of the anti-CD40 antibodies of the invention may be modified to create structurally related anti-CD40 antibodies that retain binding (*i.e.,* to the same epitope as the unmodified antibody) and, thus, are functionally equivalent. Methods for identifying residues that can be altered without removing antigen binding are well-known in the art (see, *e.g.,* Marks et al. (Biotechnology (1992) 10(7):779-83 (monoclonal antibodies diversification by shuffling light chain variable regions, then heavy chain variable regions with fixed CDR3 sequence changes), Jespers et al.(1994) Biotechnology 12(9):899-903 (selection of human antibodies from phage display repertoires to a single epitope of an antigen), Sharon et al. (1986) PNAS USA 83(8):2628-31 (site-directed mutagenesis of an invariant amino acid residue at the variable-diversity segments junction of an antibody); Casson et al. (1995) J. Immunol. 155(12):5647-54 (evolution of loss and change of specificity resulting from random mutagenesis of an antibody heavy chain variable region).

Accordingly, the CDR1, 2, and/or 3 regions of the engineered antibodies described above can comprise the exact amino acid sequence(s) as those of antibodies 3C3, 3G5, 1B4, 3B6, 6H6, 2E1.2, 1B5-NK, or 3B6-NS disclosed herein. However, antibodies may comprise derivatives from the exact CDR sequences of 3C3, 3G5, 1B4, 3B6, 6H6, 2E1.2, 1B5-NK, or 3B6-NS, yet still retain the ability of to bind CD40 effectively. Such sequence modifications may include one or more amino acid additions, deletions, or substitutions, *e.g.*, conservative sequence modifications as described above. Sequence modifications may also be based on the consensus sequences described above for the particular CDR1, CDR2, and CDR3 sequences of antibodies 3C3, 3G5, 1B4, 3B6, 6H6, 2E1.2, 1B5-NK, or 3B6-NS.

Accordingly, the engineered antibody may be composed of one or more CDRs that are, for example, 90%, 95%, 98% or 99.5% identical to one or more CDRs of antibodies 3C3, 3G5, 1B4, 3B6, 6H6, 2E1.2, 1B5-NK, or 3B6-NS. Ranges intermediate to the above-recited values, *e*.*g*., CDRs that are 90-95%, 95-98%, or 98-100% identical identity to one or more of the above sequences are also intended to be encompassed by the present invention.

One or more residues of a CDR may be altered to modify binding to achieve a more favored on-rate of binding, a more favored off-rate of binding, or both, such that an idealized binding constant is achieved. Using this strategy, an antibody having ultra high binding affinity of, for example, 10¹⁰ M⁻¹ or more, can be achieved. Affinity maturation techniques, well known in the art and those described herein, can be used to alter the CDR region(s) followed by screening of the resultant binding molecules for the desired change in binding. Accordingly, as CDR(s) are altered, changes in binding affinity as well as immunogenicity can be monitored and scored such that an antibody optimized for the best combined binding and low immunogenicity are achieved.

In addition to or instead of modifications within the CDRs, modifications can also be made within one or more of the framework regions, FR1, FR2, FR3 and FR4, of the heavy and/or the light chain variable regions of an antibody, so long as these modifications do not eliminate the binding affinity of the antibody. For example, one or more non-germline amino acid residues in the framework regions of the heavy and/or the light chain variable region of a antibody of the invention, is substituted with a germline amino acid residue, *i.e.,* the corresponding amino acid residue in the human germline sequence for the heavy or the light chain variable region, which the antibody has significant sequence identity with. For example, an antibody chain can be aligned to a germline antibody chain which it shares significant sequence identity with, and the amino acid residues which do not match between antibody framework sequence and the germline chain framework can be substituted with corresponding residues from the germline sequence. When an amino acid differs between a antibody variable framework region and an equivalent human germline sequence variable framework region, the antibody framework amino acid should usually be substituted by the equivalent human germline sequence amino acid if it is reasonably expected that the amino acid falls within one of the following categories:
(1) an amino acid residue which noncovalently binds antigen directly,
(2) an amino acid residue which is adjacent to a CDR region,
(3) an amino acid residue which otherwise interacts with a CDR region (*e.g.,* is within about 3-6 Å of a CDR region as determined by computer modeling), or
(4) an amino acid reside which participates in the VL-VH interface.

Residues which "noncovalently bind antigen directly" include amino acids in positions in framework regions which have a good probability of directly interacting with amino acids on the antigen according to established chemical forces, for example, by hydrogen bonding, Van der Waals forces, hydrophobic interactions, and the like. Accordingly, an amino acid residue in the framework region of an antibody of may be substituted with the corresponding germline amino acid residue which noncovalently binds antigen directly.

Residues which are "adjacent to a CDR region" include amino acid residues in positions immediately adjacent to one or more of the CDRs in the primary sequence of the antibody, for example, in positions immediately adjacent to a CDR as defined by Kabat, or a CDR as defined by Chothia (*see e.g.,* Chothia and Lesk J. Mol. Biol. 196:901 (1987)). Accordingly, an amino acid residue within the framework region of an antibody may be substituted with a corresponding germline amino acid residue which is adjacent to a CDR region.

Residues that "otherwise interact with a CDR region" include those that are determined by secondary structural analysis to be in a spatial orientation sufficient to affect a CDR region. Such amino acids will generally have a side chain atom within about 3 angstrom units (Å) of some atom in the CDRs and must contain an atom that could interact with the CDR atoms according to established chemical forces, such as those listed above. Accordingly, an amino acid residue within the framework region of an antibody may be substituted with the corresponding germline amino acid residue which otherwise interacts with a CDR region.

The amino acids at several positions in the framework are known to be important for determining CDR confirmation (*e.g.*, capable of interacting with the CDRs) in many antibodies (Chothia and Lesk, *supra,* Chothia *et al., supra* and Tramontano et al., J. Mol. Biol. 215:175 (1990)). These authors identified conserved framework residues important for CDR conformation by analysis of the structures of several known antibodies. The antibodies analyzed fell into a limited number of structural or "canonical" classes based on the conformation of the CDRs. Conserved framework residues within members of a canonical class are referred to as "canonical" residues. Canonical residues include residues 2, 25, 29, 30, 33, 48, 64, 71, 90, 94 and 95 of the light chain and residues 24, 26, 29, 34, 54, 55, 71 and 94 of the heavy chain. Additional residues (*e.g.*, CDR structure-determining residues) can be identified according to the methodology of Martin and Thorton (1996) J. Mol. Biol. 263:800. Notably, the amino acids at positions 2, 48, 64 and 71 of the light chain and 26-30, 71 and 94 of the heavy chain (numbering according to Kabat) are known to be capable of interacting with the CDRs in many antibodies. The amino acids at positions 35 in the light chain and 93 and 103 in the heavy chain are also likely to interact with the CDRs. Additional residues which may effect conformation of the CDRs can be identified according to the methodology of Foote and Winter (1992) J. Mol. Biol. 224:487. Such residues are termed "vernier" residues and are those residues in the framework region closely underlying *(i.e.,* forming a "platform" under) the CDRs.

Residues which "participate in the VL-VH interface" or "packing residues" include those residues at the interface between VL and VH as defined, for example, by Novotny and Haber, Proc. Natl. Acad. Sci. USA, 82:4592-66 (1985) or Chothia *et al, supra.*

Occasionally, there is some ambiguity about whether a particular amino acid falls within one or more of the above-mentioned categories. In such instances, alternative variant antibodies are produced, one of which has that particular substitution, the other of which does not. Alternative variant antibodies so produced can be tested in any of the assays described herein for the desired activity, and the preferred antibody selected.

Additional candidates for substitution within the framework region are amino acids that are unusual or "rare" for an antibody at that position. These amino acids can be substituted with amino acids from the equivalent position of the human germline sequence or from the equivalent positions of more typical antibodies. For example, substitution may be desirable when the amino acid in a framework region of the antibody is rare for that position and the corresponding amino acid in the germline sequence is common for that position in immunoglobulin sequences; or when the amino acid in the antibody is rare for that position and the corresponding amino acid in the germline sequence is also rare, relative to other sequences. It is contemplated that by replacing an unusual amino acid with an amino acid from the germline sequence that happens to be typical for antibodies, the antibody may be made less immunogenic. Substitution may also be desirable, for example in cases of unpaired cysteine residues or putative N-linked glycosylation sites.

The term "rare", as used herein, indicates an amino acid occurring at that position in less than about 20%, preferably less than about 10%, more preferably less than about 5%, even more preferably less than about 3%, even more preferably less than about 2% and even more preferably less than about 1% of sequences in a representative sample of sequences, and the term "common", as used herein, indicates an amino acid occurring in more than about 25% but usually more than about 50% of sequences in a representative sample. For example, all light and heavy chain variable region sequences are respectively grouped into "subgroups" of sequences that are especially homologous to each other and have the same amino acids at certain critical positions (Kabat *et al., supra*). When deciding whether an amino acid in an antibody sequence is "rare" or "common" among sequences, it will often be preferable to consider only those sequences in the same subgroup as the antibody sequence.

In general, the framework regions of antibodies are usually substantially identical, and more usually, identical to the framework regions of the human germline sequences from which they were derived. Of course, many of the amino acids in the framework region make little or no direct contribution to the specificity or affinity of an antibody. Thus, many individual conservative substitutions of framework residues can be tolerated without appreciable change of the specificity or affinity of the resulting immunoglobulin. Thus, the variable framework region of the antibody may share at least 85% sequence identity to a human germline variable framework region sequence or consensus of such sequences. The variable framework region of the antibody may share at least 90%, 95%, 96%, 97%, 98% or 99% sequence identity to a human germline variable framework region sequence or consensus of such sequences.

In addition to simply binding CD40, an antibody may be selected for its retention of other functional properties of antibodies of the invention, such as, for example:
(a) inducing or enhancing an immune response to an antigen independent of Fc receptor binding;
(b) inducing or enhancing an immune response to an antigen without inducing antibody-dependent cellular cytotoxicity (ADCC) of CD40 expressing cells;
(c) inducing or enhancingan immune response to an antigen without inducing complement dependent cellular cytotoxicity (CDC) of CD40 expressing cells; and/or
(d) capable of synergising with CD40L.

Additional features may include, for example:
(e) no blocking of binding of CD40L to human CD40 independent of Fc receptor binding;
(f) blocking of binding of CD40L to human CD40 independent of Fc receptor binding;
(g) activation of human CD40 expressed on an APC, independent of Fc receptor bidning;
(h) induction of apoptosis of a tumor cell;
(i) T-cell stimulatory activity; and/or
(j) enhanced B-cell activation.

### Characterization of Monoclonal Antibodies to CD40

Monoclonal antibodies of the invention can be characterized for binding to CD40 using a variety of known techniques. Generally, the antibodies are initially characterized by ELISA. Briefly, microtiter plates can be coated with purified CD40 in PBS, and then blocked with irrelevant proteins such as bovine serum albumin (BSA) diluted in PBS. Dilutions of plasma from CD40-immunized mice are added to each well and incubated for 1-2 hours at 37°C. The plates are washed with PBS/Tween 20 and then incubated with a goat-anti-human IgG Fc-specific polyclonal reagent conjugated to alkaline phosphatase for 1 hour at 37°C. After washing, the plates are developed with ABTS substrate, and analyzed at OD of 405. Preferably, mice which develop the highest titers will be used for fusions.

An ELISA assay as described above can be used to screen for antibodies and, thus, hybridomas that produce antibodies that show positive reactivity with the CD40 immunogen. Hybridomas that bind, preferably with high affinity, to CD40 can then be subcloned and further characterized. One clone from each hybridoma, which retains the reactivity of the parent cells (by ELISA), can then be chosen for making a cell bank, and for antibody purification.

To purify anti-CD40 antibodies, selected hybridomas can be grown in roller bottles, two-liter spinner-flasks or other culture systems. Supernatants can be filtered and concentrated before affinity chromatography with protein A-Sepharose (Pharmacia, Piscataway, NJ) to purify the protein. After buffer exchange to PBS, the concentration can be determined by OD₂₈₀ using 1.43 extinction coefficient or preferably by nephelometric analysis. IgG can be checked by gel electrophoresis and by antigen specific method.

To determine if the selected anti-CD40 monoclonal antibodies bind to unique epitopes, each antibody can be biotinylated using commercially available reagents (Pierce, Rockford, IL). Biotinylated MAb binding can be detected with a streptavidin labeled probe. To determine the isotype of purified antibodies, isotype ELISAs can be performed using art recognized techniques. For example, wells of microtiter plates can be coated with 10 µg/ml of anti- Ig overnight at 4°C. After blocking with 5% BSA, the plates are reacted with 10 µg/ml of monoclonal antibodies or purified isotype controls, at ambient temperature for two hours. The wells can then be reacted with either IgGl or other isotype specific conjugated probes. Plates are developed and analyzed as described above.

To test the binding of monoclonal antibodies to live cells expressing CD40, flow cytometry can be used. Briefly, cell lines and/or human PBMCs expressing membrane-bound CD40 (grown under standard growth conditions) are mixed with various concentrations of monoclonal antibodies in PBS containing 0.1% BSA at 4°C for 1 hour. After washing, the cells are reacted with Fluorescein-labeled anti- IgG antibody under the same conditions as the primary antibody staining. The samples can be analyzed by FACScan instrument using light and side scatter properties to gate on single cells and binding of the labeled antibodies is determined. An alternative assay using fluorescence microscopy may be used (in addition to or instead of) the flow cytometry assay. Cells can be stained exactly as described above and examined by fluorescence microscopy. This method allows visualization of individual cells, but may have diminished sensitivity depending on the density of the antigen.

Anti-CD40 IgGs can be further tested for reactivity with the CD40 antigen by Western blotting. Briefly, cell extracts from cells expressing CD40 can be prepared and subjected to sodium dodecyl sulfate polyacrylamide gel electrophoresis. After electrophoresis, the separated antigens will be transferred to nitrocellulose membranes, blocked with 20% mouse serum, and probed with the monoclonal antibodies to be tested. IgG binding can be detected using anti- IgG alkaline phosphatase and developed with BCIP/NBT substrate tablets (Sigma Chem. Co., St. Louis, MO).

Methods for analyzing binding affinity, cross-reactivity, and binding kinetics of various anti-CD40 antibodies include standard assays known in the art, for example, Biacore^{™} surface plasmon resonance (SPR) analysis using a Biacore^{™} 2000 SPR instrument (Biacore AB, Uppsala, Sweden), or bio-layer interferometry (BLI) using an Octet^{™} QKe instrument as described in the examples.

Agonistic anti-CD40 antibodies which bind to the same epitope as that of anti-CD40 antibodies 3C3, 3G5, 1B4, 3B6, 6H6, 2E1.2, 1B5-NK, and 3B6-NS (as determined by a given epitope mapping technique) also are provided herein. For example, as described in Example 17, antibodies disclosed herein (e.g., antibody 3C3) bind to one or more residues within amino acid residues 1-5 and 33-36 of the extracellular domain (ECD) of human CD40 (SEQ ID NO: 133), e.g., amino acids 5, 33, 34 and/or 36 of the ECD of human CD40 (SEQ ID NO: 133). Antibody 3C3 also is shown to further bind to one or more amino acids 26, 28 and/or 30 of the ECD of human CD40 (SEQ ID NO: 133), e.g., amino acids 5, 33, 34 and 36 of the ECD of human CD40 (SEQ ID NO: 133) or amino acids 5, 33 and 36 of the ECD of human CD40 (SEQ ID NO: 133).

Other antibodies disclosed herein (e.g., antibody 3G5) bind to one or more residues within amino acid residues 13-15 and 33-36 of the ECD of human CD40 (SEQ ID NO: 133), e.g., amino acids 33, 34 and 36 of the ECD of human CD40 (SEQ ID NO: 133).

Antibodies which bind to the epitopes on human CD40 described herein (e.g., the same epitopes as the exemplified antibodies) exhibit therapeutically advantageous properties. For example, as demonstrated in Examples 16 and 20, antibody 3C3 exhibits synergistic agnostic effects with soluble CD40 ligand (sCD40L), as measured by, for example, an increase in the induction of CD95 expression when incubated with Ramos cells, an increase in B cell proliferation when incubated with human B cells, and/or an increase in the induction of IL12p40 expression when incubated with dendritic cells.

Accordingly, antibodies that bind to the same epitope as 3C3 have the ability to synergize with other therapeutic agents, including those which bind to the ligand binding site of human CD40. Representative synergistic effects include, for example, upregulation of immune function (e.g. T cell mediated immune responses as in vaccine therapies, NK activation in cancer therapies), inhibition of cell growth (e.g., in cancer therapy), and/or enhanced processing and presentation of an antigen by APCs (e.g., in vaccine therapy).

As described herein, techniques for determining antibodies that bind to the "same epitope on CD40" with the antibodies described herein include, for example, epitope mapping methods, such as, x-ray analyses of crystals of antigen: antibody complexes which provides atomic resolution of the epitope. Other methods monitor the binding of the antibody to antigen fragments or mutated variations of the antigen where loss of binding due to a modification of an amino acid residue within the antigen sequence is often considered an indication of an epitope component. In addition, computational combinatorial methods for epitope mapping can also be used. Methods may also rely on the ability of an antibody of interest to affinity isolate specific short peptides (either in native three-dimensional form or in denatured form) from combinatorial phage display peptide libraries. The peptides are then regarded as leads for the definition of the epitope corresponding to the antibody used to screen the peptide library. For epitope mapping, computational algorithms have also been developed which have been shown to map conformational discontinuous epitopes.

### II. Molecular Conjugates/Immunotoxins

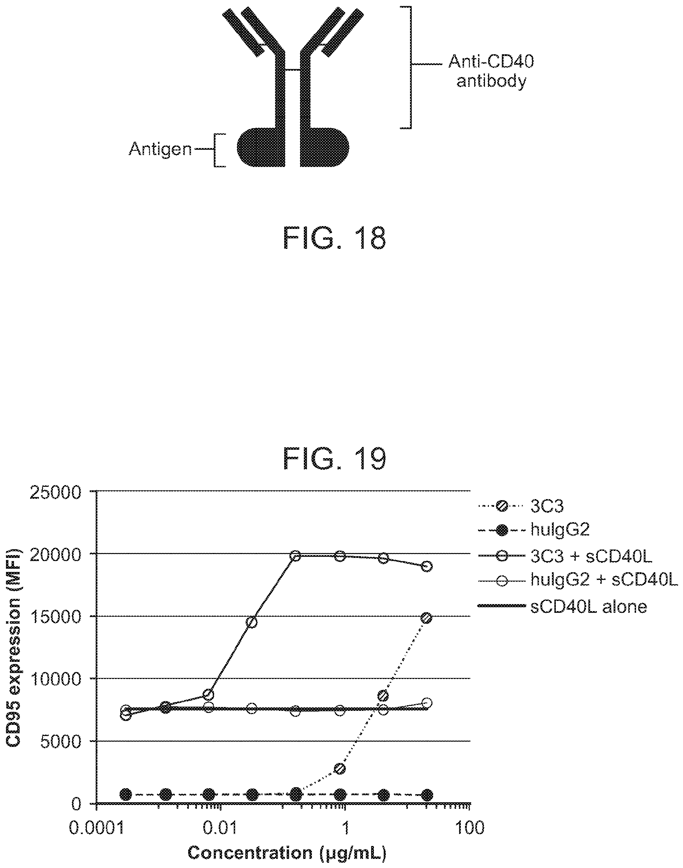

The present invention provides a variety of therapeutic molecular conjugates (e.g., vaccine conjugates) which include an antigen, such as a tumor or viral antigen, linked to an antibody that binds to CD40. This allows for targeting of the antigen to APCs, such as cells expressing CD40 *(e.g.,* dendritic cells, B cells, and macrophages) to enhance processing, presentation and, ultimately, an immune response against the antigen(s). A schematic representation of such a conjugate is shown in Figure 18 wherein, for example, an antigen is genetically fused to the CH3 domain of each of the heavy chains of a substantially complete anti-CD40 antibody. However, it will be appreciated that the antigen may alternatively be joined to other parts of such an antibody or fragment thereof, and that other forms of conjugation, such as chemical conjugation, may also be employed, as discussed further below.

Suitable antigens for use in the molecular conjugates include, for example, infectious disease antigens and tumor antigens, against which protective or therapeutic immune responses are desired, e.g., antigens expressed by a tumor cell or a pathogenic organism or infectious disease antigens. For example, suitable antigens include tumor-associated antigens for the prevention or treatment of cancers. Examples of tumor-associated antigens include, but are not limited to, sequences comprising all or part of the sequences of βhCG, gp100 or Pmel17, HER2/neu, WT1, mesothelin, CEA, gp100, MART1, TRP-2, melan-A, NY-ESO-1, NY-BR-1, NY-CO-58, MN (gp250), idiotype, MAGE-1, MAGE-3, MAGE-A3, Tyrosinase, Telomerase, SSX2 and MUC-1 antigens, and germ cell derived tumor antigens. Tumor associated antigens also include the blood group antigens, for example, Le^{a}, Le^{b}, LeX, LeY, H-2, B-1, B-2 antigens. Alternatively, more than one antigen can be included within the antigen-antibody constructs. For example, a MAGE antigen can be combined with other antigens such as melanin A, tyrosinase, and gp100 along with adjuvants such as GM-CSF or IL-12, and linked to an anti-APC antibody.

Other suitable antigens include viral antigens for the prevention or treatment of viral diseases. Examples of viral antigens include, but are not limited to, HIV-1 gag, HIV-1 env, HIV-1 nef, HBV (surface or core antigens), HPV, FAS, HSV-1, HSV-2, p17, ORF2 and ORF3 antigens. Examples of bacterial antigens include, but are not limited to, *Toxoplasma gondii* or *Treponema pallidum.* The antibody-bacterial antigen conjugates of the invention can be in the treatment or prevention of various bacterial diseases such as Anthrax, Botulism, Tetanus, Chlamydia, Cholera, Diphtheria, Lyme Disease, Syphilis and Tuberculosis.

Sequences of the above-described antigens are well known in the art. For example, an example of a MAGE-3 cDNA sequence is provided in US 6,235,525 (Ludwig Institute for Cancer Research); examples of NY-ESO-1 nucleic acid and protein sequences are provided in US 5,804,381 and US 6,069,233 (Ludwig Institute for Cancer Research); examples of Melan-A nucleic acid and protein sequences are provided in US 5,620,886 and US 5,854,203 (Ludwig Institute for Cancer Research); examples of NY-BR-1 nucleic acid and protein sequences are provided in US 6,774,226 and US 6,911,529 (Ludwig Institute for Cancer Research) and examples of NY-CO-58 nucleic acid and protein sequences are provided in WO 02090986 (Ludwig Institute for Cancer Research); an example of an amino acid sequence for the HER-2/neu protein is available at GENBANK^{®} Accession No. AAA58637; and a nucleotide sequence (mRNA) for human carcinoembryonic antigen-like 1 (CEA-1) is available at GENBANK^{®} Accession No. NM_020219.

In one embodiment, the antigen is an HPV antigen, for example, HPV-16 antigen, an HPV- 18 antigen, an HPV-31 antigen, an HPV-33 antigen and/or HPV-35 antigen. A genome of HPV-16 is described in Virology, 145:181- 185 (1985) and DNA sequences encoding HPV-18 are described in US Patent No. 5,840,306. HPV-16 antigens (e.g., seroreactive regions of the El and/or E2 proteins of HPV-16) are described in US Patent No. 6,531,127, and HPV-18 antigens (e.g., seroreactive regions of the Ll and/or L2 proteins of HPV-18) are described in US Patent No. 5,840,306. Similarly, a complete genome for HBV is available at GENBANK^{®} Accession No. NC_003977. The genome of HCV is described in European Patent Application No. 318 216. PCT/US90/01348 discloses sequence information of clones of the HCV genome, amino acid sequences of HCV viral proteins and methods of making and using such compositions for HCV vaccines comprising HCV proteins and peptides derived therefrom.

Antigenic peptides of proteins *(i.e.,* those containing T cell epitopes) can be identified in a variety of manners well known in the art. For example, T cell epitopes can be predicted by analyzing the sequence of the protein using web-based predictive algorithms (BIMAS & SYFPEITHI) to generate potential MHC class I and II- binding peptides that match an internal database of 10,000 well characterized MHC binding peptides previously defined by CTLs. High scoring peptides can be ranked and selected as "interesting" on the basis of high affinity to a given MHC molecule.

Another method for identifying antigenic peptides containing T cell epitopes involves dividing the protein into non-overlapping peptides of desired length or overlapping peptides of desired lengths which can be produced recombinantly, synthetically, or in certain limited situations, by chemical cleavage of the protein and tested for immunogenic properties, *e.g.,* eliciting a T cell response (*i.e.,* proliferation or lymphokine secretion).

In order to determine precise T cell epitopes of the protein by, for example, fine mapping techniques, a peptide having T cell stimulating activity and thus comprising at least one T cell epitope, as determined by T cell biology techniques, can be modified by addition or deletion of amino acid residues at either the amino or carboxy terminus of the peptide and tested to determine a change in T cell reactivity to the modified peptide. If two or more peptides which share an area of overlap in the native protein sequence are found to have human T cell stimulating activity, as determined by T cell biology techniques, additional peptides can be produced comprising all or a portion of such peptides and these additional peptides can be tested by a similar procedure. Following this technique, peptides are selected and produced recombinantly or synthetically. Peptides are selected based on various factors, including the strength of the T cell response to the peptide (*e.g.,* stimulation index). The physical and chemical properties of these selected peptides (*e.g.,* solubility, stability) can then be examined to determine whether the peptides are suitable for use in therapeutic compositions or whether the peptides require modification.

In addition, the vaccine conjugate can include one or more immunostimulatory agents that also enhance the immune response against the antigen. Antibody-antigen vaccine conjugates of the invention can be made genetically or chemically. In either case, the antibody portion of the conjugate may consist of the whole antibody or a portion of the antibody, such as the Fab fragment or single-chain Fv. In addition, more than one antigen and/or immunostimulatory agent can be included in the conjugate.

Chemically constructed antibody-antigen conjugates can be made using a variety of well known and readily available cross-linking reagents. These cross-linking reagents can be homofunctional or heterofunctional compounds, such as N-succinimidyl-3-(2-pyridyldithio)propionate (SPDP), N-succinimidyl-S-acetyl-thioacetate (SATA), sulfosuccinimidyl 4-(N-maleimidomethyl) cyclohaxane-1-carboxylate (sulfo-SMCC), 5,5'-dithiobis(2-nitrobenzoic acid) (DTNB), that form covalent linkages with different reactive amino acid or carbohydrate side chains on the anti-dendritic antibody and selected antigen. Other coupling and cross-linking agents also can be used to generate covalent linkages, such as protein A, carbodiimide, and o-phenylenedimaleimide (oPDM); (see *e.g.,* Karpovsky et al. (1984) J. Exp. Med. 160:1686; Liu, MA et al. (1985) Proc. Natl. Acad. Sci. USA 82:8648). Other methods include those described by Paulus (Behring Ins. Mitt. (1985) No. 78, 118-132); Brennan et al. (Science (1985) 229:81-83), and Glennie et al. (J. Immunol. (1987) 139: 2367-2375). Preferred conjugating agents are SATA and sulfo-SMCC, both available from Pierce Chemical Co. (Rockford, IL). Immunostimulatory agents can also be chemically linked to the molecular conjugates of the present invention using the same linking methods described above.

Antibodies may be linked to a therapeutic moiety, such as a cytotoxin, a drug or a radioisotope. When conjugated to a cytotoxin, these antibody conjugates are referred to as "immunotoxins." A cytotoxin or cytotoxic agent includes any agent that is detrimental to (*e.g.,* kills) cells. Examples include taxol, cytochalasin B, gramicidin D, ethidium bromide, emetine, mitomycin, etoposide, tenoposide, vincristine, vinblastine, colchicin, doxorubicin, daunorubicin, dihydroxy anthracin dione, mitoxantrone, mithramycin, actinomycin D, 1-dehydrotestosterone, glucocorticoids, procaine, tetracaine, lidocaine, propranolol, and puromycin and analogs or homologs thereof. Therapeutic agents include, but are not limited to, antimetabolites (*e.g.,* methotrexate, 6-mercaptopurine, 6-thioguanine, cytarabine, 5-fluorouracil decarbazine), alkylating agents (*e.g.,* mechlorethamine, thioepa chlorambucil, melphalan, carmustine (BSNU) and lomustine (CCNU), cyclothosphamide, busulfan, dibromomannitol, streptozotocin, mitomycin C, and cis-dichlorodiamine platinum (II) (DDP) cisplatin), anthracyclines (*e.g.,* daunorubicin (formerly daunomycin) and doxorubicin), antibiotics (*e.g.,* dactinomycin (formerly actinomycin), bleomycin, mithramycin, and anthramycin (AMC)), and anti-mitotic agents (*e.g.,* vincristine and vinblastine). An antibody can be conjugated to a radioisotope, *e*.*g*., radioactive iodine, to generate cytotoxic radiopharmaceuticals for treating a dendritic-related disorder, such as an autoimmune or inflammatory disease, or graft versus host disease.

The antibody conjugates may be used to modify a given biological response, and the drug moiety is not to be construed as limited to classical chemical therapeutic agents. For example, the drug moiety may be a protein or polypeptide possessing a desired biological activity. Such proteins may include, for example, an enzymatically active toxin, or active fragment thereof, such as abrin, ricin A, pseudomonas exotoxin, or diphtheria toxin; a protein such as tumor necrosis factor or interferon-y; or, biological response modifiers such as, for example, lymphokines, interleukin-1 ("IL-1"), interleukin-2 ("IL-2"), interleukin-6 ("IL-6"), granulocyte macrophage colony stimulating factor ("GM-CSF"), granulocyte colony stimulating factor ("G-CSF"), or other growth factors.

Techniques for conjugating such therapeutic moiety to antibodies are well known, see, *e.g.,* Arnon et al., "Monoclonal Antibodies For Immunotargeting Of Drugs In Cancer Therapy", in Monoclonal Antibodies And Cancer Therapy, Reisfeld et al. (eds.), pp. 243-56 (Alan R. Liss, Inc. 1985); Hellstrom et al., "Antibodies For Drug Delivery", in Controlled Drug Delivery (2nd Ed.), Robinson et al. (eds.), pp. 623-53 (Marcel Dekker, Inc. 1987); Thorpe, "Antibody Carriers Of Cytotoxic Agents In Cancer Therapy: A Review", in Monoclonal Antibodies '84: Biological And Clinical Applications, Pinchera et al. (eds.), pp. 475-506 (1985); "Analysis, Results, And Future Prospective Of The Therapeutic Use Of Radiolabeled Antibody In Cancer Therapy", in Monoclonal Antibodies For Cancer Detection And Therapy, Baldwin et al. (eds.), pp. 303-16 (Academic Press 1985), and Thorpe et al., "The Preparation And Cytotoxic Properties Of Antibody-Toxin Conjugates", Immunol. Rev., 62:119-58 (1982).

### III. Compositions

In another embodiment, the present invention provides a composition, *e*.*g*., a composition, containing one or a combination of monoclonal antibodies of the present invention, formulated together with a carrier (*e.g.,* a pharmaceutically acceptable carrier). Compositions containing bispecific molecules which comprise an antibody of the present invention are also provided. Compositions may include a combination of multiple (*e.g.,* two or more) isolated antibodies. Each of the antibodies of the composition may bind to a distinct, pre-selected epitope of CD40.

Pharmaceutical compositions of the invention also can be administered in combination therapy, *i.e.,* combined with other agents. For example, the combination therapy can include a composition of the present invention with at least one or more additional therapeutic agents, such as anti-inflammatory agents, DMARDs (disease-modifying anti-rheumatic drugs), immunosuppressive agents, and chemotherapeutics. The pharmaceutical compositions of the invention can also be administered in conjunction with radiation therapy. Co-administration with other antibodies is also encompassed by the invention.

As used herein, the terms "carrier" and "pharmaceutically acceptable carrier" includes any and all solvents, dispersion media, coatings, antibacterial and antifungal agents, isotonic and absorption delaying agents, and the like that are physiologically compatible. Preferably, the carrier is suitable for intravenous, intramuscular, subcutaneous, parenteral, spinal or epidermal administration (*e.g.,* by injection or infusion). Depending on the route of administration, the active compound, *i.e.,* antibody, bispecific and multispecific molecule, may be coated in a material to protect the compound from the action of acids and other natural conditions that may inactivate the compound.

Examples of adjuvants which may be used with the antibodies and constructs of the present invention include: Freund's Incomplete Adjuvant and Complete Adjuvant (Difco Laboratories, Detroit, Mich.); Merck Adjuvant 65 (Merck and Company, Inc., Rahway, N.J.); AS-2 (SmithKline Beecham, Philadelphia, Pa.); aluminum salts such as aluminum hydroxide gel (alum) or aluminum phosphate; salts of calcium, iron or zinc; an insoluble suspension of acylated tyrosine; acylated sugars; cationically or anionically derivatised polysaccharides; polyphosphazenes; biodegradable microspheres; cytokines, such as GM-CSF, interleukin-2, -7, -12, and other like factors; 3D-MPL; CpG oligonucleotide; and monophosphoryl lipid A, for example 3-de-O-acylated monophosphoryl lipid A.

MPL adjuvants are available from Corixa Corporation (Seattle, Wash; see, for example, U.S. Pat. Nos. 4,436,727; 4,877,611; 4,866,034 and 4,912,094). CpG-containing oligonucleotides (in which the CpG dinucleotide is unmethylated) are well known and are described, for example, in WO 96/02555, WO 99/33488 and U.S. Pat. Nos. 6,008,200 and 5,856,462. Immunostimulatory DNA sequences are also described, for example, by Sato et al., Science 273:352, 1996.

Further alternative adjuvants include, for example, saponins, such as Quil A, or derivatives thereof, including QS21 and QS7 (Aquila Biopharmaceuticals Inc., Framingham, Mass.); Escin; Digitonin; or Gypsophila or Chenopodium quinoa saponins; Montanide ISA 720 (Seppic, France); SAF (Chiron, California, United States); ISCOMS (CSL), MF-59 (Chiron); the SBAS series of adjuvants (e.g., SBAS-2 or SBAS-4, available from SmithKline Beecham, Rixensart, Belgium); Detox (Enhanzyn^{™}) (Corixa, Hamilton, Mont.); RC-529 (Corixa, Hamilton, Mont.) and other aminoalkyl glucosaminide 4-phosphates (AGPs); polyoxyethylene ether adjuvants such as those described in WO 99/52549A1; synthetic imidazoquinolines such as imiquimod [S-26308, R-837], (Harrison, et al., Vaccine 19: 1820-1826, 2001; and resiquimod [S-28463, R-848] (Vasilakos, et al., Cellular immunology 204: 64-74, 2000; Schiff bases of carbonyls and amines that are constitutively expressed on antigen presenting cell and T-cell surfaces, such as tucaresol (Rhodes, J. et al., Nature 377: 71-75, 1995); cytokine, chemokine and co-stimulatory molecules as either protein or peptide, including for example pro-inflammatory cytokines such as Interferon, GM-CSF, IL-1 alpha, IL-1 beta, TGF-alpha and TGF-beta, Th1 inducers such as interferon gamma, IL-2, IL-12, IL-15, IL-18 and IL-21, Th2 inducers such as IL-4, IL-5, IL-6, IL-10 and IL-13 and other chemokine and co-stimulatory genes such as MCP-1, MIP-1 alpha, MIP-1 beta, RANTES, TCA-3, CD80, CD86 and CD70; immunostimulatory agents targeting ligands such as CTLA-4 and L-selectin, apoptosis stimulating proteins and peptides such as Fas; synthetic lipid based adjuvants, such as vaxfectin, (Reyes et al., Vaccine 19: 3778-3786, 2001) squalene, alpha-tocopherol, polysorbate 80, DOPC and cholesterol; endotoxin, [LPS], (Beutler, B., Current Opinion in Microbiology 3: 23-30, 2000); ligands that trigger Toll receptors to produce Th1-inducing cytokines, such as synthetic Mycobacterial lipoproteins, Mycobacterial protein p19, peptidoglycan, teichoic acid and lipid A; and CT (cholera toxin, subunits A and B) and LT (heat labile enterotoxin from E. coli, subunits A and B), heat shock protein family (HSPs), and LLO (listeriolysin O; WO 01/72329). These and various further Toll-like Receptor (TLR) agonists are described for example in Kanzler et al, Nature Medicine, May 2007, Vol 13, No 5. A preferred immunostimulatory agent for use in combination with an anti-CD40 antibody of the invention is a TLR3 agonist, such as Poly IC.

A "pharmaceutically acceptable salt" refers to a salt that retains the desired biological activity of the parent compound and does not impart any undesired toxicological effects (see *e.g.,* Berge, S.M., et al. (1977) J. Pharm. Sci. 66:1-19). Examples of such salts include acid addition salts and base addition salts. Acid addition salts include those derived from nontoxic inorganic acids, such as hydrochloric, nitric, phosphoric, sulfuric, hydrobromic, hydroiodic, phosphorous and the like, as well as from nontoxic organic acids such as aliphatic mono- and dicarboxylic acids, phenyl-substituted alkanoic acids, hydroxy alkanoic acids, aromatic acids, aliphatic and aromatic sulfonic acids and the like. Base addition salts include those derived from alkaline earth metals, such as sodium, potassium, magnesium, calcium and the like, as well as from nontoxic organic amines, such as N,N'-dibenzylethylenediamine, N-methylglucamine, chloroprocaine, choline, diethanolamine, ethylenediamine, procaine and the like.

A composition of the present invention can be administered by a variety of methods known in the art. As will be appreciated by the skilled artisan, the route and/or mode of administration will vary depending upon the desired results. The active compounds can be prepared with carriers that will protect the compound against rapid release, such as a controlled release formulation, including implants, transdermal patches, and microencapsulated delivery systems. Biodegradable, biocompatible polymers can be used, such as ethylene vinyl acetate, polyanhydrides, polyglycolic acid, collagen, polyorthoesters, and polylactic acid. Many methods for the preparation of such formulations are patented or generally known to those skilled in the art. *See, e.g.,* Sustained and Controlled Release Drug Delivery Systems, J.R. Robinson, ed., Marcel Dekker, Inc., New York, 1978.

To administer a compound of the invention by certain routes of administration, it may be necessary to coat the compound with, or co-administer the compound with, a material to prevent its inactivation. For example, the compound may be administered to a subject in an appropriate carrier, for example, liposomes, or a diluent. Acceptable diluents include saline and aqueous buffer solutions. Liposomes include water-in-oil-in-water CGF emulsions as well as conventional liposomes (Strejan et al. (1984) J. Neuroimmunol. 7:27).

Carriers include sterile aqueous solutions or dispersions and sterile powders for the extemporaneous preparation of sterile injectable solutions or dispersion. The use of such media and agents for pharmaceutically active substances is known in the art. Except insofar as any conventional media or agent is incompatible with the active compound, use thereof in the pharmaceutical compositions of the invention is contemplated. Supplementary active compounds can also be incorporated into the compositions.

Therapeutic compositions typically must be sterile and stable under the conditions of manufacture and storage. The composition can be formulated as a solution, microemulsion, liposome, or other ordered structure suitable to high drug concentration. The carrier can be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. In many cases, it will be preferable to include isotonic agents, for example, sugars, polyalcohols such as mannitol, sorbitol, or sodium chloride in the composition. Prolonged absorption of the injectable compositions can be brought about by including in the composition an agent that delays absorption, for example, monostearate salts and gelatin.

Sterile injectable solutions can be prepared by incorporating the active compound in the required amount in an appropriate solvent with one or a combination of ingredients enumerated above, as required, followed by sterilization microfiltration. Generally, dispersions are prepared by incorporating the active compound into a sterile vehicle that contains a basic dispersion medium and the required other ingredients from those enumerated above. In the case of sterile powders for the preparation of sterile injectable solutions, the preferred methods of preparation are vacuum drying and freeze-drying (lyophilization) that yield a powder of the active ingredient plus any additional desired ingredient from a previously sterile-filtered solution thereof.

Dosage regimens are adjusted to provide the optimum desired response *(e.g.,* a therapeutic response). For example, a single bolus may be administered, several divided doses may be administered over time or the dose may be proportionally reduced or increased as indicated by the exigencies of the therapeutic situation. For example, the antibodies disclosed herein may be administered once or twice weekly by subcutaneous or intramuscular injection or once or twice monthly by subcutaneous or intramuscular injection.

It is especially advantageous to formulate parenteral compositions in dosage unit form for ease of administration and uniformity of dosage. Dosage unit form as used herein refers to physically discrete units suited as unitary dosages for the subjects to be treated; each unit contains a predetermined quantity of active compound calculated to produce the desired therapeutic effect in association with the required pharmaceutical carrier. The specification for the dosage unit forms of the invention are dictated by and directly dependent on (a) the unique characteristics of the active compound and the particular therapeutic effect to be achieved, and (b) the limitations inherent in the art of compounding such an active compound for the treatment of sensitivity in individuals.

Examples of pharmaceutically-acceptable antioxidants include: (1) water soluble antioxidants, such as ascorbic acid, cysteine hydrochloride, sodium bisulfate, sodium metabisulfite, sodium sulfite and the like; (2) oil-soluble antioxidants, such as ascorbyl palmitate, butylated hydroxyanisole (BHA), butylated hydroxytoluene (BHT), lecithin, propyl gallate, alpha-tocopherol, and the like; and (3) metal chelating agents, such as citric acid, ethylenediamine tetraacetic acid (EDTA), sorbitol, tartaric acid, phosphoric acid, and the like.

For the therapeutic compositions, formulations of the present invention include those suitable for oral, nasal, topical (including buccal and sublingual), rectal, vaginal and/or parenteral administration. The formulations may conveniently be presented in unit dosage form and may be prepared by any methods known in the art of pharmacy. The amount of active ingredient which can be combined with a carrier material to produce a single dosage form will vary depending upon the subject being treated, and the particular mode of administration. The amount of active ingredient which can be combined with a carrier material to produce a single dosage form will generally be that amount of the composition which produces a therapeutic effect. Generally, out of one hundred per cent, this amount will range from about 0.001 per cent to about ninety percent of active ingredient, preferably from about 0.005 per cent to about 70 per cent, most preferably from about 0.01 per cent to about 30 per cent.

Formulations of the present invention which are suitable for vaginal administration also include pessaries, tampons, creams, gels, pastes, foams or spray formulations containing such carriers as are known in the art to be appropriate. Dosage forms for the topical or transdermal administration of compositions of this invention include powders, sprays, ointments, pastes, creams, lotions, gels, solutions, patches and inhalants. The active compound may be mixed under sterile conditions with a pharmaceutically acceptable carrier, and with any preservatives, buffers, or propellants which may be required.

The phrases "parenteral administration" and "administered parenterally" as used herein means modes of administration other than enteral and topical administration, usually by injection, and includes, without limitation, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal, epidural and intrasternal injection and infusion.

Examples of suitable aqueous and nonaqueous carriers which may be employed in the pharmaceutical compositions of the invention include water, ethanol, polyols (such as glycerol, propylene glycol, polyethylene glycol, and the like), and suitable mixtures thereof, vegetable oils, such as olive oil, and injectable organic esters, such as ethyl oleate. Proper fluidity can be maintained, for example, by the use of coating materials, such as lecithin, by the maintenance of the required particle size in the case of dispersions, and by the use of surfactants.

These compositions may also contain adjuvants such as preservatives, wetting agents, emulsifying agents and dispersing agents. Prevention of presence of microorganisms may be ensured both by sterilization procedures, supra, and by the inclusion of various antibacterial and antifungal agents, for example, paraben, chlorobutanol, phenol sorbic acid, and the like. It may also be desirable to include isotonic agents, such as sugars, sodium chloride, and the like into the compositions. In addition, prolonged absorption of the injectable pharmaceutical form may be brought about by the inclusion of agents which delay absorption such as aluminum monostearate and gelatin.

When the compounds are administered as pharmaceuticals, to humans and animals, they may be given alone or as a pharmaceutical composition containing, for example, 0.001 to 90% (more preferably, 0.005 to 70%, such as 0.01 to 30%) of active ingredient in combination with a pharmaceutically acceptable carrier.

Regardless of the route of administration selected, the compounds of the present invention, which may be used in a suitable hydrated form, and/or the pharmaceutical compositions of the present invention, are formulated into pharmaceutically acceptable dosage forms by conventional methods known to those of skill in the art.

Actual dosage levels of the active ingredients in the pharmaceutical compositions of the present invention may be varied so as to obtain an amount of the active ingredient which is effective to achieve the desired therapeutic response for a particular patient, composition, and mode of administration, without being toxic to the patient. The selected dosage level will depend upon a variety of pharmacokinetic factors including the activity of the particular compositions of the present invention employed, or the ester, salt or amide thereof, the route of administration, the time of administration, the rate of excretion of the particular compound being employed, the duration of the treatment, other drugs, compounds and/or materials used in combination with the particular compositions employed, the age, sex, weight, condition, general health and prior medical history of the patient being treated, and like factors well known in the medical arts. A physician or veterinarian having ordinary skill in the art can readily determine and prescribe the effective amount of the pharmaceutical composition required. For example, the physician or veterinarian could start doses of the compounds of the invention employed in the pharmaceutical composition at levels lower than that required in order to achieve the desired therapeutic effect and gradually increase the dosage until the desired effect is achieved. In general, a suitable daily dose of a composition of the invention will be that amount of the compound which is the lowest dose effective to produce a therapeutic effect. Such an effective dose will generally depend upon the factors described above. It is preferred that administration be intravenous, intramuscular, intraperitoneal, or subcutaneous, preferably administered proximal to the site of the target. If desired, the effective daily dose of a therapeutic composition may be administered as two, three, four, five, six or more sub-doses administered separately at appropriate intervals throughout the day, optionally, in unit dosage forms. While it is possible for a compound of the present invention to be administered alone, it is preferable to administer the compound as a pharmaceutical formulation (composition).

Therapeutic compositions can be administered with medical devices known in the art. For example, a therapeutic composition can be administered with a needleless hypodermic injection device, such as the devices disclosed in U.S. Patent Nos. 5,399,163, 5,383,851, 5,312,335, 5,064,413, 4,941,880, 4,790,824, or 4,596,556. Examples of well-known implants and modules include: U.S. Patent No. 4,487,603, which discloses an implantable micro-infusion pump for dispensing medication at a controlled rate; U.S. Patent No. 4.,486,194, which discloses a therapeutic device for administering medicants through the skin; U.S. Patent No. 4,447,233, which discloses a medication infusion pump for delivering medication at a precise infusion rate; U.S. Patent No. 4,447,224, which discloses a variable flow implantable infusion apparatus for continuous drug delivery; U.S. Patent No. 4,439,196, which discloses an osmotic drug delivery system having multi-chamber compartments; and U.S. Patent No. 4,475,196, which discloses an osmotic drug delivery system. Many other such implants, delivery systems, and modules are known to those skilled in the art.

In certain embodiments, the antibodies of the invention can be formulated to ensure proper distribution *in vivo.* For example, the blood-brain barrier (BBB) excludes many highly hydrophilic compounds. To ensure that the therapeutic compounds of the invention cross the BBB (if desired), they can be formulated, for example, in liposomes. For methods of manufacturing liposomes, see, *e.g.,* U.S. Patents 4,522,811; 5,374,548; and 5,399,331. The liposomes may comprise one or more moieties which are selectively transported into specific cells or organs, thus enhance targeted drug delivery (*see, e.g.,* V.V. Ranade (1989) J. Clin. Pharmacol. 29:685). Exemplary targeting moieties include folate or biotin (see, *e.g.,* U.S. Patent 5,416,016 to Low et al.); mannosides (Umezawa et al., (1988) Biochem. Biophys. Res. Commun. 153:1038); antibodies (P.G. Bloeman et al. (1995) FEBS Lett. 357:140; M. Owais et al. (1995) Antimicrob. Agents Chemother. 39:180); surfactant protein A receptor (Briscoe et al. (1995) Am. J. Physiol. 1233:134), different species of which may comprise the formulations of the inventions, as well as components of the invented molecules; p120 (Schreier et al. (1994) J. Biol. Chem. 269:9090); see also K. Keinanen; M.L. Laukkanen (1994) FEBS Lett. 346:123; J.J. Killion; I.J. Fidler (1994) Immunomethods 4:273. In one embodiment of the invention, the therapeutic compounds of the invention are formulated in liposomes; in a more preferred embodiment, the liposomes include a targeting moiety. Therapeutic compounds in the liposomes may be delivered by bolus injection to a site proximal to the tumor or infection. The composition must be fluid to the extent that easy syringability exists. It must be stable under the conditions of manufacture and storage and must be preserved against the contaminating action of microorganisms such as bacteria and fungi.

The ability of a compound to inhibit cancer can be evaluated in an animal model system predictive of efficacy in human tumors. Alternatively, this property of a composition can be evaluated by examining the ability of the compound to inhibit, such inhibition *in vitro* by assays known to the skilled practitioner. A therapeutically effective amount of a therapeutic compound can decrease tumor size, or otherwise ameliorate symptoms in a subject. One of ordinary skill in the art would be able to determine such amounts based on such factors as the subject's size, the severity of the subject's symptoms, and the particular composition or route of administration selected.

The composition must be sterile and fluid to the extent that the composition is deliverable by syringe. In addition to water, the carrier can be an isotonic buffered saline solution, ethanol, polyol (for example, glycerol, propylene glycol, and liquid polyetheylene glycol, and the like), and suitable mixtures thereof. Proper fluidity can be maintained, for example, by use of coating such as lecithin, by maintenance of required particle size in the case of dispersion and by use of surfactants. In many cases, it is preferable to include isotonic agents, for example, sugars, polyalcohols such as mannitol or sorbitol, and sodium chloride in the composition. Long-term absorption of the injectable compositions can be brought about by including in the composition an agent which delays absorption, for example, aluminum monostearate or gelatin.

When the active compound is suitably protected, as described above, the compound may be orally administered, for example, with an inert diluent or an assimilable edible carrier.

### IV. Uses and Methods of the Invention

Antibodies, molecular conjugates, bispecific molecules, and compositions of the present invention may be for use in a method of treating and/or preventing (e.g., immunize against) a variety of diseases and conditions.

One of the primary disease indications is cancer. Types of cancers include, but are not limited to, leukemia, acute lymphocytic leukemia, acute myelocytic leukemia, myeloblasts promyelocyte myelomonocytic monocytic erythroleukemia, chronic leukemia, chronic myelocytic (granulocytic) leukemia, chronic lymphocytic leukemia, mantle cell lymphoma, primary central nervous system lymphoma, Burkitt's lymphoma and marginal zone B cell lymphoma, Polycythemia vera Lymphoma, Hodgkin's disease, non-Hodgkin' s disease, multiple myeloma, Waldenstrom's macroglobulinemia, heavy chain disease, solid tumors, sarcomas, and carcinomas, fibrosarcoma, myxosarcoma, liposarcoma, chrondrosarcoma, osteogenic sarcoma, osteosarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, colon sarcoma, colorectal carcinoma, pancreatic cancer, breast cancer, ovarian cancer, prostate cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, choriocarcinoma, seminoma, embryonal carcinoma, Wilm's tumor, cervical cancer, uterine cancer, testicular tumor, lung carcinoma, small cell lung carcinoma, non small cell lung carcinoma, bladder carcinoma, epithelial carcinoma, glioma, astrocytoma, medulloblastoma, craniopharyngioma, ependymoma, pinealoma, hemangioblastoma, acoustic neuroma, oligodendroglioma, menangioma, melanoma, neuroblastoma, retinoblastoma, nasopharyngeal carcinoma, esophageal carcinoma, basal cell carcinoma, biliary tract cancer, bladder cancer, bone cancer, brain and central nervous system (CNS) cancer, cervical cancer, choriocarcinoma, colorectal cancers, connective tissue cancer, cancer of the digestive system, endometrial cancer, esophageal cancer, eye cancer, head and neck cancer, gastric cancer, intraepithelial neoplasm, kidney cancer, larynx cancer, liver cancer, lung cancer (small cell, large cell), melanoma, neuroblastoma; oral cavity cancer(for example lip, tongue, mouth and pharynx), ovarian cancer, pancreatic cancer, retinoblastoma, rhabdomyosarcoma, rectal cancer; cancer of the respiratory system, sarcoma, skin cancer, stomach cancer, testicular cancer, thyroid cancer, uterine cancer, and cancer of the urinary system. Particular cancers include CD40-expressing tumors selected from the group consisting of chronic lymphocytic leukemia, mantle cell lymphoma, primary central nervous system lymphoma, Burkitt's lymphoma and marginal zone B cell lymphoma.

Antibodies and conjugates of the invention may also be for use in a method of treating bacterial, fungal, viral and parasitic infectious diseases.

When used in therapy, the antibodies of the invention may be administered to a subject directly *(i.e., in vivo),* either alone or with other therapies such as an immunostimulatory agent, a vaccine, chemotherapy or radiation therapy. In all cases, the antibodies, conjugates, bispecifics, compositions, and immunostimulatory agents and other therapies are administered in an effective amount to exert their desired therapeutic effect. The term "effective amount" refers to that amount necessary or sufficient to realize a desired biologic effect. For example, an effective amount could be that amount necessary to eliminate a tumor, cancer, or bacterial, viral or fungal infection. The effective amount for any particular application can vary depending on such factors as the disease or condition being treated, the particular antibody being administered, the size of the subject, or the severity of the disease or condition. One of ordinary skill in the art can empirically determine the effective amount of a particular molecule without necessitating undue experimentation.

Preferred routes of administration include, for example, injection (e.g., subcutaneous, intravenous, parenteral, intraperitoneal, intrathecal). The injection can be in a bolus or a continuous infusion. Other routes of administration include oral administration.

An antibody may be administered in combination with a vaccine antigen, to enhance the immune response against the vaccine antigen, such as a tumor antigen (to thereby enhance the immune response against the tumor) or an antigen from an infectious disease pathogen (to thereby enhance the immune response against the infectious disease pathogen). The vaccine antigen can be any antigen or antigenic composition capable of eliciting an immune response against a tumor or against an infectious disease pathogen such as a virus, a bacteria, a parasite or a fungus. It may also be, for example, a neoantigen such as those derived from sequencing of patients' tumors. The antigen or antigens can be, for example, peptides/proteins, polysaccharides and/or lipids, or may be administered as nucleic acids (such as DNA) coding for peptide or protein antigens which may be expressed in vivo. The antigen or antigens be derived from tumors, such as the various tumor antigens previously disclosed herein. Alternatively, the antigen or antigens can be derived from pathogens such as viruses, bacteria, parasites and/or fungi, such as the various pathogen antigens previously disclosed herein. Additional examples of suitable pathogen antigens include, but are not limited to, the following:
Viral antigens or antigenic determinants can be derived from, for example,: Cytomegalovirus ( especially Human, such as gB or derivatives thereof); Epstein Barr virus (such as gp350); flaviviruses (e.g. Yellow Fever Virus, Dengue Virus, Tick-borne encephalitis virus, Japanese Encephalitis Virus); hepatitis virus such as hepatitis B virus (for example Hepatitis B Surface antigen such as the PreSl, PreS2 and S antigens described in EP-A-414 374; EP-A-0304 578, and EP-A-198474), hepatitis A virus, hepatitis C virus and hepatitis E virus; HIV-1, (such as tat, nef, gpl20 or gpl60); human herpes viruses, such as gD or derivatives thereof or Immediate Early protein such as ICP27 from HSV1 or HSV2; human papilloma viruses (for example HPV6, 11, 16, 18); Influenza virus (whole live or inactivated virus, split influenza virus, grown in eggs or MDCK cells, or Vero cells or whole flu virosomes (as described by Gluck, Vaccine, 1992,10, 915-920) or purified or recombinant proteins thereof, such as NP, NA, HA, or M proteins); measles virus; mumps virus; parainfluenza virus; rabies virus; Respiratory Syncytial virus (such as F and G proteins); rotavirus (including live attenuated viruses); smallpox virus; Varicella Zoster Virus (such as gpI, II and IE63); and the HPV viruses responsible for cervical cancer (for example the early proteins E6 or E7 in fusion with a protein D carrier to form Protein D-E6 or E7 fusions from HPV 16, or combinations thereof; or combinations of E6 or E7 with L2 (see for example WO 96/26277).

Bacterial antigens or antigenic determinants can be derived from, for example,: Bacillus spp., including B. anthracis (e.g., botulinum toxin); Bordetella spp, including B. pertussis (for example pertactin, pertussis toxin, filamenteous hemagglutinin, adenylate cyclase, fimbriae); Borrelia spp., including B. burgdorferi (eg OspA, OspC, DbpA, DbpB), B. garinii (eg OspA, OspC, DbpA, DbpB), B. afzelii (eg OspA, OspC, DbpA, DbpB), B. andersonii (eg OspA, OspC, DbpA, DbpB), B. hermsii; Campylobacter spp, including C. jejuni (for example toxins, adhesins and invasins) and C. coli; Chlamydia spp., including C. trachomatis (eg MOMP, heparin-binding proteins), C. pneumonie (eg MOMP, heparin-binding proteins), C. psittaci; Clostridium spp., including C. tetani (such as tetanus toxin), C. botulinum (for example botulinum toxin), C. difficile (eg clostridium toxins A or B); Corynebacterium spp., including C. diphtheriae (eg diphtheria toxin); Ehrlichia spp., including E. equi and the agent of the Human Granulocytic Ehrlichiosis; Rickettsia spp, including R.rickettsii; Enterococcus spp., including E. faecalis, E. faecium; Escherichia spp, including enterotoxic E. coli (for example colonization factors, heat-labile toxin or derivatives thereof, or heat-stable toxin), enterohemorragic E. coli, enteropathogenic E. coli (for example shiga toxin-like toxin); Haemophilus spp., including H. influenzae type B (eg PRP), non-typable H. influenzae, for example OMP26, high molecular weight adhesins, P5, P6, protein D and lipoprotein D, and fimbrin and fimbrin derived peptides (see for example US 5,843,464); Helicobacter spp, including H. pylori (for example urease, catalase, vacuolating toxin); Pseudomonas spp, including P. aeruginosa; Legionella spp, including L. pneumophila ; Leptospira spp., including L. interrogans; Listeria spp., including L. monocytogenes; Moraxella spp, including M catarrhalis, also known as Branhamella catarrhalis (for example high and low molecular weight adhesins and invasins); Morexella Catarrhalis (including outer membrane vesicles thereof, and OMP106 (see for example WO97/41731)); Mycobacterium spp., including M. tuberculosis (for example ESAT6, Antigen 85A, -B or -C), M. bovis, M. leprae, M. avium, M. paratuberculosis, M. smegmatis; Neisseria spp, including N. gonorrhea and N. meningitidis (for example capsular polysaccharides and conjugates thereof, transferrin-binding proteins, lactoferrin binding proteins, PilC, adhesins); Neisseria mengitidis B (including outer membrane vesicles thereof, and NspA ( see for example WO 96/29412); Salmonella spp, including S. typhi, S. paratyphi, S. choleraesuis, S. enteritidis; Shigella spp, including S. sonnei, S. dysenteriae, S. flexnerii; Staphylococcus spp., including S. aureus, S. epidermidis; Streptococcus spp, including S. pneumonie (eg capsular polysaccharides and conjugates thereof, PsaA, PspA, streptolysin, choline-binding proteins) and the protein antigen Pneumolysin (Biochem Biophys Acta, 1989,67,1007; Rubins et al., Microbial Pathogenesis, 25,337-342), and mutant detoxified derivatives thereof (see for example WO 90/06951; WO 99/03884); Treponema spp., including T. pallidum (eg the outer membrane proteins), T. denticola, T. hyodysenteriae; Vibrio spp, including V. cholera (for example cholera toxin); and Yersinia spp, including Y. enterocolitica (for example a Yop protein), Y. pestis, Y. pseudotuberculosis.

Parasitic/fungal antigens or antigenic determinants can be derived from, for example,: Babesia spp., including B. microti; Candida spp., including C. albicans; Cryptococcus spp., including C. neoformans; Entamoeba spp., including E. histolytica; Giardia spp., including ;G. lamblia; Leshmania spp., including L. major; Plasmodium. faciparum (MSP1, AMA1, MSP3, EBA, GLURP, RAP1, RAP2, Sequestrin, PfEMP1, Pf332, LSA1, LSA3, STARP, SALSA, PfEXPl, Pfs25, Pfs28, PFS27/25, Pfsl6, Pfs48/45, Pfs230 and their analogues in Plasmodium spp.); Pneumocystis spp., including P. ;carinii; Schisostoma spp., including S. mansoni; Trichomonas spp., including T. vaginalis; Toxoplasma spp., including T. gondii (for example SAG2, SAG3, Tg34); Trypanosoma spp., including T. cruzi.

It will be appreciated that in accordance with this aspect of the present invention, antigens and antigenic determinants can be used in many different forms. For example, antigens or antigenic determinants can be present as isolated proteins or peptides (for example in so-called "subunit vaccines") or, for example, as cell-associated or virus-associated antigens or antigenic determinants (for example in either live or killed pathogen strains). Live pathogens will preferably be attenuated in known manner. Alternatively, antigens or antigenic determinants may be generated *in situ* in the subject by use of a polynucleotide coding for an antigen or antigenic determinant (as in so-called "DNA vaccination"), although it will be appreciated that the polynucleotides which can be used with this approach are not limited to DNA, and may also include RNA and modified polynucleotides as discussed above.

When used in therapy, molecular conjugates (i.e., vaccine conjugates) of the invention can be administered to a subject directly *(i.e., in vivo),* either alone or with an immunostimulatory agent. In one aspect, the immunostimulatory agent is linked to the conjugate. Alternatively, the conjugates can be administered to a subject indirectly by first contacting the conjugates (e.g., by culturing or incubating) with APCs, such as dendritic cells, and then administering the cells to the subject (*i.e., ex vivo*)*.* The contacting and delivering of the conjugates to APCs, such that they are processed and presented by the APCs prior to administration, is also referred to as antigen or cell "loading." Techniques for loading antigens to APCs are well known in the art and include, for example, Gunzer and Grabbe, Crit Rev Immunol 21 (1-3):133-45 (2001) and Steinman, Exp Hematol 24(8): 859-62 (1996).

In all cases, the vaccine conjugates and the immunostimulatory agents are administered in an effective amount to exert their desired therapeutic effect.

Antibodies, molecular conjugates, bispecific molecules, and compositions of the invention also can be coadministered with adjuvants and other therapeutic agents. It will be appreciated that the term "coadministered" as used herein includes any or all of simultaneous, separate, or sequential administration of the antibodies and conjugates of the present invention with adjuvants and other agents, including administration as part of a dosing regimen. The antibodies are typically formulated in a carrier alone or in combination with such agents. Examples of such carriers include solutions, solvents, dispersion media, delay agents, emulsions and the like. The use of such media for pharmaceutically active substances is well known in the art. Any other conventional carrier suitable for use with the molecules falls within the scope of the instant invention.

Suitable agents for co-administration with the antibodies, conjugates, bispecifics, and compositions include other antibodies, cytotoxins and/or drugs, as well as adjuvants, immunostimulatory agents and/or immunosuppressive agents. The agent may be a chemotherapeutic agent. Antibodies, bispecifics, and compositions may be administered in combination with radiation.

Chemotherapeutic agents suitable for coadministration with the antibodies and conjugates of the present invention in the treatment of tumors include, for example: taxol, cytochalasin B, gramicidin D, ethidium bromide, emetine, mitomycin, etoposide, tenoposide, vincristine, vinblastine, colchicin, doxorubicin, daunorubicin, dihydroxy anthracin dione, mitoxantrone, mithramycin, actinomycin D, 1-dehydrotestosterone, glucocorticoids, procaine, tetracaine, lidocaine, propranolol, and puromycin and analogs or homologs thereof. Further agents include, for example, antimetabolites (e.g., methotrexate, 6-mercaptopurine, 6-thioguanine, cytarabine, 5-fluorouracil decarbazine), alkylating agents (*e.g.,* mechlorethamine, thioepa chlorambucil, melphalan, carmustine (BSNU) and lomustine (CCNU), cyclothosphamide, busulfan, dibromomannitol, streptozotocin, mitomycin C, and cis-dichlorodiamine platinum (II) (DDP) cisplatin), anthracyclines (e.g., daunorubicin (formerly daunomycin) and doxorubicin), antibiotics (e.g., dactinomycin (formerly actinomycin), bleomycin, mithramycin, and anthramycin (AMC)), and anti-mitotic agents (e.g., vincristine and vinblastine) and temozolomide.

Agents that delete or inhibit immunosuppressive activities, for example, by immune cells (for example regulatory T-cells, NKT cells, macrophages, myeloid-derived suppressor cells, immature or suppressive dendritic cells) or suppressive factors produced by the tumor or host cells in the local microenvironment of the tumor (for example, TGFbeta, indoleamine 2,3 dioxygenase - IDO), may also be administered with the antibodies and conjugates of the present invention. Such agents include antibodies and small molecule drugs such as IDO inhibitors such as 1 methyl tryptophan or derivatives.

Suitable agents for coadministration with the antibodies, conjugates, and bispecifics of the present invention for inducement or enhancement of an immune response include, for example, adjuvants and/or immunostimulatory agents, non-limiting examples of which have been disclosed hereinbefore. A preferred immunostimulatory agent is a TLR3 agonist, such as Poly IC.

### V. Combination Therapies

The anti-CD40 antibodies described herein may be for use in combination therapy, e.g., for use in a method of treating cancer. The methods of combination therapy comprise co-administering an anti-CD40 antibody with one or more additional agents, e.g., small molecule drugs, antibodies or antigen binding portions thereof, and/or protein ligands that are effective in stimulating immune responses to thereby further enhance, stimulate or upregulate immune responses in a subject. Moreover, as shown in the Examples herein, administration of an agonist anti-CD40 antibody and soluble CD40 ligand had a synergic effect in inducing T cell receptor-mediated signals, e.g., as shown by the increase in the expression of CD95 in tumor cells.

For example, an anti-CD40 antibody, e.g., described herein, can be combined with (i) an agonist of a stimulatory (e.g., co-stimulatory) molecule (e.g., receptor or ligand) and/or (ii) an antagonist of an inhibitory signal or molecule (e.g., receptor or ligand) on immune cells, such as T cells, both of which result in amplifying immune responses, such as antigen-specific T cell responses. In certain aspects, an immuno-oncology agent is (i) an agonist of a stimulatory (including a co-stimulatory) molecule (e.g., receptor or ligand) or (ii) an antagonist of an inhibitory (including a co-inhibitory) molecule (e.g., receptor or ligand) on cells involved in innate immunity, e.g., NK cells, and wherein the immuno-oncology agent enhances innate immunity. Such immuno-oncology agents are often referred to as immune checkpoint regulators, e.g., immune checkpoint inhibitor or immune checkpoint stimulator.

In one embodiment, an anti-CD40 antibody is administered with an agent that targets a stimulatory or inhibitory molecule that is a member of the immunoglobulin super family (IgSF). For example, anti-CD40 antibodies, e.g., described herein, may be administered to a subject with an agent that targets a member of the IgSF family to increase an immune response. For example, an anti-CD40 antibody may be administered with an agent that targets (or binds specifically to) a member of the B7 family of membrane-bound ligands that includes B7-1, B7-2, B7-H1 (PD-L1), B7-DC (PD-L2), B7-H2 (ICOS-L), B7-H3, B7-H4, B7-H5 (VISTA), and B7-H6 or a co-stimulatory or co-inhibitory receptor binding specifically to a B7 family member.

An anti-CD40 antibody may also be administered with an agent that targets a member of the TNF and TNFR family of molecules (ligands or receptors), such as CD40 and CD40L (e.g., human CD40 and human CD40L), OX-40, OX-40L, CD70, CD27L, CD30, CD30L, 4-1BBL, CD137, TRAIL/Apo2-L, TRAILR1/DR4, TRAILR2/DR5, TRAELR3, TRAELR4, OPG, RANK, RANKL, TWEAKR/Fn14, TWEAK, BAFFR, EDAR, XEDAR, TACI, APRIL, BCMA, LTβR, LIGHT, DcR3, HVEM, VEGI/TL1A, TRAMP/DR3, EDA1, EDA2, TNFR1, Lymphotoxin α/TNFβ, TNFR2, TNFα, LTβR, Lymphotoxin α 1β2, FAS, FASL, RELT, DR6, TROY, and NGFR (see, e.g., Tansey (2009) Drug Discovery Today 00:1).

T cell responses can be stimulated by a combination of anti-CD40 antibodies described herein, e.g., 3C3 and 3G5, and one or more of an antagonist (inhibitor or blocking agent) of a protein that inhibits T cell activation (e.g., immune checkpoint inhibitors), such as CTLA-4, PD-1, PD-L1, PD-L2, and LAG-3, as described above, and any of the following proteins: TIM-3, Galectin 9, CEACAM-1, BTLA, CD69, Galectin-1, TIGIT, CD113, GPR56, VISTA, B7-H3, B7-H4, 2B4, CD48, GARP, PD1H, LAIR1, TIM-1, and TIM-4, and/or one or more of an agonist of a protein that stimulates T cell activation, such as B7-1, B7-2, CD28, 4-1BB (CD137), 4-1BBL, ICOS, ICOS-L, OX40, OX40L, CD70, CD27, CD40, DR3 and CD28H.

Agents that modulate one of the above proteins and may be combined with agonist anti-CD40 antibodies, e.g., those described herein, for treating cancer, include: Yervoy^{™} (ipilimumab) or Tremelimumab (to CTLA-4), galiximab (to B7.1), BMS-936558/nivolumab (to PD-1), MK-3475/pembrolizumab (to PD-1), AMP224 (to B7DC), BMS-936559 (to B7-H1), MPDL3280A/atezolizumab (to B7-H1), MEDI-570 (to ICOS), AMG557 (to B7H2), MGA271 (to B7H3), IMP321 (to LAG-3), BMS-663513 (to CD137), PF-05082566 (to CD137), CDX-1127 (to CD27), anti-OX40 (Providence Health Services), huMAbOX40L (to OX40L), Atacicept (to TACI), CP-870893 (to CD40), Lucatumumab (to CD40), Dacetuzumab (to CD40), Muromonab-CD3 (to CD3), Ipilumumab (to CTLA-4).

Other molecules that can be combined with agonist anti-CD40 antibodies for the treatment of cancer include antagonists of inhibitory receptors on NK cells or agonists of activating receptors on NK cells. For example, anti-CD40 agonist antibodies can be combined with antagonists of KIR (e.g., lirilumab).

T cell activation is also regulated by soluble cytokines, and anti-CD40 antibodies may be administered to a subject, e.g., having cancer, with antagonists of cytokines that inhibit T cell activation or agonists of cytokines that stimulate T cell activation.

In another embodiment, anti-CD40 antibodies can be used in combination with (i) antagonists (or inhibitors or blocking agents) of proteins of the IgSF family or B7 family or the TNF family that inhibit T cell activation or antagonists of cytokines that inhibit T cell activation (e.g., IL-6, IL-10, TGF-β, VEGF; "immunosuppressive cytokines") and/or (ii) agonists of stimulatory receptors of the IgSF family, B7 family or the TNF family or of cytokines that stimulate T cell activation, for stimulating an immune response, e.g., for treating proliferative diseases, such as cancer.

Other agents for combination therapies include agents that inhibit or deplete macrophages or monocytes, including but not limited to CSF-1R antagonists such as CSF-1R antagonist antibodies including RG7155 (WO11/70024, WO11/107553, WO11/131407, WO13/87699, WO13/119716, WO13/132044) or FPA-008 (WO11/140249; WO13169264; WO14/036357).

Anti-CD40 antibodies may also be administered with agents that inhibit TGF-β signaling.

Additional agents that may be combined with an anti-CD40 antibody include agents that enhance tumor antigen presentation, e.g., dendritic cell vaccines, GM-CSF secreting cellular vaccines, CpG oligonucleotides, and imiquimod, or therapies that enhance the immunogenicity of tumor cells (e.g., anthracyclines).

Other therapies that may be combined with an anti-CD40 antibody include therapies that deplete or block Treg cells, e.g., an agent that specifically binds to CD25.

Another therapy that may be combined with an anti-CD40 antibody is a therapy that inhibits a metabolic enzyme such as indoleamine dioxigenase (IDO), dioxigenase, arginase, or nitric oxide synthetase.

Another class of agents that may be used with an anti-CD40 antibody includes agents that inhibit the formation of adenosine or inhibit the adenosine A2A receptor.

Other therapies that may be combined with an anti-CD40 antibody for treating cancer include therapies that reverse/prevent T cell anergy or exhaustion and therapies that trigger an innate immune activation and/or inflammation at a tumor site.

An anti-CD40 antibody may be combined with more than one immuno-oncology agent, and may be, e.g., combined with a combinatorial approach that targets multiple elements of the immune pathway, such as one or more of the following: a therapy that enhances tumor antigen presentation (e.g., dendritic cell vaccine, GM-CSF secreting cellular vaccines, CpG oligonucleotides, imiquimod); a therapy that inhibits negative immune regulation e.g., by inhibiting CTLA-4 and/or PD1/PD-L1/PD-L2 pathway and/or depleting or blocking Tregs or other immune suppressing cells; a therapy that stimulates positive immune regulation, e.g., with agonists that stimulate the CD-137, OX-40, and/or GITR pathway and/or stimulate T cell effector function; a therapy that increases systemically the frequency of anti-tumor T cells; a therapy that depletes or inhibits Tregs, such as Tregs in the tumor, e.g., using an antagonist of CD25 (e.g., daclizumab) or by ex vivo anti-CD25 bead depletion; a therapy that impacts the function of suppressor myeloid cells in the tumor; a therapy that enhances immunogenicity of tumor cells (e.g., anthracyclines); adoptive T cell or NK cell transfer including genetically modified cells, e.g., cells modified by chimeric antigen receptors (CAR-T therapy); a therapy that inhibits a metabolic enzyme such as indoleamine dioxigenase (IDO), dioxigenase, arginase, or nitric oxide synthetase; a therapy that reverses/prevents T cell anergy or exhaustion; a therapy that triggers an innate immune activation and/or inflammation at a tumor site; administration of immune stimulatory cytokines; or blocking of immuno repressive cytokines.

Agonist anti-CD40 antibodies described herein can be used together with one or more of agonistic agents that ligate positive costimulatory receptors, blocking agents that attenuate signaling through inhibitory receptors, antagonists, and one or more agents that increase systemically the frequency of anti-tumor T cells, agents that overcome distinct immune suppressive pathways within the tumor microenvironment (e.g., block inhibitory receptor engagement (e.g., PD-L1/PD-1 interactions), deplete or inhibit Tregs (e.g., using an anti-CD25 monoclonal antibody (e.g., daclizumab) or by ex vivo anti-CD25 bead depletion), inhibit metabolic enzymes such as IDO, or reverse/prevent T cell anergy or exhaustion) and agents that trigger innate immune activation and/or inflammation at tumor sites.

Methods for stimulating an immune response in a subject may comprise administering to the subject an agonist anti-CD40 molecule, e.g., an antibody, and one or more additional immunostimulatory antibodies, such as an anti-PD-1 antagonist, e.g., antagonist antibody, an anti-PD-L1 antagonist, e.g., antagonist antibody, an antagonist anti-CTLA-4 antagonist, e.g., antagonist antibody and/or an anti-LAG3 antagonist, e.g., an antagonist antibody, such that an immune response is stimulated in the subject, for example to inhibit tumor growth or to stimulate an anti-viral response. The additional immunostimulatory antibody (e.g., an antagonist anti-PD-1, an antagonist anti-PD-L1, an antagonist anti-CTLA-4 and/or an antagonist anti-LAG3 antibody) may be a human antibody.

Methods for treating a hyperproliferative disease (e.g., cancer) may comprise administering an agonist anti-CD40 antibody and an antagonist PD-1 antibody to a subject. The subject may be human. The anti-PD-1 antibody may be a human sequence monoclonal antibody and the anti-CD40 antibody is human sequence monoclonal antibody, such as an antibody comprising the CDRs or variable regions of 3C3 and 3G5 described herein or another agonist anti-CD40 antibody described herein.

Suitable PD-1 antagonists for use in the methods described herein, include, without limitation, ligands, antibodies (e.g., monoclonal antibodies and bispecific antibodies), and multivalent agents. In one embodiment, the PD-1 antagonist is a fusion protein, e.g., an Fc fusion protein, such as AMP-244. In one embodiment, the PD-1 antagonist is an anti-PD-1 or anti-PD-L1 antibody.

An exemplary anti-PD-1 antibody is nivolumab (BMS-936558) or an antibody that comprises the CDRs or variable regions of one of antibodies 17D8, 2D3, 4H1, 5C4, 7D3, 5F4 and 4A11 described in WO 2006/121168. In certain embodiments, an anti-PD1 antibody is MK-3475 (Lambrolizumab) described in WO2012/145493; and AMP-514 described in WO 2012/145493. Further known PD-1 antibodies and other PD-1 inhibitors include those described in WO 2009/014708, WO 03/099196, WO 2009/114335, WO 2011/066389, WO 2011/161699, WO 2012/145493, U.S. Patent Nos. 7,635,757 and 8,217,149, and U.S. Patent Publication No. 2009/0317368. Any of the anti-PD-1 antibodies disclosed in WO2013/173223 may also be used. An anti-PD-1 antibody that competes for binding with, and/or binds to the same epitope on PD-1 as, as one of these antibodies may also be used in combination treatments. Another approach to target the PD-1 receptor is the recombinant protein composed of the extracellular domain of PD-L2 (B7-DC) fused to the Fc portion of IgG1, called AMP-224.

Methods for treating a hyperproliferative disease (e.g., cancer), may comprise administering an agonist anti-CD40 antibody and an antagonist PD-L1 antibody to a subject. The subject may be human. The anti-PD-L1 antibody may be a human sequence monoclonal antibody and the anti-CD40 antibody may be a human sequence monoclonal antibody, such as an antibody comprising the CDRs or variable regions of 3C3 and 3G5 described herein or another agonist anti-CD40 antibody described herein.

The anti-PD-L1 antibody may be BMS-936559 (referred to as 12A4 in WO 2007/005874 and US Patent No. 7,943,743), or an antibody that comprises the CDRs or variable regions of 3G10, 12A4, 10A5, 5F8, 10H10, 1B12, 7H1, 11E6, 12B7 and 13G4, which are described in PCT Publication WO 07/005874 and US Patent No. 7,943,743. An anti-PD-L1 antibody may be MEDI4736 (also known as Anti-B7-H1), MPDL3280A (also known as RG7446), MSB0010718C (WO2013/79174), or rHigM12B7. Any of the anti-PD-L1 antibodies disclosed in WO2013/173223, WO2011/066389, WO2012/145493, U.S. Patent Nos. 7,635,757 and 8,217,149 and U.S. Publication No. 2009/145493 may also be used. Anti-PD-L1 antibodies that compete with and/or bind to the same epitope as that of any of these antibodies may also be used in combination treatments.

Methods for treating a hyperproliferative disease (e.g., cancer), may comprise administering an anti-CD40 antibody described herein and a CTLA-4 antagonist antibody to a subject. The subject may be human. The anti-CTLA-4 antibody may be an antibody selected from the group of: Yervoy^{™} (ipilimumab or antibody 10D1, described in PCT Publication WO 01/14424), tremelimumab (formerly ticilimumab, CP-675,206), monoclonal or an anti-CTLA-4 antibody described in any of the following publications: WO 98/42752; WO 00/37504; U.S. Pat. No. 6,207,156; Hurwitz et al. (1998) Proc. Natl. Acad. Sci. USA 95(17): 10067-10071; Camacho et al. (2004) J. Clin. Oncology 22(145): Abstract No. 2505 (antibody CP-675206); and Mokyr et al. (1998) Cancer Res. 58:5301-5304. Any of the anti-CTLA-4 antibodies disclosed in WO2013/173223 may also be used.

Methods for treating a hyperproliferative disease (e.g., cancer), may comprise administering an anti-CD40 antibody and an anti-LAG-3 antibody to a subject. The subject may be human. The anti-PD-L1 antibody may be a human sequence monoclonal antibody and the anti-CD40 antibody may be a human sequence monoclonal antibody, such as an antibody comprising the CDRs or variable regions of 3C3 or 3G5 described herein or another agonist anti-CD40 antibody described herein. Examples of anti-LAG3 antibodies include antibodies comprising the CDRs or variable regions of antibodies 25F7, 26H10, 25E3, 8B7, 11F2 or 17E5, which are described in U.S. Patent Publication No. US2011/0150892, WO10/19570 and WO2014/008218. An anti-LAG-3 antibody may be BMS-986016. Other art recognized anti-LAG-3 antibodies that can be used include IMP731 and IMP-321, described in US 2011/007023, WO08/132601, and WO09/44273. Anti-LAG-3 antibodies that compete with and/or bind to the same epitope as that of any of these antibodies may also be used in combination treatments.

Administration of anti-CD40 antibodies described herein and antagonists, e.g., antagonist antibodies, to one or more second target antigens such as LAG-3 and/or CTLA-4 and/or PD-1 and/or PD-L1 can enhance the immune response to cancerous cells in the patient. Cancers whose growth may be inhibited using the antibodies of the instant disclosure include cancers typically responsive to immunotherapy and those that are not typically responsive to immunotherapy. Representative examples of cancers for treatment with the combination therapy of the instant disclosure include those cancers listed herein.

The combination of therapeutic antibodies discussed herein may be administered concurrently as a single composition in a pharmaceutically acceptable carrier, or concurrently as separate compositions with each antibody in a pharmaceutically acceptable carrier. The combination of therapeutic antibodies may be be administered sequentially. For example, an anti-CTLA-4 antibody and an anti-CD40 antibody can be administered sequentially, such as anti-CTLA-4 antibody being administered first and anti-CD40 antibody second, or anti-CD40 antibody being administered first and anti-CTLA-4 antibody second. Additionally or alternatively, an anti-PD-1 antibody and an anti-CD40 antibody can be administered sequentially, such as anti-PD-1 antibody being administered first and anti-CD40 antibody second, or anti-CD40 antibody being administered first and anti-PD-1 antibody second. Additionally or alternatively, an anti-PD-L1 antibody and an anti-CD40 antibody can be administered sequentially, such as anti-PD-L1 antibody being administered first and anti-CD40 antibody second, or anti-CD40 antibody being administered first and anti-PD-L1 antibody second. Additionally or alternatively, an anti-LAG-3 antibody and an anti-CD40 antibody can be administered sequentially, such as anti-LAG-3 antibody being administered first and anti-CD40 antibody second, or anti-CD40 antibody being administered first and anti-LAG-3 antibody second.

Furthermore, if more than one dose of the combination therapy is administered sequentially, the order of the sequential administration can be reversed or kept in the same order at each time point of administration, sequential administrations can be combined with concurrent administrations, or any combination thereof. For example, the first administration of a combination anti-CTLA-4 antibody and anti-CD40 antibody can be concurrent, the second administration can be sequential with anti-CTLA-4 antibody first and anti-CD40 antibody second, and the third administration can be sequential with anti-CD40 antibody first and anti-CTLA-4 antibody second, etc. Additionally or alternatively, the first administration of a combination anti-PD-1 antibody and anti-CD40 antibody can be concurrent, the second administration can be sequential with anti-PD-1 antibody first and anti-CD40 antibody second, and the third administration can be sequential with anti-CD40 antibody first and anti-PD-1 antibody second, etc. Additionally or alternatively, the first administration of a combination anti-PD-L1 antibody and anti-CD40 antibody can be concurrent, the second administration can be sequential with anti-PD-L1 antibody first and anti-CD40 antibody second, and the third administration can be sequential with anti-CD40 antibody first and anti-PD-L1 antibody second, etc. Additionally or alternatively, the first administration of a combination anti-LAG-3 antibody and anti-CD40 antibody can be concurrent, the second administration can be sequential with anti-LAG-3 antibody first and anti-CD40 antibody second, and the third administration can be sequential with anti-CD40 antibody first and anti-LAG-3 antibody second, etc. Another representative dosing scheme can involve a first administration that is sequential with anti-CD40 first and anti-CTLA-4 antibody (and/or anti-PD-1 antibody and/or anti-PD-L1 antibody and/or anti-LAG-3 antibody) second, and subsequent administrations may be concurrent.

A subject having a disease that may benefit from stimulation of the immune system, e.g., cancer or an infectious disease, may be treated by administration to the subject of an anti-CD40 antibody and an immuno-oncology agent, wherein the immuno-oncology agent is a CD137 (4-1BB) agonist, such as an agonistic CD137 antibody. Suitable CD137 antibodies include, for example, urelumab or PF-05082566 (WO12/32433).

A subject having a disease that may benefit from stimulation of the immune system, e.g., cancer or an infectious disease, may be treated by administration to the subject of an anti-CD40 antibody and an immuno-oncology agent, wherein the immuno-oncology agent is an OX40 agonist, such as an agonistic OX40 antibody. Suitable OX40 antibodies include, for example, MEDI-6383, MEDI-6469 or MOXR0916 (RG7888; WO06/029879).

A subject having a disease that may benefit from stimulation of the immune system, e.g., cancer or an infectious disease, may be treated by administration to the subject of an anti-CD40 antibody and an immuno-oncology agent, wherein the immuno-oncology agent is a second CD40 agonist, such as another agonistic CD40 antibody.

A subject having a disease that may benefit from stimulation of the immune system, e.g., cancer or an infectious disease, may be treated by administration to the subject of an anti-CD40 antibody and an immuno-oncology agent, wherein the immuno-oncology agent is a CD27 agonist, such as an agonistic CD27 antibody. Suitable CD27 antibodies include, for example, varlilumab (CDX-1127).

A subject having a disease that may benefit from stimulation of the immune system, e.g., cancer or an infectious disease, may be treated by administration to the subject of an anti-CD40 antibody and an immuno-oncology agent, wherein the immuno-oncology agent is MGA271 (to B7H3) (WO11/109400).

A subject having a disease that may benefit from stimulation of the immune system, e.g., cancer or an infectious disease, may be treated by administration to the subject of an anti-CD40 antibody and an immuno-oncology agent, wherein the immuno-oncology agent is a KIR antagonist, such as lirilumab.

A subject having a disease that may benefit from stimulation of the immune system, e.g., cancer or an infectious disease, may be treated by administration to the subject of an anti-CD40 antibody and an immuno-oncology agent, wherein the immuno-oncology agent is an IDO antagonist. Suitable IDO antagonists include, for example, INCB-024360 (WO2006/122150, WO07/75598, WO08/36653, WO08/36642), indoximod, NLG-919 (WO09/73620, WO09/1156652, WO11/56652, WO12/142237) or F001287.

A subject having a disease that may benefit from stimulation of the immune system, e.g., cancer or an infectious disease, imay be treated by administration to the subject of an anti-CD40 antibody and an immuno-oncology agent, wherein the immuno-oncology agent is a Toll-like receptor agonist, e.g., a TLR2/4 agonist (e.g., Bacillus Calmette-Guerin); a TLR7 agonist (e.g., Hiltonol or Imiquimod); a TLR7/8 agonist (e.g., Resiquimod); or a TLR9 agonist (e.g., CpG7909).

A subject having a disease that may benefit from stimulation of the immune system, e.g., cancer or an infectious disease, may be treated by administration to the subject of an anti-CD40 antibody and an immuno-oncology agent, wherein, the immuno-oncology agent is a TGF-β inhibitor, e.g., GC1008, LY2157299, TEW7197, or IMC-TR1.

An anti-CD40 antibody may be sequentially administered prior to administration of a second agent, e.g., an immuno-oncology agent. An anti-CD40 antibody may be administered concurrently with the second agent, e.g., an immunology-oncology agent. An anti-CD40 antibody may be sequentially administered after administration of the second agent. The administration of the two agents may start at times that are, e.g., 30 minutes, 60 minutes, 90 minutes, 120 minutes, 3 hours, 6 hours, 12 hours, 24 hours, 36 hours, 48 hours, 3 days, 5 days, 7 days, or one or more weeks apart, or administration of the second agent may start, e.g., 30 minutes, 60 minutes, 90 minutes, 120 minutes, 3 hours, 6 hours, 12 hours, 24 hours, 36 hours, 48 hours, 3 days, 5 days, 7 days, or one or more weeks after the first agent has been administered.

An anti-CD40 antibody and a second agent, e.g., an immuno-oncology agent, may be administered simultaneously, e.g., are infused simultaneously, e.g., over a period of 30 or 60 minutes, to a patient. Alternatively, the anti-CD40 antibody may be co-formulated with a second agent, e.g., an immuno-oncology agent.

Optionally, the anti-CD40 is administered as the sole immunotherapeutic agent, or a combination of the anti-CD40 antibody and one or more additional immunotherapeutic antibodies (e.g., anti-CTLA-4 and/or anti-PD-1 and/or anti-PD-L1 and/or anti-LAG-3 antibody) can be further combined with an immunogenic agent, such as cancerous cells, purified tumor antigens (including recombinant proteins, peptides, and carbohydrate molecules), cells, and cells transfected with genes encoding immune stimulating cytokines (He et al. (2004) J. Immunol. 173:4919-28). Non-limiting examples of tumor vaccines that can be used include peptides of melanoma antigens, such as peptides of gp100, MAGE antigens, Trp-2, MART1 and/or tyrosinase, or tumor cells transfected to express the cytokine GM-CSF (discussed further below). The anti-CD40 antibody and one or more additional antibodies (e.g., anti-CTLA-4 and/or anti-PD-1 and/or anti-PD-L1 and/or anti-LAG-3 antibodies) can also be further combined with standard cancer treatments. For example, the anti-CD40 antibody and one or more additional antibodies (e.g., anti-CTLA-4 and/or anti-PD-1 and/or anti-PD-L1 and/or anti-LAG-3 antibodies) can be effectively combined with chemotherapeutic regimes. In these instances, it is possible to reduce the dose of other chemotherapeutic reagent administered with the combination of the instant disclosure (Mokyr et al. (1998) Cancer Research 58: 5301-5304). An example of such a combination is a combination of anti-CD40 agonist antibody (with or without and an additional antibody, such as anti-CTLA-4 antibodies and/or anti-PD-1 antibodies and/or anti-PD-L1 antibodies and/or anti-LAG-3 antibodies) in combination with decarbazine for the treatment of melanoma. Another example is a combination of anti-CD40 antibody (with or without anti-CTLA-4 antibodies and/or anti-PD-1 antibodies and/or anti-PD-L1 antibodies and/or LAG-3 antibodies) in combination with interleukin-2 (IL-2) for the treatment of melanoma. The scientific rationale behind the combined use of an anti-CD40 antibody and anti-CTLA-4 and/or anti-PD-1 and/or anti-PD-L1 and/or anti-LAG-3 antibodies with chemotherapy is that cell death, which is a consequence of the cytotoxic action of most chemotherapeutic compounds, should result in increased levels of tumor antigen in the antigen presentation pathway. Other combination therapies that may result in synergy with an anti-CD40 antibody (with or without an anti-CTLA-4 and/or anti-PD-1 and/or anti-PD-L1 and/or anti-LAG-3 antibody) include radiation, surgery, or hormone deprivation. Each of these protocols creates a source of tumor antigen in the host. Angiogenesis inhibitors can also be combined with a combined an anti-CD40 antibody and an anti-CTLA-4 antibody and/or anti-PD-1 antibody and/or anti-PD-L1 antibody and/or anti-LAG-3 antibody. Inhibition of angiogenesis leads to tumor cell death, which can be a source of tumor antigen fed into host antigen presentation pathways.

An anti-CD40 agonist antibody as sole immunotherapeutic agent, or a combination of CD40 agonistic and CTLA-4 and/or PD-1 and/or PD-L1 and/or LAG-3 blocking antibodies also can be used in combination with bispecific antibodies that target Fcα or Fcγ receptor-expressing effector cells to tumor cells (see, e.g., U.S. Pat. Nos. 5,922,845 and 5,837,243). Bispecific antibodies can be used to target two separate antigens. The T cell arm of these responses would be augmented by the use of a combined anti-CD40 antibody and anti-CTLA-4 antibody and/or anti-PD-1 antibody and/or anti-PD-L1 antibody and/or anti-LAG-3 antibody.

An anti-CD40 agonist antibody as the sole immunotherapeutic agent or a combination of an anti-CD40 antibody and additional immunostimulating agent, e.g., anti-CTLA-4 antibody and/or anti-PD-1 antibody and/or anti-PD-L1 antibody and/or LAG-3 agent, e.g., antibody, can be used in conjunction with an anti-neoplastic antibody, such as Rituxan^{®} (rituximab), Herceptin^{®} (trastuzumab), Bexxar^{®} (tositumomab), Zevalin^{®} (ibritumomab), Campath^{®} (alemtuzumab), Lymphocide^{®} (eprtuzumab), Avastin^{®} (bevacizumab), and Tarceva^{®} (erlotinib), and the like. By way of example and not wishing to be bound by theory, treatment with an anti-cancer antibody or an anti-cancer antibody conjugated to a toxin can lead to cancer cell death (e.g., tumor cells) which would potentiate an immune response mediated by the immunostimulating agent, e.g., CD40, CTLA-4, PD-1, PD-L1 or LAG-3 agent, e.g., antibody. Treatment of a hyperproliferative disease (e.g., a cancer tumor) may include an anti-cancer agent, e.g., antibody, in combination with anti-CD40 and optionally an additional immunostimulating agent, e.g., anti-CTLA-4 and/or anti-PD-1 and/or anti-PD-L1 and/or anti-LAG-3 agent, e.g., antibody, concurrently or sequentially or any combination thereof, which can potentiate an anti-tumor immune responses by the host.

Tumors evade host immune surveillance by a large variety of mechanisms. Many of these mechanisms may be overcome by the inactivation of proteins, which are expressed by the tumors and which are immunosuppressive. These include, among others, TGF-β (Kehrl et al. (1986) J. Exp. Med. 163: 1037-1050), IL-10 (Howard & O'Garra (1992) Immunology Today 13: 198-200), and Fas ligand (Hahne et al. (1996) Science 274: 1363-1365). Antibodies to each of these entities can be further combined with an anti-CD40 antibody with or without an additional immunostimulating agent, e.g., an anti-CTLA-4 and/or anti-PD-1 and/or anti-PD-L1 and/or anti-LAG-3 agent, such as antibody, to counteract the effects of immunosuppressive agents and favor anti-tumor immune responses by the host.

Other agents, e.g., antibodies, that can be used to activate host immune responsiveness can be further used in combination with an anti-CD40 antibody with or without an additional immunostimulating agent, such as anti-CTLA-4 and/or anti-PD-1 and/or anti-PD-L1 and/or anti-LAG-3 antibody. These include molecules on the surface of dendritic cells that activate DC function and antigen presentation. Anti-CD40 antibodies (Ridge et al., supra) can be used in conjunction with an anti-CD40 antibody and optionally an additional immunostimulating agent, e.g., an anti-CTLA-4 and/or anti-PD-1 and/or anti-PD-L1 and/or anti-LAG-3 agent, e.g., antibody. Other activating antibodies to T cell costimulatory molecules Weinberg et al., *supra,* Melero et al. *supra,* Hutloff et al., *supra,* may also provide for increased levels of T cell activation.

As discussed above, bone marrow transplantation is currently being used to treat a variety of tumors of hematopoietic origin. Anti-CD40 immunotherapy alone or combined with an anti-CTLA-4 antibody and/or anti-PD-1 antibody and/or anti-PD-L1 antibody and/or anti-LAG-3 antibody can be used to increase the effectiveness of the donor engrafted tumor specific T cells.

Several experimental treatment protocols involve *ex vivo* activation and expansion of antigen specific T cells and adoptive transfer of these cells into recipients in order to antigen-specific T cells against tumor (Greenberg & Riddell, supra). These methods can also be used to activate T cell responses to infectious agents such as CMV. *Ex vivo* activation in the presence of anti-CD40 with or without an additional immunostimulating therapy, e.g., anti-CTLA-4 and/or anti-PD-1 and/or anti-PD-L1 and/or anti-LAG-3 antibodies can be expected to increase the frequency and activity of the adoptively transferred T cells.

Provided herein are methods for altering an adverse event associated with treatment of a hyperproliferative disease (e.g., cancer) with an immunostimulatory agent, comprising administering an anti-CD40 antibody with or without anti-CTLA-4 and/or anti-PD-1 and/or anti-PD-L1 and/or anti-LAG-3 agent, e.g., antibody, to a subject. For example, the methods described herein provide for a method of reducing the incidence of immunostimulatory therapeutic antibody-induced colitis or diarrhea by administering a non-absorbable steroid to the patient. As used herein, a "non-absorbable steroid" is a glucocorticoid that exhibits extensive first pass metabolism such that, following metabolism in the liver, the bioavailability of the steroid is low, i.e., less than about 20%.The non-absorbable steroid may be budesonide. Budesonide is a locally-acting glucocorticosteroid, which is extensively metabolized, primarily by the liver, following oral administration. ENTOCORT EC^{®} (Astra-Zeneca) is a pH- and time-dependent oral formulation of budesonide developed to optimize drug delivery to the ileum and throughout the colon. ENTOCORT EC^{®} is approved in the U.S. for the treatment of mild to moderate Crohn's disease involving the ileum and/or ascending colon. The usual oral dosage of ENTOCORT EC^{®} for the treatment of Crohn's disease is 6 to 9 mg/day. ENTOCORT EC^{®} is released in the intestines before being absorbed and retained in the gut mucosa. Once it passes through the gut mucosa target tissue, ENTOCORT EC^{®} is extensively metabolized by the cytochrome P450 system in the liver to metabolites with negligible glucocorticoid activity. Therefore, the bioavailability is low (about 10%). The low bioavailability of budesonide results in an improved therapeutic ratio compared to other glucocorticoids with less extensive first-pass metabolism. Budesonide results in fewer adverse effects, including less hypothalamic-pituitary suppression, than systemically-acting corticosteroids. However, chronic administration of ENTOCORT EC^{®} can result in systemic glucocorticoid effects such as hypercorticism and adrenal suppression. See PDR 58^{th} ed. 2004; 608-610.

The anti-CD40 antibody, with or without immunostimulatory therapeutic antibodies anti-CD40 and optionally anti-CTLA-4 and/or anti-PD-1 and/or anti-PD-L1 and/or anti-LAG-3 antibodies, in conjunction with a non-absorbable steroid may be further combined with a salicylate. Salicylates include 5-ASA agents such as, for example: sulfasalazine (AZULFIDINE^{®}, Pharmacia & UpJohn); olsalazine (DIPENTUM^{®}, Pharmacia & UpJohn); balsalazide (COLAZAL^{®}, Salix Pharmaceuticals, Inc.); and mesalamine (ASACOL^{®}, Procter & Gamble Pharmaceuticals; PENTASA^{®}, Shire US; CANASA^{®}, Axcan Scandipharm, Inc.; ROWASA^{®}, Solvay).

A salicylate may be administered in combination with anti-CD40, with or without anti-CTLA-4 and/or anti-PD-1 and/or anti-PD-L1 and/or LAG-3 antibodies, and a non-absorbable steroid for the purpose of decreasing the incidence of colitis induced by the immunostimulatory antibodies. Thus, for example, methods for reducing the incidence of colitis induced by the immunostimulatory antibodies described herein encompass administering a salicylate and a non-absorbable concurrently or sequentially (e.g., a salicylate is administered 6 hours after a non-absorbable steroid), or any combination thereof. Further, a salicylate and a non-absorbable steroid can be administered by the same route (e.g., both are administered orally) or by different routes (e.g., a salicylate is administered orally and a non-absorbable steroid is administered rectally), which may differ from the route(s) used to administer the anti-CD40 and anti-CTLA-4 and/or anti-PD-1 and/or anti-PD-L1 and/or anti-LAG-3 antibodies.

The anti-CD40 antibodies and combination antibody therapies described herein may also be used in conjunction with other well known therapies that are selected for their particular usefulness against the indication being treated (e.g., cancer). Combinations of the anti-CD40 antibodies described herein may be used sequentially with known pharmaceutically acceptable agent(s).

For example, the anti-CD40 antibodies and combination antibody therapies described herein can be used in combination (e.g., simultaneously or separately) with an additional treatment, such as irradiation, chemotherapy (e.g., using camptothecin (CPT-11), 5-fluorouracil (5-FU), cisplatin, doxorubicin, irinotecan, paclitaxel, gemcitabine, cisplatin, paclitaxel, carboplatin-paclitaxel (Taxol), doxorubicin, 5-fu, or camptothecin + apo2l/TRAIL (a 6X combo)), one or more proteasome inhibitors (e.g., bortezomib or MG132), one or more Bcl-2 inhibitors (e.g., BH3I-2' (bcl-xl inhibitor), indoleamine dioxygenase-1 inhibitor (e.g., INCB24360, indoximod, NLG-919, or F001287), AT-101 (R-(-)-gossypol derivative), ABT-263 (small molecule), GX-15-070 (obatoclax), or MCL-1 (myeloid leukemia cell differentiation protein-1) antagonists), iAP (inhibitor of apoptosis protein) antagonists (e.g., smac7, smac4, small molecule smac mimetic, synthetic smac peptides (see Fulda et al., Nat Med 2002;8:808-15), ISIS23722 (LY2181308), or AEG-35156 (GEM-640)), HDAC (histone deacetylase) inhibitors, anti-CD20 antibodies (e.g., rituximab), angiogenesis inhibitors (e.g., bevacizumab), anti-angiogenic agents targeting VEGF and VEGFR (e.g., Avastin), synthetic triterpenoids (see Hyer et al., Cancer Research 2005;65:4799-808), c-FLIP (cellular FLICE-inhibitory protein) modulators (e.g., natural and synthetic ligands of PPARγ (peroxisome proliferator-activated receptor γ), 5809354 or 5569100), kinase inhibitors (e.g., Sorafenib), Trastuzumab, Cetuximab, Temsirolimus, mTOR inhibitors such as rapamycin and temsirolimus, Bortezomib, JAK2 inhibitors, HSP90 inhibitors, PI3K-AKT inhibitors, Lenalildomide, GSK3β inhibitors, IAP inhibitors and/or genotoxic drugs.

The anti-CD40 antibodies and combination antibody therapies described herein can further be used in combination with one or more anti-proliferative cytotoxic agents. Classes of compounds that may be used as anti-proliferative cytotoxic agents include, but are not limited to, the following:
Alkylating agents (including, without limitation, nitrogen mustards, ethylenimine derivatives, alkyl sulfonates, nitrosoureas and triazenes): Uracil mustard, Chlormethine, Cyclophosphamide (CYTOXAN^{™}) fosfamide, Melphalan, Chlorambucil, Pipobroman, Triethylenemelamine, Triethylenethiophosphoramine, Busulfan, Carmustine, Lomustine, Streptozocin, Dacarbazine, and Temozolomide.

Antimetabolites (including, without limitation, folic acid antagonists, pyrimidine analogs, purine analogs and adenosine deaminase inhibitors): Methotrexate, 5-Fluorouracil, Floxuridine, Cytarabine, 6-Mercaptopurine, 6-Thioguanine, Fludarabine phosphate, Pentostatine, and Gemcitabine.

Suitable anti-proliferative agents for combining with agonist anti-CD40 antibodies, without limitation, taxanes, paclitaxel (paclitaxel is commercially available as TAXOL^{™}), docetaxel, discodermolide (DDM), dictyostatin (DCT), Peloruside A, epothilones, epothilone A, epothilone B, epothilone C, epothilone D, epothilone E, epothilone F, furanoepothilone D, desoxyepothilone Bl, [17]-dehydrodesoxyepothilone B, [18]dehydrodesoxyepothilones B, C12,13-cyclopropyl-epothilone A, C6-C8 bridged epothilone A, trans-9,10-dehydroepothilone D, cis-9,10-dehydroepothilone D, 16-desmethylepothilone B, epothilone B10, discoderomolide, patupilone (EPO-906), KOS-862, KOS-1584, ZK-EPO, ABJ-789, XAA296A (Discodermolide), TZT-1027 (soblidotin), ILX-651 (tasidotin hydrochloride), Halichondrin B, Eribulin mesylate (E-7389), Hemiasterlin (HTI-286), E-7974, Cyrptophycins, LY-355703, Maytansinoid immunoconjugates (DM-1), MKC-1, ABT-751, T1-38067, T-900607, SB-715992 (ispinesib), SB-743921, MK-0731, STA-5312, eleutherobin, 17beta-acetoxy-2-ethoxy-6-oxo-B-homo-estra-1,3,5(10)-trien-3-ol, cyclostreptin, isolaulimalide, laulimalide, 4-epi-7-dehydroxy-14,16-didemethyl-(+)-discodermolides, and cryptothilone 1, in addition to other microtubuline stabilizing agents known in the art.

In cases where it is desirable to render aberrantly proliferative cells quiescent in conjunction with or prior to treatment with anti-CD40 antibodies described herein, hormones and steroids (including synthetic analogs), such as 17a-Ethinylestradiol, Diethylstilbestrol, Testosterone, Prednisone, Fluoxymesterone, Dromostanolone propionate, Testolactone, Megestrolacetate, Methylprednisolone, Methyl-testosterone, Prednisolone, Triamcinolone, Chlorotrianisene, Hydroxyprogesterone, Aminoglutethimide, Estramustine, Medroxyprogesteroneacetate, Leuprolide, Flutamide, Toremifene, ZOLADEX^{™}, can also be administered to the patient. When employing the methods or compositions described herein, other agents used in the modulation of tumor growth or metastasis in a clinical setting, such as antimimetics, can also be administered as desired.

Methods for the safe and effective administration of chemotherapeutic agents are known to those skilled in the art. In addition, their administration is described in the standard literature. For example, the administration of many of the chemotherapeutic agents is described in the Physicians' Desk Reference (PDR), e.g., 1996 edition (Medical Economics Company, Montvale, N.J. 07645-1742, USA).

The chemotherapeutic agent(s) and/or radiation therapy can be administered according to therapeutic protocols well known in the art. It will be apparent to those skilled in the art that the administration of the chemotherapeutic agent(s) and/or radiation therapy can be varied depending on the disease being treated and the known effects of the chemotherapeutic agent(s) and/or radiation therapy on that disease. Also, in accordance with the knowledge of the skilled clinician, the therapeutic protocols (e.g., dosage amounts and times of administration) can be varied in view of the observed effects of the administered therapeutic agents on the patient, and in view of the observed responses of the disease to the administered therapeutic agents.

### VI. Outcomes

As shown in the Examples herein, co-administration of an anti-CD40 antibody with one or more additional therapeutic agents (e.g., soluble CD40 ligand or another antibody, such as an anti-PD-1 antibody, an anti-PD-L1 antibody, an anti-CTLA-4 antibody, and/or an anti-LAG-3 antibody) provides improved efficacy compared to treatment with the antibody alone or with the one or more additional therapeutic agents in the absence of antibody therapy. Preferably, a combination of an anti-CD40 antibody with one or more additional therapeutic agents exhibits therapeutic synergy.

"Therapeutic synergy" refers to a phenomenon where treatment of patients with a combination of therapeutic agents manifests a therapeutically superior outcome to the outcome achieved by each individual constituent of the combination used at its optimum dose (T. H. Corbett et al., 1982, Cancer Treatment Reports, 66, 1187). In this context a therapeutically superior outcome is one in which the patients either a) exhibit fewer incidences of adverse events while receiving a therapeutic benefit that is equal to or greater than that where individual constituents of the combination are each administered as monotherapy at the same dose as in the combination, or b) do not exhibit dose-limiting toxicities while receiving a therapeutic benefit that is greater than that of treatment with each individual constituent of the combination when each constituent is administered in at the same doses in the combination(s) as is administered as individual components. In xenograft models, a combination, used at its maximum tolerated dose, in which each of the constituents will be present at a dose generally not exceeding its individual maximum tolerated dose, manifests therapeutic synergy when, for example, a decrease in tumor growth is achieved by administration of the combination which is greater than the value of the decrease in tumor growth of the best constituent when the constituent is administered alone.

Thus, in combination, the components of such combinations have an additive or superadditive effect on suppressing tumor growth, as compared to monotherapy with the anti-CD40 antibody or treatment with the additional therapeutic agent(s) in the absence of antibody therapy. By "additive" is meant a result that is greater in extent (e.g., in the degree of reduction of tumor mitotic index or of tumor growth or in the degree of tumor shrinkage or the frequency and/or duration of symptom-free or symptom-reduced periods) than the best separate result achieved by monotherapy with each individual component, while "superadditive" is used to indicate a result that exceeds in extent the sum of such separate results. In one embodiment, the additive effect is measured as slowing or stopping of tumor growth. The additive effect can also be measured as, e.g., reduction in size of a tumor, reduction of tumor mitotic index, reduction in number of metastatic lesions over time, increase in overall response rate, or increase in median or overall survival. The additive effect may be measured as increasing induction of CD95 expression when incubated with Ramos cells, increasing B cell proliferation when incubated with human B cells, and/or increasing increased induction of IL12p40 expression when incubated with dendritic cells.

One non-limiting example of a measure by which effectiveness of a therapeutic treatment can be quantified is by calculating the log10 cell kill, which is determined according to the following equation:$log 10 cell kill = T C \left(days\right) / 3.32 \times Td$ in which T C represents the delay in growth of the cells, which is the average time, in days, for the tumors of the treated group (T) and the tumors of the control group (C) to have reached a predetermined value (1 g, or 10 mL, for example), and Td represents the time, in days necessary for the volume of the tumor to double in the control animals. When applying this measure, a product is considered to be active if log10 cell kill is greater than or equal to 0.7 and a product is considered to be very active if log10 cell kill is greater than 2.8. Using this measure, a combination, used at its own maximum tolerated dose, in which each of the constituents is present at a dose generally less than or equal to its maximum tolerated dose, exhibits therapeutic synergy when the log10 cell kill is greater than the value of the log10 cell kill of the best constituent when it is administered alone. In an exemplary case, the log10 cell kill of the combination exceeds the value of the log10 cell kill of the best constituent of the combination by at least 0.1 log cell kill, at least 0.5 log cell kill, or at least 1.0 log cell kill.

### EXAMPLES

### Example 1

### Generation of CD40-Specific Human Monoclonal Antibodies

Human anti-CD40 monoclonal antibodies were generated by immunizing the H2L2 strain of Harbour^{®} transgenic mice with a soluble human CD40 antigen. Harbour^{®} transgenic mice have had the endogenous mouse heavy chain (HC) and kappa light chain (κ-chain) DNA sequences knocked out and have had sequences for the human variable (V) regions and rat constant (C) regions stably incorporated into the mouse genome.

Antigen and Immunization: The antigen was a soluble fusion protein comprising a CD40 extracellular domain fused with an antibody Fc domain (R&D Systems), or a recombinant human CD40-msG2a chimeric protein (made in-house). The antigen was mixed with Complete Freund's (Sigma) adjuvant for the first immunization. Thereafter, the antigen was mixed with Incomplete Freund's (Sigma). Additional mice were immunized with the soluble CD40 protein in MPL plus TDM adjuvant system (Sigma). 5-25 micrograms soluble recombinant CD40 antigen in PBS or 5 x 10⁶ NSO cells transfected for surface expression of human CD40 in PBS were mixed 1:1 with the adjuvant. Mice were injected with 200 microliters of the prepared antigen into the peritoneal cavity every 14 days. Animals that developed anti-CD40 titers were given an iv injection of 5-10 micrograms soluble recombinant CD40 antigen three to four days prior to fusion. Mouse spleens were harvested, and the isolated splenocytes used for hybridoma preparation.

Hybridoma Preparation: The P3x63Ag8.653 murine myeloma cell line (ATCC CRL 1580) was used for the fusions. RPMI 1640 (Invitrogen) containing 10% FBS was used to culture the myeloma cells. Additional media supplements were added to the Hybridoma growth media, which included: up to 10% Hybridoma Enhancing Supplement (Sigma), 10% FBS (Sigma), L-glutamine (Gibco) 0.1% gentamycin (Gibco), 2-mercaptoethanol (Gibco), with HAT (Sigma; 1.0 x 10⁴ M hypoxanthine, 4.0 x 10⁻⁷ M aminopterin, 1.6 x 10⁻⁵ M thymidine media.

Spleen cells were mixed with the P3x63Ag8.653myeloma cells in a 6:1 ratio and pelleted by centrifugation. Polyethylene glycol was added dropwise with careful mixing to facilitate fusion. Hybridomas were allowed to grow out for one to two weeks until visible colonies become established. Supernatant was harvested and used for initial screening for rat IgG via ELISA using a human soluble CD40 fusion protein and a rat Fc specific detection. IgG positive supernatants were then assayed for CD40 specificity via flow cytometry. The hybridomas were also screened for cross-reactivity with cynomolgus macaque CD40 and all were positive for binding.

Hybridoma cells were expanded and cell pellets were frozen for RNA isolation and sequencing. The V_{H} and V_{L} coding regions of human mAbs were identified using RNA from the corresponding hybridomas. RNA was reverse transcribed to cDNA, the V coding regions were amplified by PCR and the PCR product was sequenced, inserted into human IgG2 vector, transiently expressed and purified by protein A column chromatography which led to the isolation of a number of antibodies of particular interest, which were designated as 3C3, 3G5, 1B4, 3B6, 6H6, 6H6, 2E1.2, 1B5-NK (in the latter case following N75K modification on FR3 of the heavy chain), and 3B6-NS (following N63S modification of antibody 3B6 on FR3 of the light chain to remove an N-linked glycosylation site).
Tables 1, 2, and 3 summarize the germline information and amino acid sequences of the V_{H} and V_{L} regions of the human mAbs (in the case of the amino acid sequences, the Complementarity Determining Regions (CDRs) are underlined). The corresponding nucleic acid sequences are provided in the sequence table headed "Summary of Sequence Listing" at the end of these Examples.

**Table 1 - Germline Data**

| **mAb** | VH/VL | Germline | | |
|---|---|---|---|---|
| | | V | D | J |
| | | | | |
| **3G5** | H | IGHV3-33*01 F (VH3-33) | IGHD3-10*01 F (D3-10) | IGHJ4*02 F (JH4b) |
| | L | IGKV3-15*01 F (L2) | | IGKJ5*01 F (JK5) |
| **3C3** | H | IGHV3-33*01 F (VH3-33) | IGHD3-10*02 F (D4-b) | IGHJ4*02 F (JH4b) |
| | L | IGKV1-27*01 F (A20) | | IGKJ3*01 F (JK3) |
| **3B6** | H | IGHV3-23*01 F (VH3-23) | IGHD2-15*01 F (D2-15) | IGHJ6*02 F (JH6b) |
| | L | IGKV2-28*01 F (A19) | | IGKJ1*01 F (JK1) |
| **6H6** | H | IGHV3-33*01 F (VH3-33) | IGHD3-10*01 F (D3-10) | IGHJ4*02 F (JH4b) |
| | L | IGKV3-15*01 F (L2) | | IGKJ4*01 F (JK4) |
| **1B4** | H | IGHV3-23*01 F (VH3-23) | IGHD1-26*01 F (D2-15) | IGHJ6*02 F (JH6b) |
| | L | IGKV2-28*01 F (A19) | | IGKJ1*01 F (JK1) |
| **1B5-NK** | H | IGHV3-33*03 F (VH3-33) | IGHD6-19*01 F (D2-15) | IGHJ2*01 F (JH2) |
| | L | IGKV1-27*01 F (A20) | | IGKJ2*01 F (JK2) |
| **2 E1.2** | H | IGHV3-33*01 F (VH3-33) | IGHD3-10*01 F (D3-10) | IGHJ4*02 F (JH4B) |
| | L2 | IGKV3-15*01 F (L2) | | IGKJ4*01 F (JK4) |
| **3B6-NS** | H | IGHV3-23*01 F (VH3-23) | IGHD2-15*01 F (D2-15) | IGHJ6*02 F (JH6b) |
| | L2 | IGKV2-28*01 F (A19) | | IGKJ1*01 F (JK1) |

**Table 2 - CDR Sequences**

| **mAb** | VH/ VL | Kabat CDRs (Chothia) | | |
|---|---|---|---|---|
| | | CDR1 | CDR2 | CDR3 |
| | | | | |
| **3G5** | H | SNGIH (GFTFSSN) | VIWSDGSNKFYADSVK G (WSDGSN) | ASGSGSYYNFFDY (ASGSGSYYNFFDY) |
| | L | RASQSVRSNLA (RASQSVRSNLA) | GASTRAT (GASTRAT) | QQHNKWIT (QQHNKWIT) |
| **3C3** | H | RYGMY (GFIFSRY) | VIWYDGSYKYYADSVK G (WYDGSY) | ESPWYYFDY (ESPWYYFDY) |
| | L | RASQGISNYLA (RASQGISNYLA) | AASTLQS (AASTLQS) | QKYKSAPFT (QKYKSAPFT) |
| **3B6** | H | SYAMS (GFTFSSY) | GITGTGGSTYYADSVKG (TGTGGS) | RAGGSFYYYYGMDV (RAGGSFYYYYGMDV) |
| | L | RSSQSLLHSTGYNY LD (RSSQSLLHSTGYNY LD) | LGSNRAS (LGSNRAS) | MQALQTPWT (MQALQTPWT) |
| **6H6** | H | SYGMH (GFTLSSY) | VIWDDGSNKYYADSVK G (WDDGSN) | AGGSGRYYNYFDY (AGGSGRYYNYFDY) |
| | L | RASQSVRSNLA (RASQSVRSNLA) | GASTRAT (GASTRAT) | QQHNNWLT (QQHNNWLT) |
| **1B4** | H | SYAMT (GFTFSSY) | GITGSGANTFYTDSVKG (TGSGAN) | RNGGSYYYYYGMDV (RNGGSYYYYYGMDV) |
| | L | RSSQSLLHSSGYNYL D (RSSQSLLHSSGYNY LD) | LGSNRAS (LGSNRAS) | MQALQIPWT (MQALQIPWT) |
| **1B5-NK** | H | SFGMH (GFTFSSF) | LIWFDGSSKYYADSV KG (WFDGSS) | GFAAVAGWYFDF (GFAAVAGWYFDF) |
| | L | RASQGVRKYLA (RASQSVRSNLA) | AASTLQS (AASTLQS) | QKYFSAPYT (QKYFSAPYT) |
| **2E1. 2** | H | SYGMH (GFTFSSY) | VIWDDGSNKYYADSV KG (WDDGSN) | AGSSGRYYNYFDY (AGSSGRYYNYFDY) |
| | L | RASQSVRSNLA (RASQSVRSNLA) | GASTRAT (GASTRAT) | QQYNKWLI (QQYNKWLI) |
| **3B6-NS** | H | SYAMS (GFTFSSY) | GITGTGGSTYYADSVKG (TGTGGS) | RAGGSFYYYYGMDV (RAGGSFYYYYGMDV) |
| | L | RSSQSLLHSTGYNY LD (RSSQSLLHSTGYNY LD) | LGSNRAS (LGSNRAS) | MQALQTPWT (MQALQTPWT) |

**Table 3 - Full-Length Variable Region Sequences**

| **mAb** | VH/VL | Sequence |
|---|---|---|
| **3G5** | H | QVQLVESGGGVVQPGKSLRLSCAASGFTFSSNGIHWVRQAPGKGL EWVAVIWSDGSNKFYADSVKGRFTISRDNSKNTLYLQMNSLRAE DTAVYYCARASGSGSYYNFFDYWGQGTLVTVSS |
| | L | EIVMTQSPATLSVSPGERATLSCRASQSVRSNLAWYQQKPGQAPR LLIYGASTRATGIPARFSGSGSGTEFTLTINSLQSEDFAVYYCQQHN KWITFGQGTRLEIK |
| **3C3** | H | QVQLVESGGGVVQPGRSLRLSCAGSGFIFSRYGMYWVRQAPGKG LEWVAVIWYDGSYKYYADSVKGRFTISRDNSKNTLYLQMNSLRA EDTAVYYCARESPWYYFDYWGQGTLVTVSS |
| | L | DIQMTQSPSSLSASVGDRVTITCRASQGISNYLAWYQQKPGKVPK LLIYAASTLQSGVPSRFSGSGSGTDFTLTISSLQPEDVATYYCQKYK SAPFTFGPGTKVDIK |
| **3B6** | H | EVQLLESGGGLVQPGGSLRLSCAASGFTFSSYAMSWVRQAPGKGL EWVSGITGTGGSTYYADSVKGRFTISRDNSKNTLYVQMNSLRAE DTAVYYCAKRAGGSFYYYYGMDVWGQGTTVTVSS |
| | L | DIVMTQSPLSLPVTPGEPASISCRSSQSLLHSTGYNYLDWYLQKPG QSPQLLIYLGSNRASGVPDRFNGSGSGTDFTLKISRVEAEDFGVYY CMQALQTPWTFGHGTKVEIK |
| **6H6** | H | QVQLVESGGGVVQPGRSLRFSCAASGFTLSSYGMHWVRQAPGKG LEWVAVIWDDGSNKYYADSVKGRFTISRDNSKNTLYLQMNSLRA EDTAVYYCARAGGSGRYYNYFDYWGQGTLVTVSS |
| | L | EIVMTQSPATLSVSPGERATLSCRASQSVRSNLAWYQQKPGQAPR LLIYGASTRATGIPARFSGSGSGTDFTLTISSLQSEDFAVYYCQQHN NWLTFGGGTKVEIK |
| **1B4** | H | EVQLLESGGGLVQPGGSLRLSCAASGFTFSSYAMTWVRQVPGKGL EWVSGITGSGANTFYTDSVKGRFTISRDNSNNSLYLQMNSLRADD TAVYYCAKRNGGSYYYYYGMDVWGQGTTVTVSS |
| | L | DIVMTQSPLSLPVTPGEPASISCRSSQSLLHSSGYNYLDWYLQKPG QSPQLLIYLGSNRASGVPDRFSGSGSGTDFTLKISRVEAEDVGVYY CMQALQIPWTFGQGTKVEIK |
| **1B5-NK** | H | QVQLVESGGGVVQPGRSLRLSCAASGFTFSSFGMHWVRQAPGKG LEWVTLIWFDGSSKYYADSVKGRFTISRDNSKNTLYLQMNSLRA EDTAVYYCVRGFAAVAGWYFDFWGRGTLVTVSS |
| | L | DIQMTQSPSSLSASVGDRVTITCRASQGVRKYLAWYQQKPGKVPK LLIYAASTLQSGVPSRFSGSGSGTDFTLTISSLQPEDVATYYCQKYF SAPYTFGQGTKLEIK |
| **2E1. 2** | H | QVQLVESGGGVVQPGRSLRLSCAASGFTFSSYGMHWVRQAPGKG LEWVAVIWDDGSNKYYADSVKGRFTISRDNSKNTLYLQMNSLRA EDTAVYYCARAGSSGRYYNYFDYWGQGTLVTVSS |
| | L | EIVMTQSPATLSVSPGERATLSCRASQSVRSNLAWYQQKPGQAPR LLIYGASTRATGIPDRFSGSGSGTEFTLTISSLQSEDFAVYHCQQYN KWLIFGGGTKVEIK |
| **3B6-NS** | H | EVQLLESGGGLVQPGGSLRLSCAASGFTFSSYAMSWVRQAPGKGL EWVSGITGTGGSTYYADSVKGRFTISRDNSKNTLYVQMNSLRAE |
| | | DTAVYYCAKRAGGSFYYYYGMDVWGQGTTVTVSS |
| | L | DIVMTQSPLSLPVTPGEPASISCRSSQSLLHSTGYNYLDWYLQKPG QSPQLLIYLGSNRASGVPDRFSGSGSGTDFTLKISRVEAEDFGVYY CMQALQTPWTFGHGTKVEIK |

The full amino acid sequences of the heavy and light chains of the antibody 3C3 was as follows:
**Light chain sequence (with leader sequence removed)** 
DIQMTQSPSSLSASVGDRVTITCRASQGISNYLAWYQQKPGKVPKLLIYAASTLQSGVPSRFS
GSGSGTDFTLTISSLQPEDVATYYCQKYKSAPFTFGPGTKVDIKRTVAAPSVFIFPPSDEQ
LKSGTASVVCLLNNFYPREAKVQWKVDNALQSGNSQESVTEQDSKDSTYSLSST
LTLSKADYEKHKVYACEVTHQGLSSPVTKSFNRGEC
**Heavy chain sequence (with leader sequence removed)**

QVQLVESGGGVVQPGRSLRLSCAGSGFIFSRYGMYWVRQAPGKGLEWVAVIWYDGSYKYY
ADSVKGRFTISRDNSKNTLYLQMNSLRAEDTAVYYCARESPWYYFDYWGQGTLVTVSSAST
KGPSVFPLAPCSRSTSESTAALGCLVKDYFPEPVTVSWNSGALTSGVHTFPAVLQ
SSGLYSLSSVVTVPSSNFGTQTYTCNVDHKPSNTKVDKTVERKCCVECPPCPAPP
VAGPSVFLFPPKPKDTLMISRTPEVTCVVVDVSHEDPEVQFNWYVDGVEVHNAK
TKPREEQFNSTFRVVSVLTVVHQDWLNGKEYKCKVSNKGLPAPIEKTISKTKGQ
PREPQVYTLPPSREEMTKNQVSLTCLVKGFYPSDIAVEWESNGQPENNYKTTPP
MLDSDGSFFLYSKLTVDKSRWQQGNVFSCSVMHEALHNHYTQKSLSLSPG

In each case the variable sequence is shown in italics and the constant domain is shown in bold. The constant domain sequence is an IgG2 sequence from which the C-terminal lysines have been removed.

The same constant domain sequence was used for the other antibodies with their respective variable sequences as listed above.

### Example 2

### Determination of Affinity and Rate Constants of Human mAbs by Bio-Layer Interferometry (BLI)

Binding affinity and binding kinetics of various human anti-CD40 antibodies were examined by bio-layer interferometry (BLI) using an Octet^{™} QK^{e} instrument (Pall ForteBio, Menlo Park, CA) according to the manufacturer's guidelines.

Purified antibodies from Example 1 were captured on Anti-Human Fc Capture (AHC) biosensors (Fortebio Product No. 18-5060). Each antibody was prepared in dilution buffer (10mMPO4+150mM NaCl+1mg/mL BSA+ 0.5%Tween 20, pH 7.2) to 0.5µg/mL and loaded on freshly hydrated AHC biosensors for 35-50sec at 25°C and 1000rpm plate shake speed to achieve a target response of 0.2nm. Low levels of ligand were captured to limit any effects of mass transport of analyte on kinetic parameters. For one assay, eight biosensors were loaded with the same antibody.

Binding was determined by exposing six of the antibody loaded biosensors to analyte: soluble human CD40-MsIgG2a (Celldex, 60kD by SDS-PAGE). Affinity measurements were determined using 2-fold serial dilutions of analyte ranging from 3.13 to 0.098nM in dilution buffer at 25°C and 1000rpm plate shake speed. Association of the antibody loaded biosensors in analyte wells was carried out for 1200 seconds, the biosensors were then moved to dilution buffer wells for 2.5hrs (9000sec) for dissociation measurements.

Corresponding controls were conducted in each case by keeping the two remaining biosensors with captured antibody in dilution buffer wells for association and dissociation steps. The data for the control biosensors was used to subtract background and account for biosensor drift and antibody dissociation from the biosensors.

Fortebio's Data Analysis Software version 8.2.0.7 (Pall ForteBio, Menlo Park, CA) was used in each case to derive kinetic parameters from the concentration series of analyte in dilution buffer binding to captured antibody. The association and dissociation curves were fitted to a 1:1 binding model using the data analysis software according to the manufacturer's guidelines.

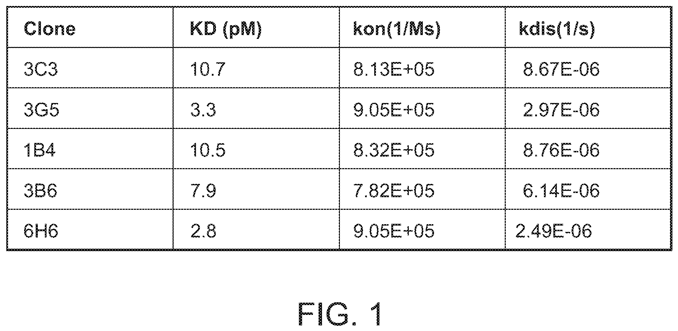

The affinity and kinetic parameters (with background subtracted) as determined are shown in Figure 1, where kon = rate constant of association, kdis = rate constant of dissociation, and K_{D} = dissociation equilibrium binding constant, determined by the ratio kdis/kon.

### Example 3

### Assays to Determine Human mAb Binding Characteristics to CD40

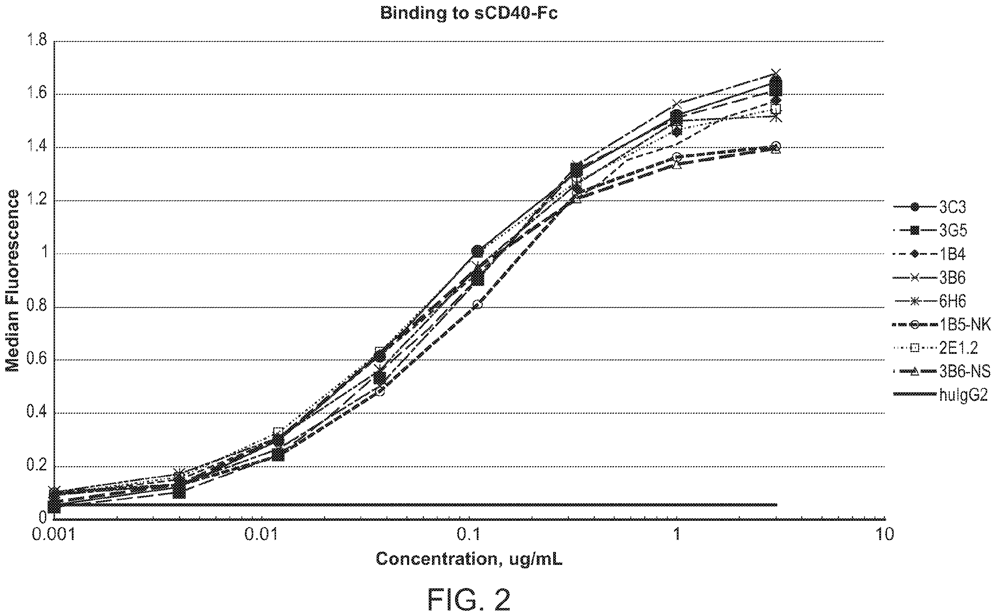

Microtiter plates were coated with recombinant human CD40-Fc in PBS, and then blocked with 5% bovine serum albumin in PBS. Protein A purified human mAbs from Example 1 and an isotype control were added at various concentrations and incubated at 37°C. The plates were washed with PBS/Tween and then incubated with a goat-anti-human IgG F(ab')2-specific polyclonal reagent conjugated to horseradish peroxidase at 37°C. After washing, the plates were developed with HRP substrate, and analyzed at OD 450-650 using a microtiter plate reader. Representatives binding curves are shown in Figure 2.

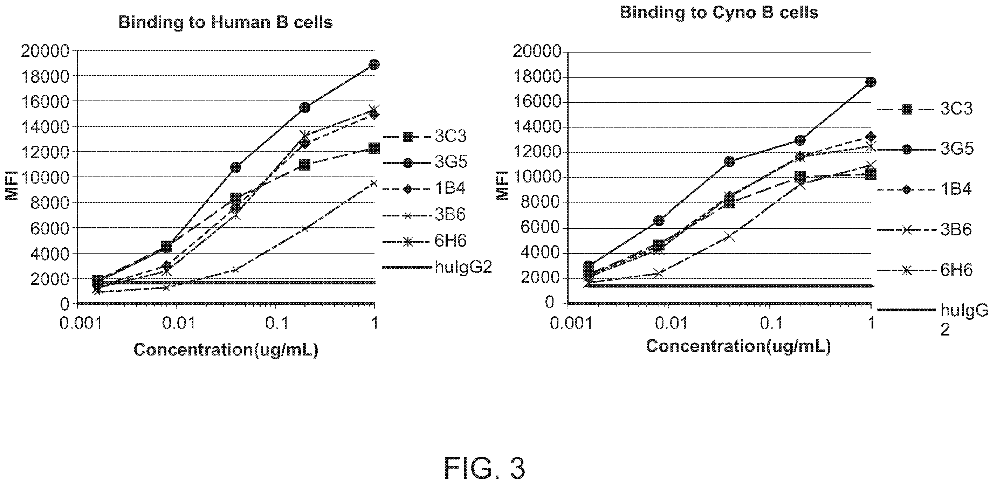

To establish that cynomolgus macques are a relevant model for testing anti-CD40 mAbs, purified macaque PBMC's or human PBMC's were incubated with varying concentrations of anti-human CD40 mAb for 20 minutes at room temperature on a plate shaker. The cells were then washed twice with PBS containing 0.1% BSA and 0.05% NaN₃ (PBA). A goat anti-human IgG Fc-PE antibody was added for 20 minutes at room temperature on a plate shaker. B cells were identified by subsequent staining with an allophycocyanin (APC) conjugated CD20 antibody. Cells were analyzed by flow cytometry and binding curves are shown in Figure 3, which indicate similar binding to CD40 from macaque and human.

### Example 4

### Blocking of sCD40L Binding by ELISA

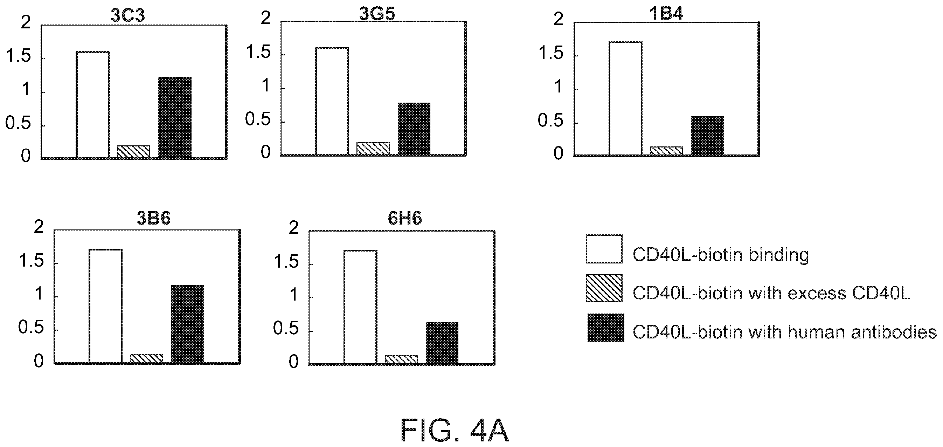

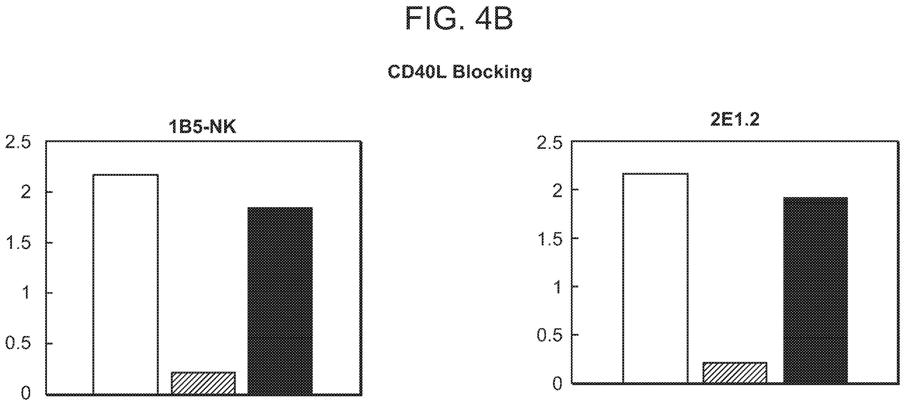

The effect of the human mAbs from Example 1 on the binding of soluble CD40 Ligand (sCD40L) to CD40 protein was measured by ELISA. A microtiter plate was coated with 2 µg/ml soluble recombinant human CD40/Fc chimera from R&D Systems, then blocked with 5% PBA. The anti-CD40 antibodies ( [final] = 100µg/mL) were added to the plate, followed by soluble human recombinant CD40L-biotin from Immunex ([final] = 0.5µg/mL). CD40-captured rCD40L was detected with streptavidin-HRP and substrate Super Blue TMB. The results are shown in Figures 4A and B with controls as indicated.

### Example 5

### Binding to CD40 cells

The ability of anti-CD40 human mAbs to bind to CD40 on cells expressing human CD40 on their surface was investigated by flow cytometry as follows:
Antibodies from Example 1 were tested for binding to human cell lines expressing human CD40 on their surface. Protein A purified human mAbs 3C3, 3G5, 1B4, 3B6, and 6H6 were incubated with, Raji and Ramos cells expressing human CD40 at room temperature on a plate shaker. After 20 minutes, the cells were washed with PBS containing 0.1% BSA and 0.05% NaN₃ (PBA) and the bound antibodies were detected by incubating the cells with a PE labeled goat anti-human IgG Fc-specific probe. The excess probe was washed from the cells with PBA and the cell associated fluorescence was determined by analysis using a FACSCanto II^{™} instrument (BD Biosciences, NJ, USA) according to the manufacturer's directions.

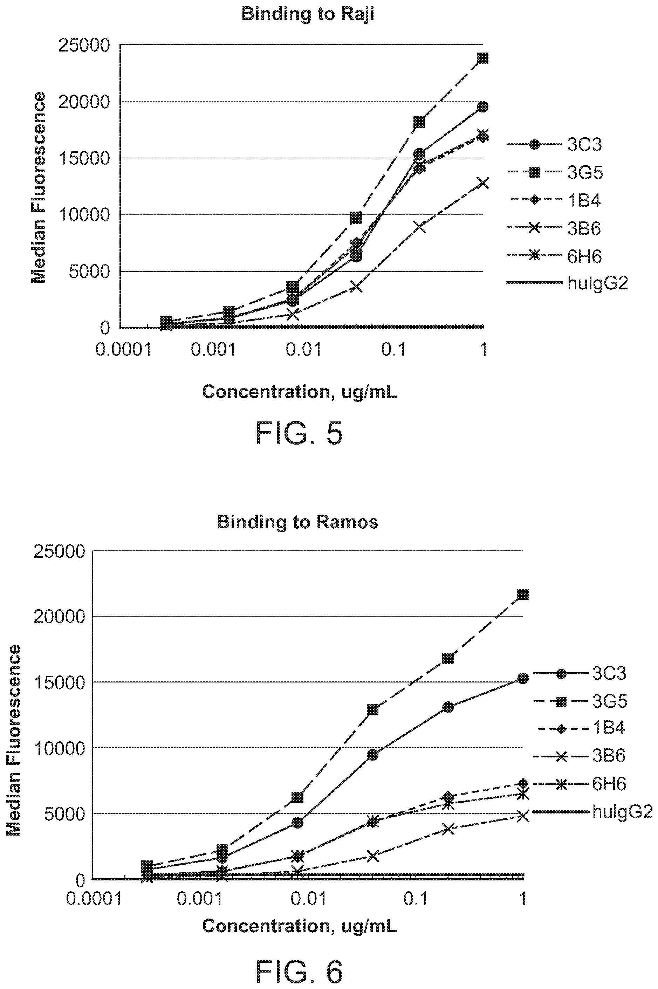

As shown in Figures 5 (binding to Raji cells) and Figure 6 (binding to Ramos cells), the human mAbs demonstrated high level binding to cells expressing human CD40 as a function of antibody concentration.

### Example 6

### CD95 Induction on Ramos Cells

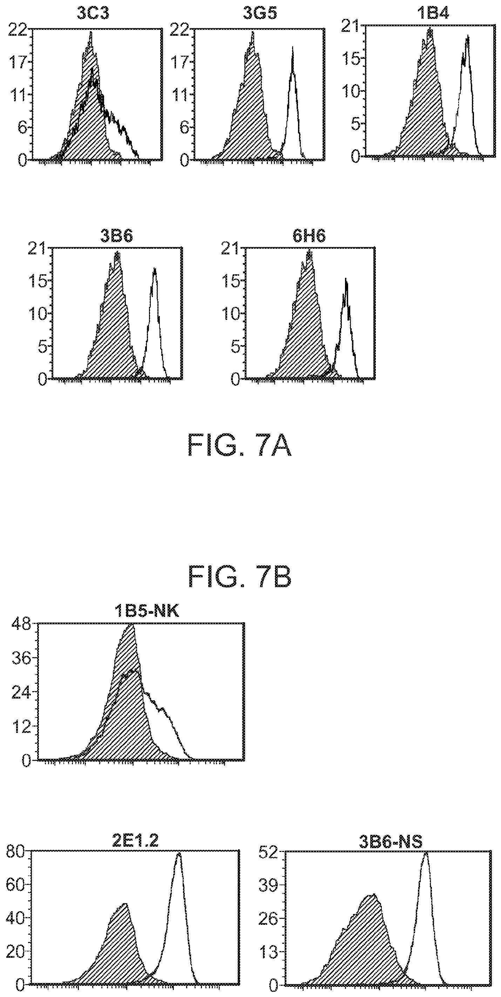

Ramos cells were incubated overnight at 37°C, 6%CO₂ with 2ug/mL of the human anti-CD40 mAbs from Example 1. Then next day, they were washed once with PBA and stained with PE-conjugated anti-CD95 antibody (Becton Dickinson) for 20 minutes at room temperature, with shaking. The excess labeled antibody was washed off and the samples read on a FACSCanto II^{™} instrument (BD Biosciences, NJ, USA). As shown in Figures 7A and B ((in which the shaded plots represent untreated/control cells and the black lines represent cells treated with the antibodies as indicated), the 3C3 and the 1B5-NK antibodies show increases in CD95 and the other antibodies 3G5, 1B4, 3B6, 6H6, 2E1.2, and 3B6-NS were able to induce a strong increase in expression of surface expressed CD95.

### Example 7

### Dendritic Cell activation

Dendritic cells were derived from human monocytes as follows:
PMBC's were added to a T175cm² flasks and monocytes allowed to adhere for ~2 hours at 37°C, 6%CO₂. The cells were removed and the monocytes cultured for 7 days in RPMI containing 10% FBS, 10ng/mL IL-4 (R&D Systems) and 100ng/mL GM-CSF (R&D Systems). The cells were harvested and confirmed to be dendritic cells by expression of CD11c (not shown).

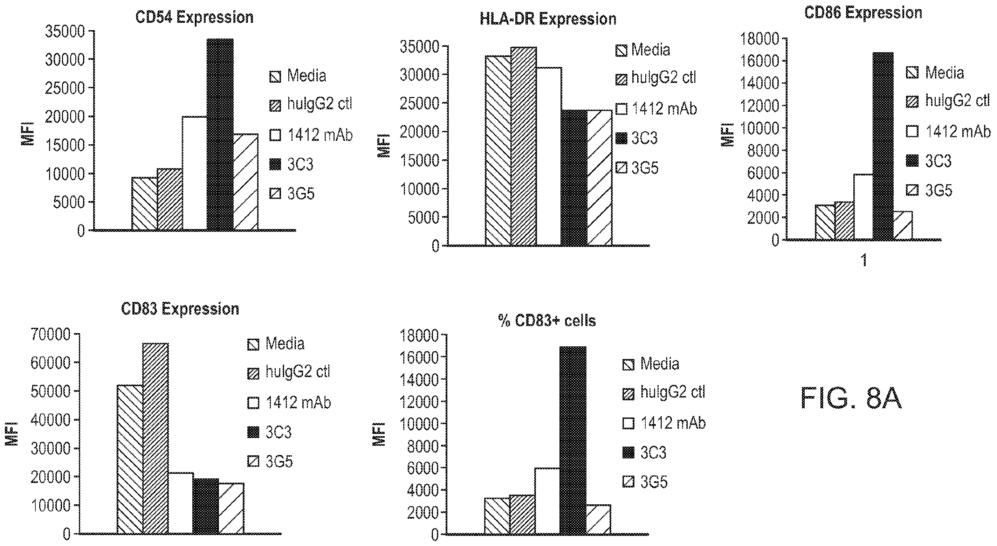

The cells were then incubated in the presence of 10ug/mL 3C3 and 3G5 human anti-CD40 antibodies from Example 1and appropriate controls at 37°C, 6%CO₂. After 72 hours, the cells were harvested and the supernatant was collected and stored for cytokine analysis. The cells were stained with the following labeled antibodies for 20 minutes at room temperature, shaking: HLA-DR V450, CD54 PE, CD86 APC, and CD83 BV510 (all from BD). Cells were then washed twice and analyzed on a FACSCanto II^{™} instrument (BD Biosciences, NJ, USA). Figure 8A shows the level of expression for each of these markers when incubated with the indicated antibody or control.

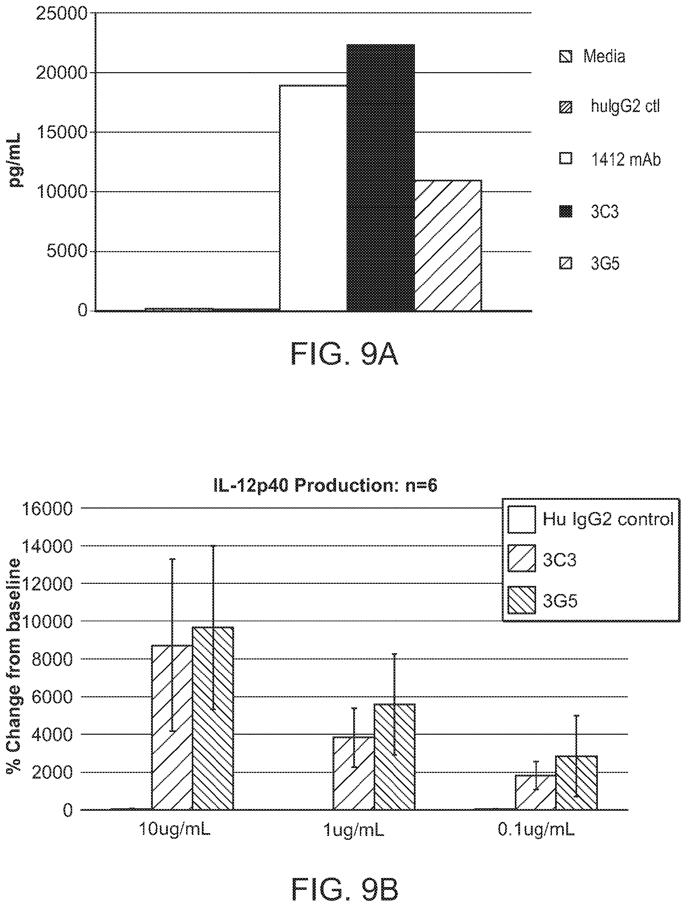

Induction of IL-12p40 was evaluated in the supernatents from these 72 hour cultures by ELISA (R&D Systems). Figure 9A shows the increase in IL-12p40 production with the 3C3 and 3G5 anti-CD40 antibodies relative to controls as indicated.

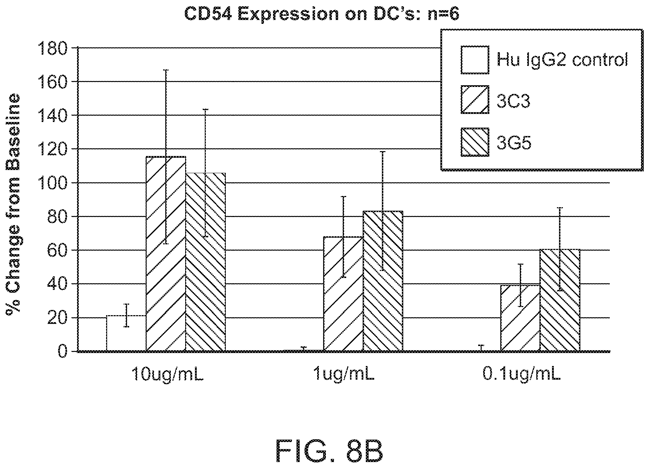

In a further experiment cells were incubated in the presence of 10, 1 and 0.1 ug/mL 3C3 and 3G5 human anti-CD40 antibodies from Example 1 and appropriate controls at 37°C, 6%CO₂. After 48 hours, the cells were harvested and the supernatant was collected and stored for cytokine analysis. The cells were stained with CD54 labeled antibody (BD) for 20 minutes at room temperature, shaking. Cells were then washed twice and analyzed on a FACSCanto II^{™} instrument (BD Biosciences, NJ, USA). Figure 8B shows the level of expression for CD54 when incubated with the indicated antibody or control.

Induction of IL-12p40 was evaluated in the supernatents from these 48 hour cultures by ELISA (R&D Systems). Figure 9B shows the increase in IL-12p40 production with the 3C3 and 3G5 anti-CD40 antibodies relative to controls as indicated.

### Example 8

### B cell Activation

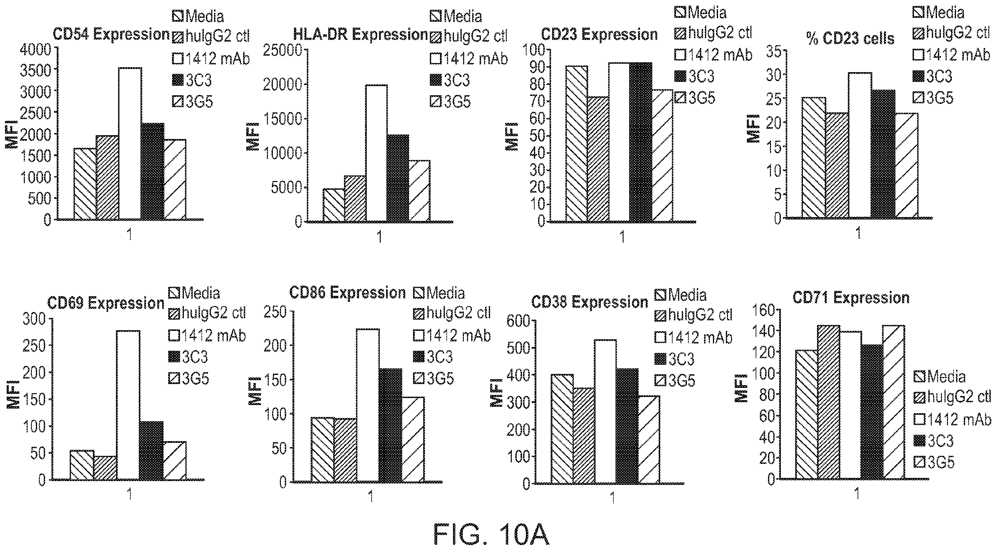

Whole blood was incubated with 10ug/mL of 3C3 and 3G5 anti-CD40 antibodies from Example 1 overnight at 37°C, 6% CO2. The next day, the following labeled antibodies were used to stain B cells and activation markers: CD54 PE, HLA-DR V450, CD23 PerCP-Cy5.5, CD69 APC, CD86 APC, CD38 PerCP-Cy5.5 and CD71 PE. The cells were stained for 20 minutes at room temperature, shaking, then washed twice and read on a FACSCanto II^{™} instrument (BD Biosciences, NJ, USA). Figure 10A shows the change in level of expression on each of these markers relative to controls as indicated.

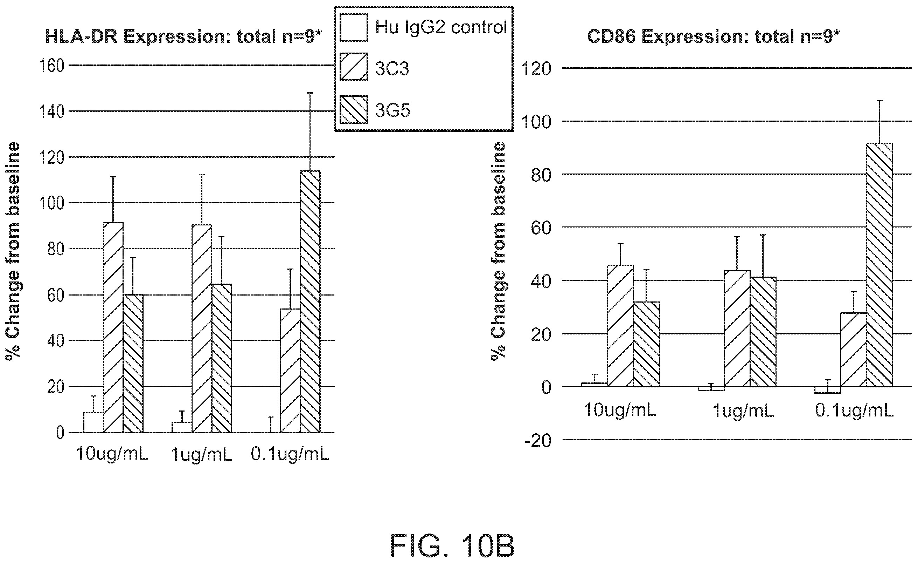

In a further experiment whole blood was incubated with 10, 1, and 0.1ug/mL of 3C3 and 3G5 anti-CD40 antibodies from Example 1 overnight at 37°C, 6% CO2. The next day, the following labeled antibodies were used to stain B cells and activation markers: CD19 V500, HLA-DR V450, CD86 APC (all from BD). The cells were stained for 20 minutes at room temperature, shaking, then washed twice and read on a FACSCanto II^{™} instrument (BD Biosciences, NJ, USA). Figure 10B shows the change in level of expression on each of these markers relative to controls as indicated.

### Example 9

### NFκB Activation

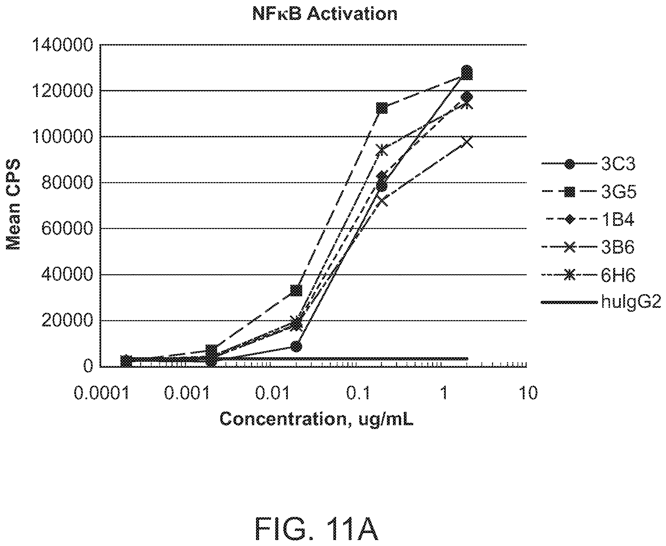

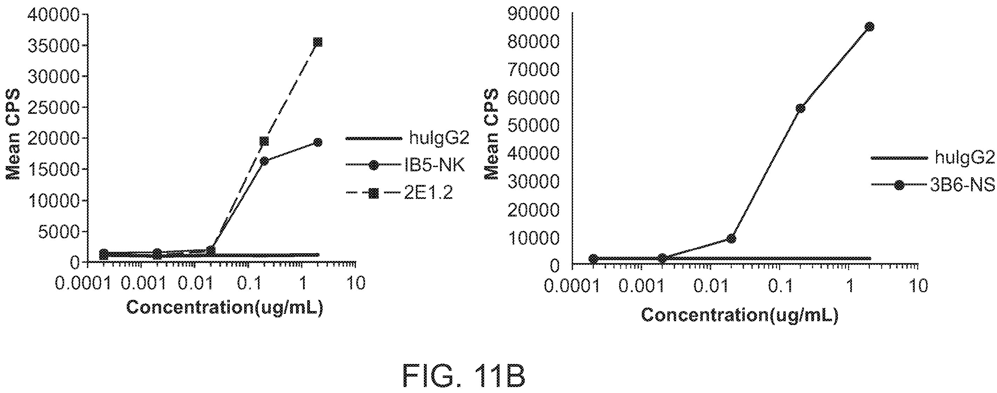

A luciferase reporter cell line expressing CD40 was incubated for 6 hours at 37°C, 6% CO₂ with various concentrations of the human anti-CD40 antibodies from Example 1. Luciferase expression was detected with the Luciferase Assay System by Promega according to the manufacturer's guidelines. Figures 11A and 11B show the high level of NFkB activation induced by 3C3, 3G5, 1B4, 3B6, 6H6, 2E1.2, 1B5-NK, and 3B6-NS antibodies as a function of antibody concentration.

### Example 10

### Tumor Killing in Raji Xenograft SCID Mouse Model

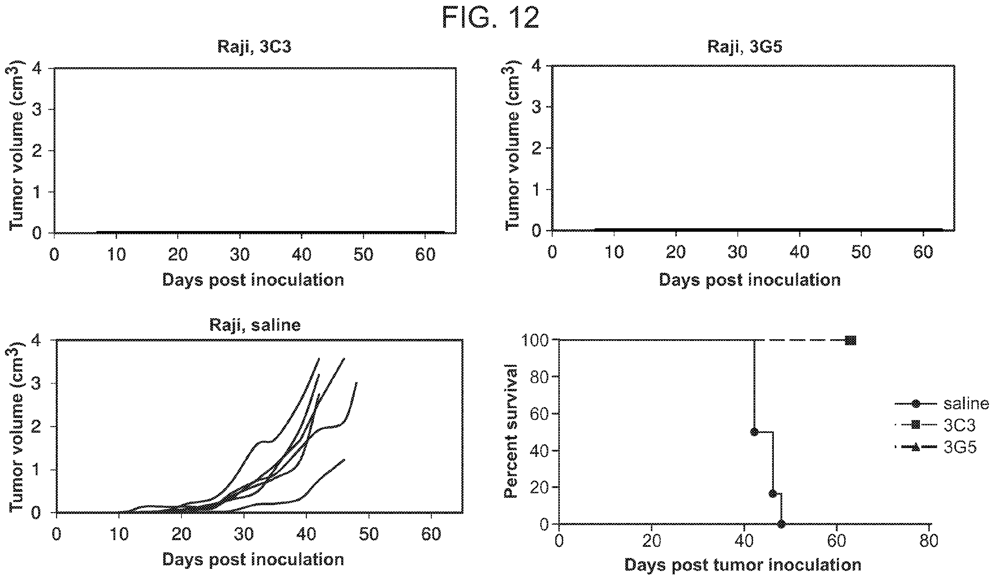

CB.17 SCID mice (purchased from Taconic Biosciences, Inc.) were maintained in a pathogen-free mouse facility. Lymphoma Raji cells (1 x 10⁶) were subcutaneously injected into SCID mice, 5 mice per group. On day 1, 5 and 11, these mice were treated with CD40 human mAbs clone 3C3 and 3G5 via intraperitoneal administration, 0.3 mg per dose. Tumor growth was measured with calipers 2 times a week. Results of tumor growth and survival analysis are shown in Figure 12, from which it can be seen that, in the tumor challenged mice, treatment with the anti-CD40 antibodies inhibited the growth of tumors and significantly prolonged survival relative to saline treated controls.

### Example 11

### Tumor Killing in Ramos Xenograft SCID Mouse Model

CB.17 SCID mice (purchased from Taconic Biosciences, Inc.) were maintained in a pathogen-free mouse facility. Human lymphoma Ramos cells (1 x 10⁶) were subcutaneously injected into SCID mice on day 0, 5 mice per group. On day 1, 5 and 11, these mice were treated with anti-CD40 human mAb 3C3 or 3G5 via intraperitoneal administration, 0.3 mg per dose. Tumor growth was measured with calipers 2 times a week.

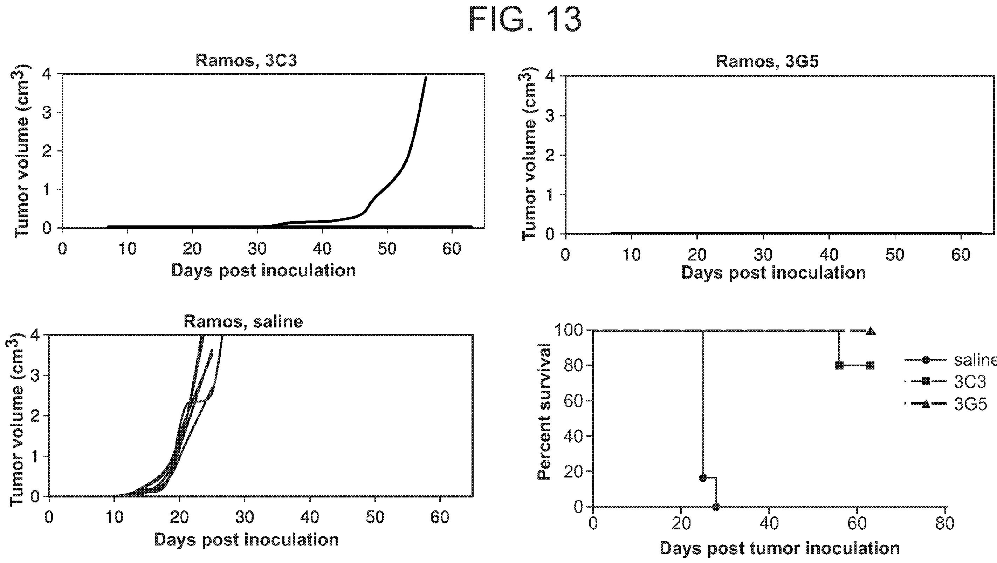

The results, shown in Figure 13, indicate that the anti-CD40 mAbs significantly inhibited the growth in tumor volume compared to saline treated controls, resulting in the survival of 100 % (3G5) or 80% (3C3) of the tumor challenged mice.

### Example 12

### T-cell proliferation

Human Peripheral Blood Mononuclear Cells (PBMCs) isolated from buffy coat prepparations were labeled with 0.5uM carboxyfluorescein succinimidyl ester (CFSE) at room temperature while rotating for 5 minutes. The CFSE labeled PBMCs (1.5 x 10⁶) were dispensed into wells dry coated with anti-CD3 antibody (OKT3) at 0.2ug/mL.

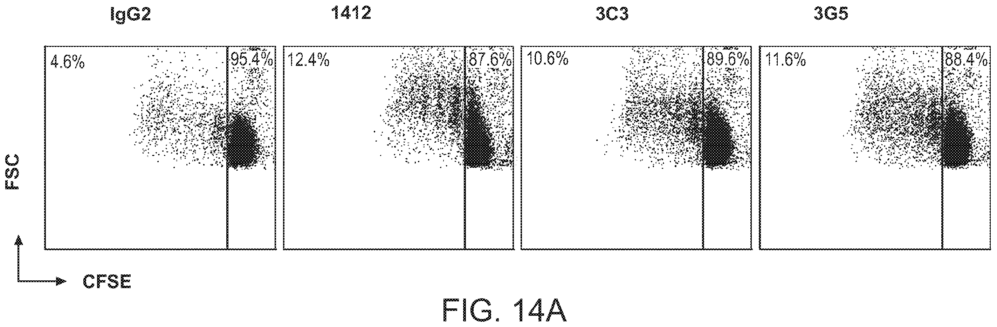

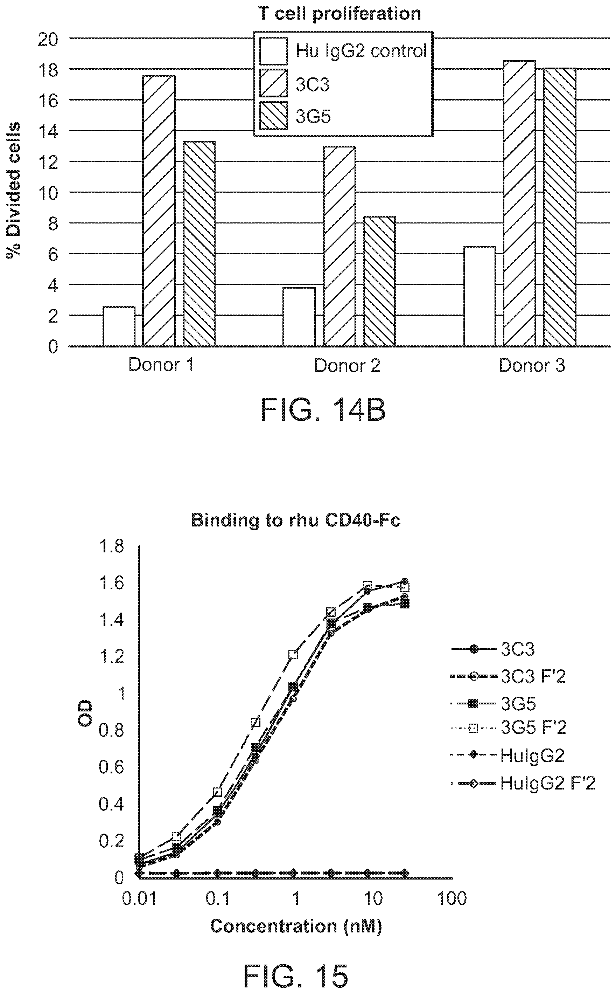

The CD40 antibodies (3G5, 3C3, 1412) or the isotype control (IgG2) were dispensed into the wells in soluble form at a final concentration of 10ug/mL. The plates were incubated at 37°C (5% CO₂) On day 6, the cells were harvested and stained with either anti-CD3- APC or the isotype control and analyzed by flow cytometry. Representative plots are shown in Figure 14A from which it can be seen that the antibodies significantly enhanced T-cell proliferation as evidenced by the reduced intensities of CFSE staining in the CD3+ gate. Results from a repeat experiment are shown in Figure 14B which shows the increase in dividing cells with the anti-CD40 antibodies relative to the isotype control.

### Example 13

### Binding to CD40 independent of Fc receptor interaction

Microtiter plates were coated with recombinant human CD40-Fc in PBS, and then blocked with 5% bovine serum albumin in PBS. Protein A purified human mAbs (whole IgG and F(ab')2 fragments as indicated) were added at various concentrations and incubated at 37°C. The plates were washed with PBS/Tween and then incubated with a goat-anti-human IgG F(ab')2-specific polyclonal reagent conjugated to horseradish peroxidase at 37°C. After washing, the plates were developed with HRP substrate, and analyzed at OD 450-650 using a microtiter plate reader. Results are shown in Figure 15. The IgG2 and F(ab)'2 versions of each antibody show a similar concentration dependence for binding to CD40-Fc.

### Example 14

### CD40 activation independent of Fc receptor interaction

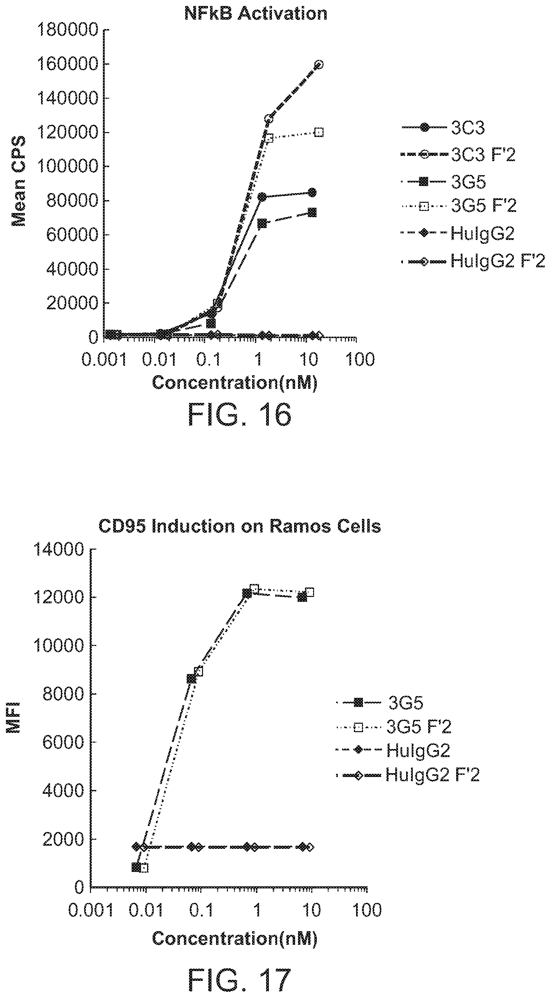

The luciferase reporter cell line expressing CD40 from Example 9 above was incubated for 6 hours at 37°C, 6% CO₂ with various concentrations of the human anti-CD40 antibodies (both whole IgG and F(ab')2 fragments as indicated). Luciferase expression was detected with the Promega Luciferase Assay System according to the manufacturer's guidelines. Results are shown in Figure 16. These show that binding to the Fc receptor is not required for CD40 mediated activation of the reporter cell line by 3C3 and 3G5 because intact antibodies with Fc domains and their corresponding F(ab)'2 versions lacking Fc domains are both able to activate NFkB in the reporter cell line.

### Example 15

### CD95 induction independent of Fc receptor interaction

Ramos cells were incubated overnight at 37°C, 6%CO₂ with various concentrations of the human anti-CD40 mAb's, (both whole IgG and F(ab')2 fragments as indicated). The next day they were washed once with PBA and stained with PE-conjugated anti-CD95 antibody (Becton Dickinson) for 20 minutes at room temperature with shaking. The excess labeled antibody was washed off and the samples read a FACSCanto II^{™} instrument (BD Biosciences, NJ, USA). Results are shown in Figure 17. These data indicate that Fc receptor interactions are not required by 3G5 to induce the expression of CD95 on the CD40+ human lymphoblastoid line Ramos.

### Example 16

### Synergy with sCD40L

Ramos cells were incubated overnight with the antibody 3C3 plus or minus 0.1 mg/ml soluble CD40 Ligand. The cells were then stained with anti-CD95-PE antibody and analyzed by flow cytometry. Results are shown in Figure 19 and indicate that the anti-CD40 antibody 3C3 acted synergistically with sCD40L. Accordingly, antibody 3C3 (and anti-CD40 antibodies which bind to the same epiutope as 3C3) exhibit synergistic agnostic effects with soluble CD40 ligand (sCD40L) and, therefore, have the ability to synergize with other therapeutic agents, including those which bind to the ligand binding site of human CD40. Representative synergistic effects include, for example, upregulation of immune function (e.g. T cell mediated immune responses as in vaccine therapies, NK activation in cancer therapies), inhibition of cell growth (e.g., in cancer therapy), and/or enhanced processing and presentation of an antigen by APCs (e.g., in vaccine therapy).

### Example 17

### Epitope mapping of anti-CD40 human antibodies 3C3 and 3G5 and sCD40

### i) Generation of truncated and mutated fragments of soluble CD40 (sCD40).

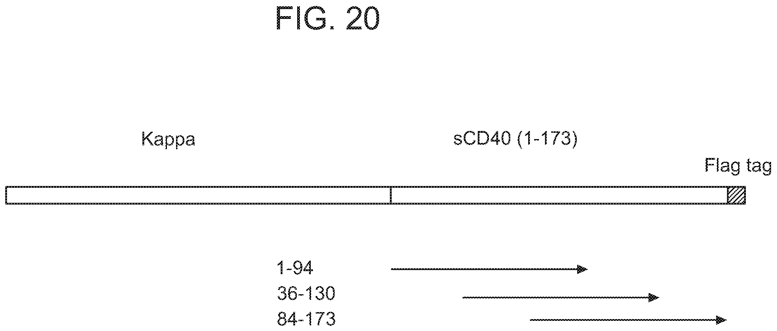

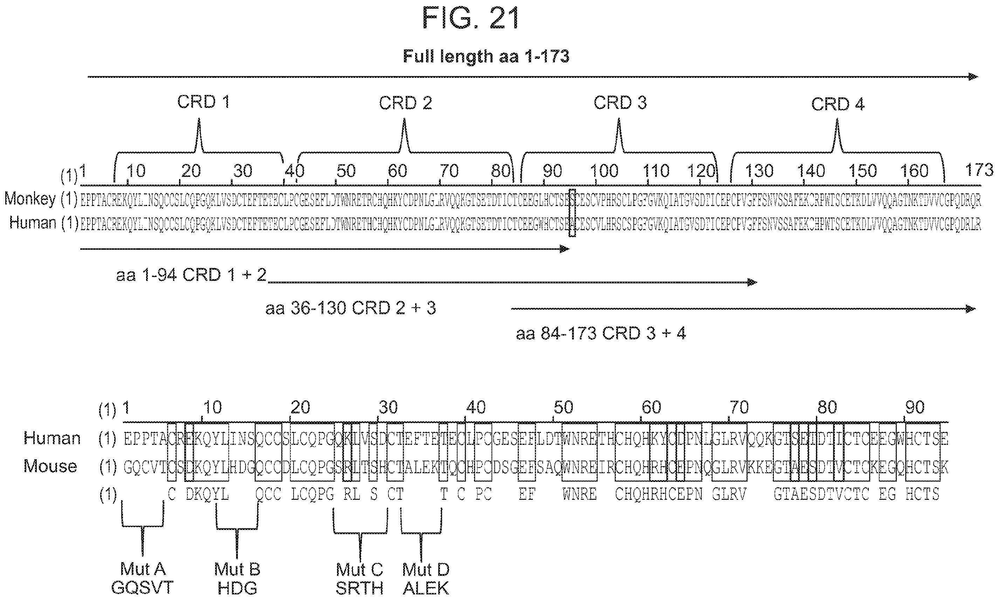

Soluble CD40 (sCD40) cDNA encoding the full length extra cellular domain (ECD) spanning amino acid residues 1-173 (SEQ ID NO: 133), as well as three smaller fragments coding amino acids 1-94, 36-130 and 84-173, were synthesized by GenScript and inserted in-frame into a mammalian expression vector with an N-terminal human kappa light chain and a C-terminal Flag tag. The resulting kappa-sCD40-Flag fusion proteins were expressed by transient transfection into ExpiCHO-S cells (SAFC). Since the CD40 antibody 3C3 recognizes human and monkey but not mouse CD40, a series of mutated sCD40aa 1-94 cDNA were designed based on the differences between the human and mouse sequences, as shown in the alignments in Figures 20 and 21. The mutants were synthesized and cloned by GenScript. All these truncated or mutated fragments were cloned into the same vector and expressed by the same cell line as aforementioned.

### ii) Determining binding by ELISA

The binding of 3C3 to the series of sCD40 fragments was tested by ELISA. 1 µg/ml of purified kappa-sCD40-Flag fusion proteins or CHO cell supernatants containing the sCD40 fusion proteins were captured to microtiter plates that were pre-coated with 5 ug/ml mouse anti-Flag antibody (Sigma) in PBS and blocked with 5% bovine serum albumin in PBS. Following incubation with the CD40 antibody, the microplates were washed with PBS/Tween, and incubated with a goat anti-human IgG Fc polyclonal reagent conjugated to horseradish peroxidase. After washing, the plates were developed with HRP substrate, and analyzed at OD 450-650 using a microtiter plate reader. An ELISA with a goat anti-human IgG Fab2-HRP to measure kappa chain binding was carried out in parallel to validate the sCD40 fusion protein expression from different transfections.

ELISA analysis with ~ 1ug/ml full length sCD40 and the 3 truncated fragments determined that sCD40 N-terminal residues 1-94 are essential and sufficient for the binding of 3C3, since the fragment encoding amino acid residues 1-94 bound to 3C3 as well as the entire ECD, but the fragments encoding amino acid residues 36-130 or 84-173 of this sequence did not bind at all (see Table 4).

**Table 4**

| | **Average OD** | |
|---|---|---|
| **Fragment amino acid residues** | **3C3** | **αFab2HRP** |
| 1-173 | 1.264 | 1.264 |
| 1-94 | 1.803 | 1.720 |
| 36-130 | 0.024 | 1.695 |
| 84-173 | 0.024 | 1.669 |

| **Mutant fragment of amino acids 1-94** | | |
|---|---|---|
| A (1-5) | 0.189 | 1.718 |
| B (13-15) | 2.032 | 1.730 |
| C (25, 26, 28, 30) | 1.487 | 1.685 |
| D (33-36) | 0.092 | 1.631 |

Based on these results, the critical recognition sites for 3C3 are within amino acids 1-35.

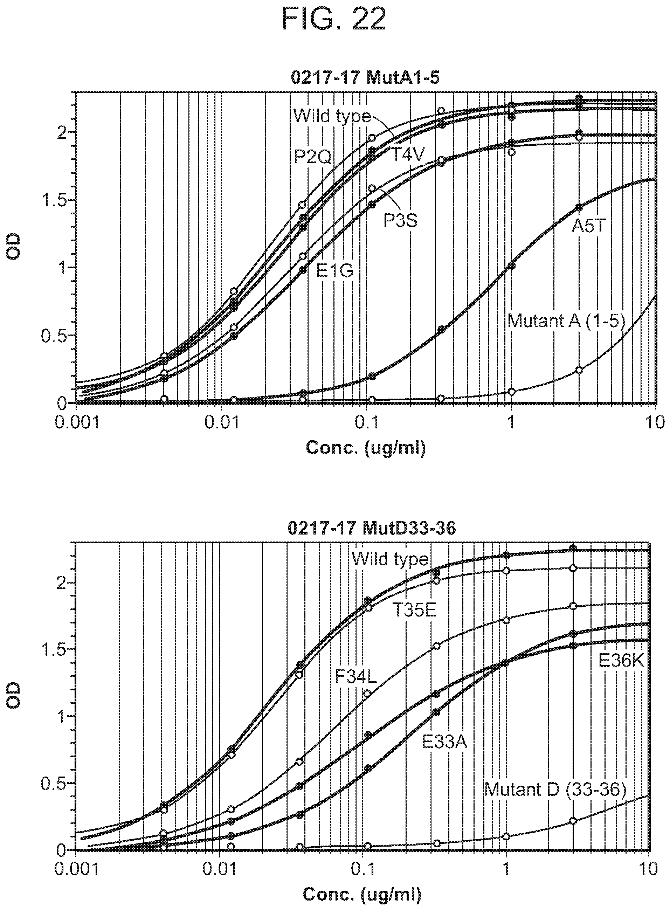

To further identify critical regions and amino acid residues for the conformational organization of the binding site of 3C3, ~ 2ug/ml 13 mutated sCD40 (amino acid residues 1-94) fragments (4 regional multiple mutations and 9 single mutations) were tested by ELISA (see Tables 5 and 6 showing results from separate experiments, and Figure 22).

**Table 5**

| | **Average OD** | |
|---|---|---|
| **Fragment amino acid residues** | **3C3** | **αFab2HRP** |
| 1-94 | 2.157 | 1.473 |

| **Mutant fragment of amino acids 1-94** | | |
|---|---|---|
| A (1-5) | 0.167 | 1.489 |
| D (33-36) | 0.124 | 1.429 |

| **Point Mutation** | | |
|---|---|---|
| E1G | 1.965 | 1.487 |
| P2Q | 2.077 | 1.490 |
| P3S | 2.011 | 1.489 |
| T4V | 2.152 | 1.519 |
| A5T | 1.126 | 1.517 |
| E33A | 1.620 | 1.521 |
| F34L | 1.883 | 1.500 |
| T35E | 2.072 | 1.487 |
| E36K | 1.369 | 1.433 |
| PBA | 0.031 | 0.011 |

**Table 6**

| | | **OD** | | |
|---|---|---|---|---|
| **Fragment amino acid residues** | | **3C3** | **3G5** | **αFab2HRP** |
| Full length 1-173 | | 2.364 | 2.214 | 1.525 |
| 1-94 | | 2.151 | 2.170 | 1.755 |
| 36-130 | | **0.029** | **0.048** | 1.716 |
| 84-173 | | **0.024** | **0.038** | 11. 599 |

| **Mutant fragment of amino acids 1-94** | | | | |
|---|---|---|---|---|
| A (1-5) | | **0.250** | 2.139 | 1.699 |
| B (13-15) | | 2.375 | 1.876 | 1.710 |
| C (25, 26, 28, 30) | | 2.016 | 2.161 | 1.604 |
| D (33-36) | | **0.233** | **0.042** | 1.548 |

| **Point Mutation** | | | | |
|---|---|---|---|---|
| E1G | | 2.011 | 2.083 | 1.720 |
| P2Q | | 2.197 | 2.158 | 1.754 |
| P3S | | 2.012 | 2.188 | 1.712 |
| T4V | | 2.213 | 2.210 | 1.664 |
| A5T | | 1.511 | 2.201 | 1.698 |
| E33A | | 1.695 | **0.074** | 1.709 |
| F34L | | 1.845 | **1.192** | 1.686 |
| T35E | | 2.102 | 2.128 | 1.682 |
| E36K | | 1.689 | 1.930 | 1.674 |
| **<0.25** | | | | |
| **0.25<x<1.2** | | | | |
| 1.2<x<1.9 | | | | |

Multiple mutations of residues 1-5 almost completely abrogated 3C3. Point mutations of residues 1-4 did not reduce binding to 3C3. The point mutation of residue 5 dramatically reduced binding but not to the extent of the multiple mutation protein.

Multiple mutations of residues 13-15 did not reduce 3C3 binding. Multiple mutations of residues 25, 26, 28 and 30 caused a slight reduction in 3C3. Point mutations were not tested in these regions.

Multiple mutations of residues 33-36 almost completely abrogated 3C3 binding. The point mutation of residue 35 had no effect on binding. The point mutations of 33, 34 and 36 decreased 3C3 binding but not to the extent of the multiple mutation protein. An alternate CD40 antibody, 3G5, was tested for binding to all fragments and mutants and was shown to be different than 3C3 (Table 6). The multiple mutation of residues 1-5 did not reduce binding while the mutation of residues 33-36 eliminated binding. Unlike 3C3, the point mutation of residue 33 completely eliminated binding and the mutation of 34 significantly reduced binding.

### Example 18

### Biological and toxicity profile

A non-GLP pilot study was performed in naive cynomolgus macaques. This study was designed to provide preliminary data on the biological and toxicity profile of 3C3. An alternative anti-CD40 antibody (3G5) was also evaluated. The test articles were administered by intravenous injection in a saphenous vein on Day 1 (0.2 mg/kg or vehicle) and again on Day 29 (2 mg/kg or vehicle). Animals also received a subcutaneous (1 mg) injection of keyhole limpet hemocyanin (KLH) on Day 1 and 29. Evaluations for potential test article-related effects were based on clinical signs, body temperature, clinical pathology parameters (hematology, coagulation, clinical chemistry, and urinalysis), anti-drug antibodies, cytokines, T-cell dependent antibody response analyses (TDAR), flow cytometry, and toxicokinetic parameters. Body weights were recorded once prior to test article administration and weekly thereafter. This was designed as a survival study with no planned necropsy.

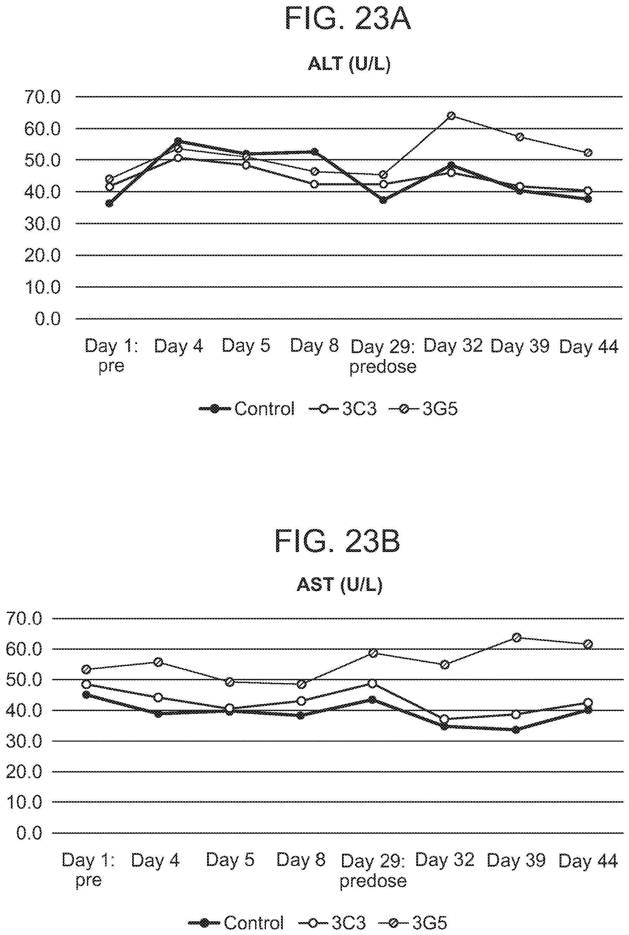

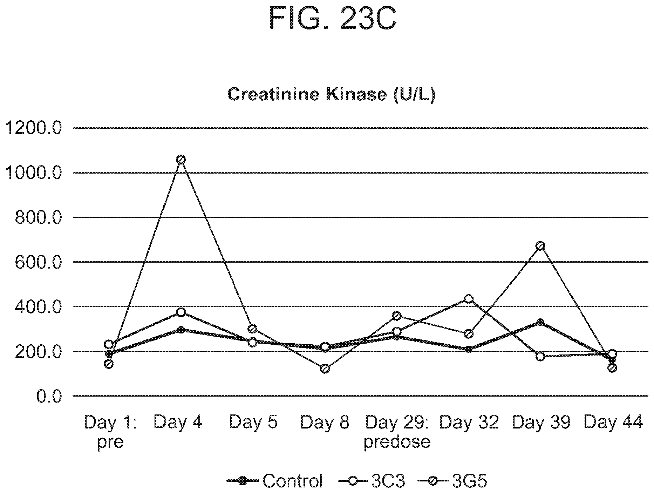

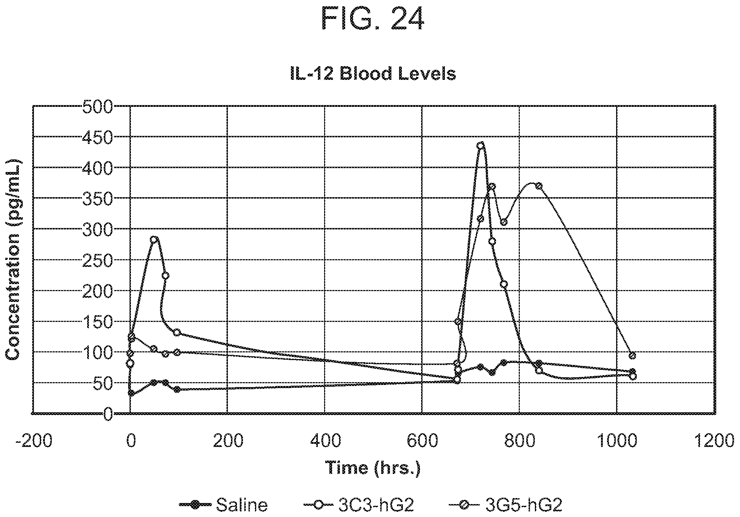

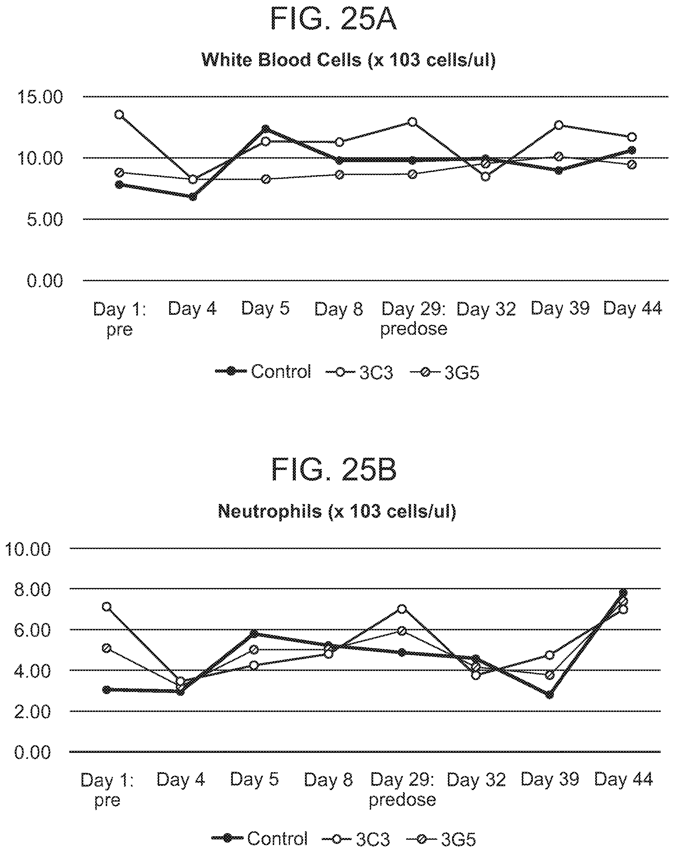

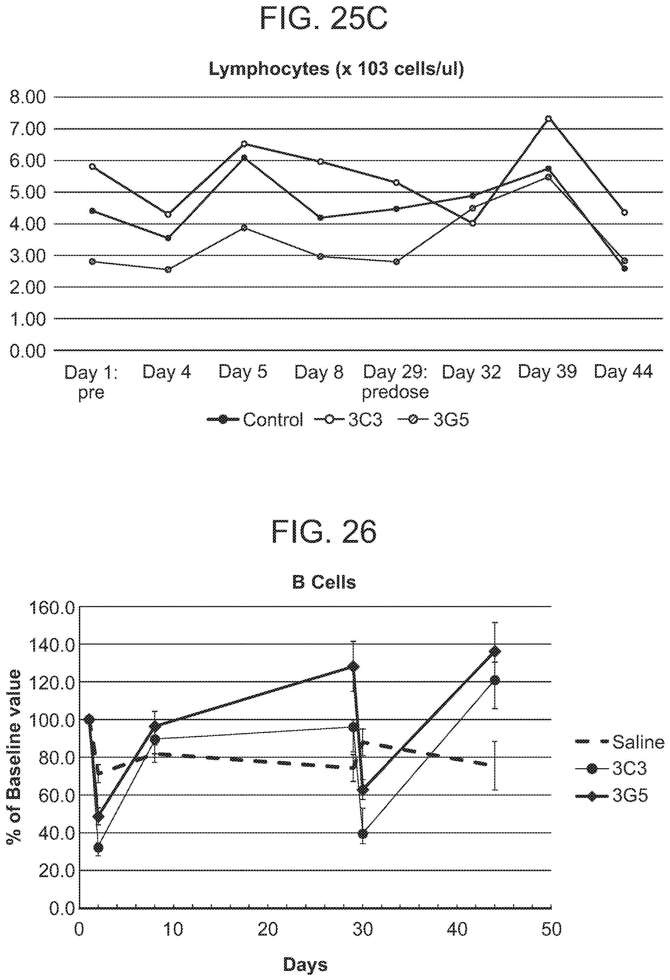

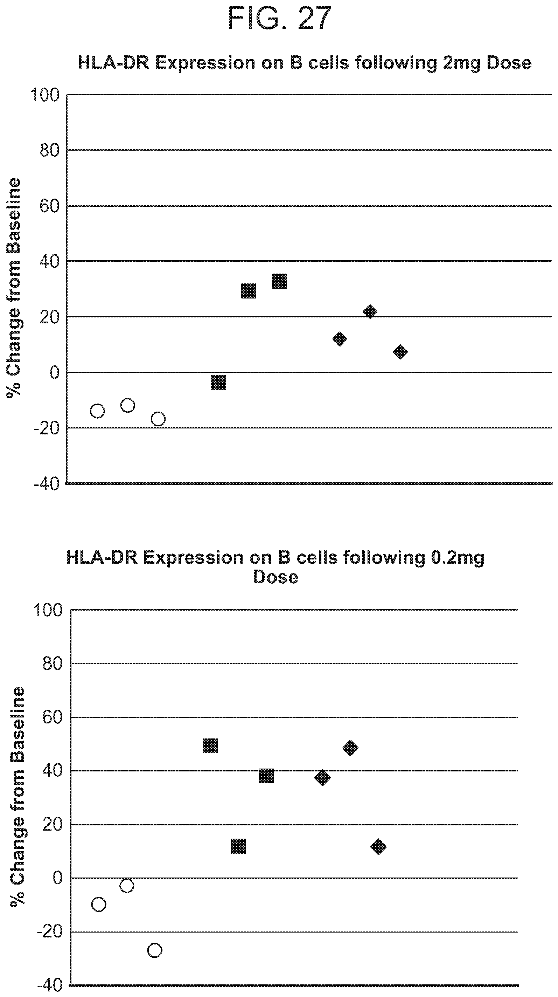

Administration of 3C3 or 3G5 in this study was well tolerated in cynomolgus monkeys without any toxicity parameter being significantly outside of control levels. Of note was the minimal elevations of asparate aminotransferase (AST), alanine aminotransferase (ALT) and creatinine kinase in monkeys dosed with 3C3 (Figures 23A-23C). Pharmacologic decreases in IL-12 (Figure 24) white blood cells (Figure 25A), neutrophils (Figure 25B) and lymphocytes (Figure 25C), were seen in both anti-CD40 dosed animals, with most significantly a transient decrease in B cells (Figures 26 and 27). In conclusion, 3C3 and 3G5 under the conditions of this showed minimal evidence of toxicity.

### Example 19

### B cell Proliferation independent of Fc interaction

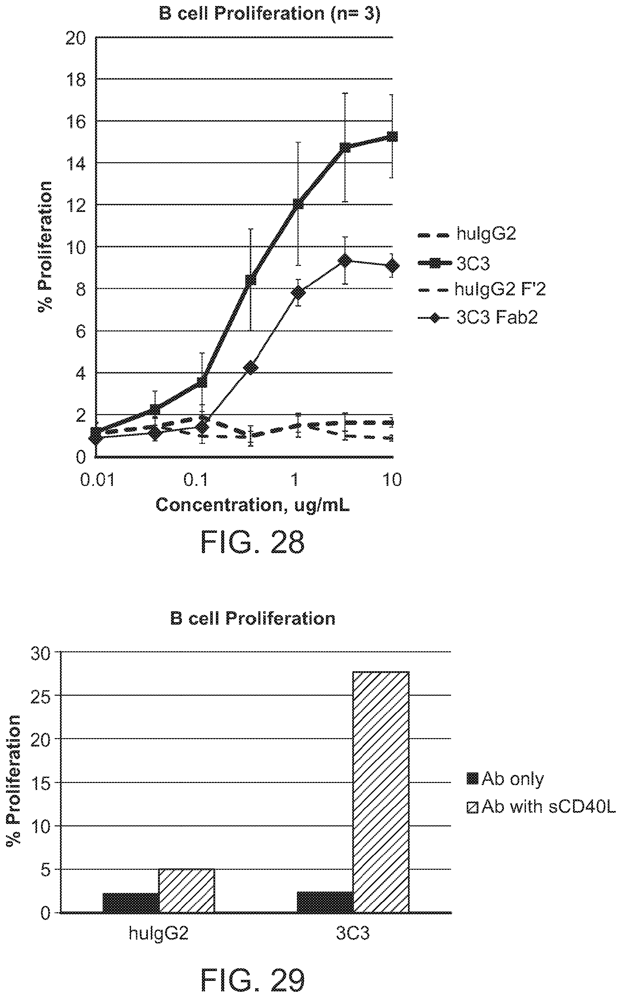

Human B cells were isolated from peripheral blood mononuclear cells by magnetic selection using CD19 beads. The cells were labeled with 0.5uM carboxyfluorescein succinimidyl ester (CFSE) at room temperature while rotating for 5 minutes. The labeled cells were cultured in the presence of either the anti-CD40 mAb 3C3 or an isotype control (both whole IgG and F(ab')2 fragments) for 6 days. Cells were then harvested and analyzed by flow cytometry for proliferation. The results are shown in Figure 28 and indicate that binding to the Fc receptor is not required for CD40 mediated proliferation with 3C3 because intact antibodies with Fc domains and their corresponding F(ab)'2 versions lacking Fc domains are both able to induce proliferation of B cells.

### Example 20

### Synergy with CD40L in Human B cells

Human B cells were isolated and labeled as in Example 19. The anti-CD40 mAb 3C3 or an isotype control at 0.1ug/mL were incubated with the cells for 6 days in the presence or absence of 0.1ug/mL soluble CD40L (Immunex). Figure 29 shows that no significant proliferation is observed with either the 3C3 alone or the isotype control antibody combined with CD40L, however proliferation is induced when CD40L is combined with 3C3 in the culture.

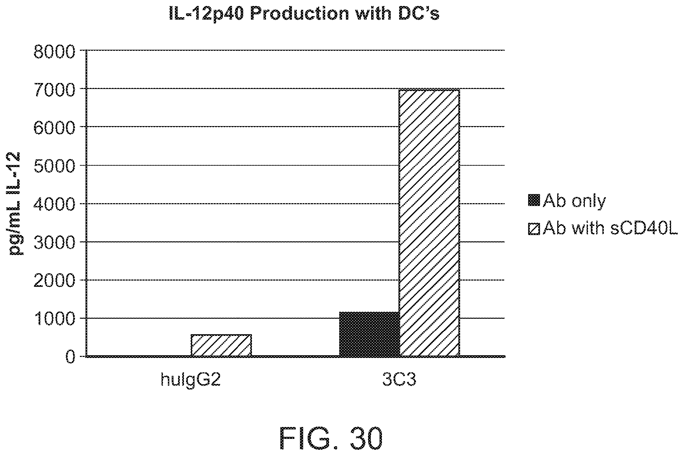

Dendritic cells were prepared and cultured with 0.5ug/mL of 3C3 as in Example 7 either with or without 0.1ug/mL soluble CD40L added. IL-12p40 production was measured by ELISA (R&D Systems). Figure 30 shows that relative to the low level of production by 3C3 alone or the isotype control with CD40L, the combination of 3C3 and CD40L induced higher levels of IL-12p40.

### Example 21

### Cytokine Response in Whole Blood

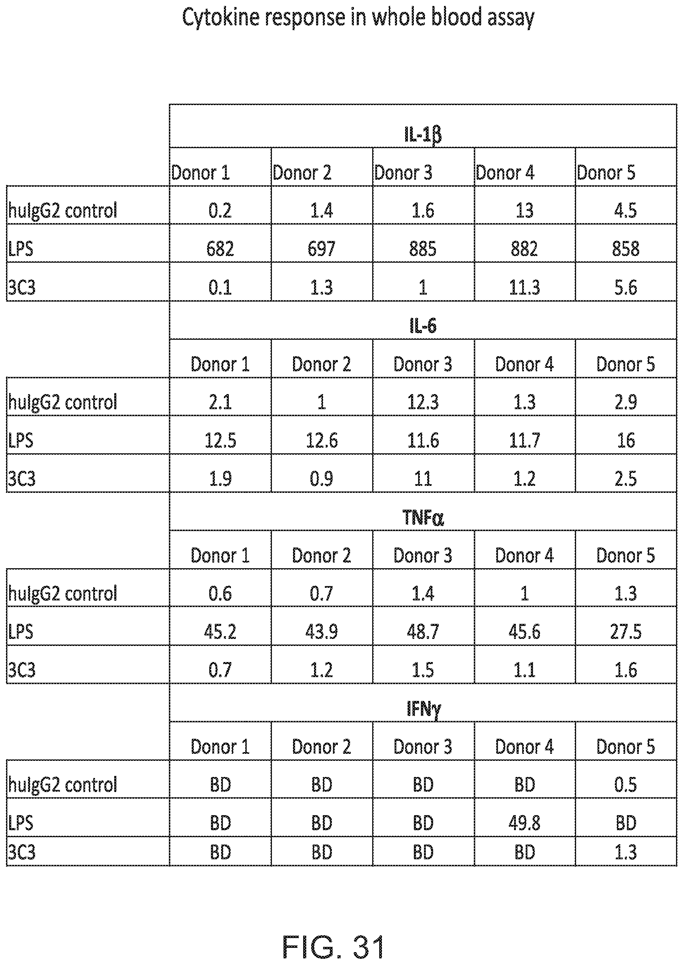

Whole blood was incubated overnight with 10ug/mL isotype control or 3C3, or LPS as a positive control. Next day, the plasma was collected and cytokines measured by ELISA (R&D Systems). The results are shown in Figure 31 and indicate no significant production of inflammatory cytokines.

### SUMMARY OF SEQUENCE LISTING

| **SEQ ID NO:** | **DESCRIPTION** |
|---|---|
| 1 | **Human CD40 (GenBank Accession No.: P25942)** |
| | MVRLPLQCVL WGCLLTAVHP EPPTACREKQ YLINSQCCSL CQPGQKLVSD CTEFTETECL PCGESEFLDT WNRETHCHQH KYCDPNLGLR VQQKGTSETD TICTCEEGWH CTSEACESCV LHRSCSPGFG VKQIATGVSD TICEPCPVGF FSNVSSAFEK CHPWTSCETK DLVVQQAGTN KTDVVCGPQD RLRALVVIPI IFGILFAILL VLVFIKKVAK KPTNKAPHPK QEPQEINFPD DLPGSNTAAP VQETLHGCQP VTQEDGKESR ISVQERQ |
| 2 | **Human CD40L (GenBank Accession No.: NP_000065)** |
| | MIETYNQTSP RSAATGLPIS MKIFMYLLTV FLITQMIGSA LFAVYLHRRL DKIEDERNLH EDFVFMKTIQ RCNTGERSLS LLNCEEIKSQ FEGFVKDIML NKEETKKENS FEMQKGDQNP QIAAHVISEA SSKTTSVLQW AEKGYYTMSN NLVTLENGKQ LTVKRQGLYY IYAQVTFCSN REASSQAPFI ASLCLKSPGR FERILLRAAN THSSAKPCGQ QSIHLGGVFE LQPGASVFVN VTDPSQVSHG TGFTSFGLLK |
| 3 | **3G5 - VH** |
| | QVQLVESGGGVVQPGKSLRLSCAASGFTFSSNGIHWVRQAPGKGLEWVAVI WSDGSNKFYADSVKGRFTISRDNSKNTLYLQMNSLRAEDTAVYYCARASG SGSYYNFFDYWGQGTLVTVSS |
| 4 | **3G5 - VL** |
| | EIVMTQSPATLSVSPGERATLSCRASQSVRSNLAWYQQKPGQAPRLLIYGAS TRATGIPARFSGSGSGTEFTLTINSLQSEDFAVYYCQQHNKWITFGQGTRLEI K |
| 5 | **3G5 - VH CDR1 (KABAT)** |
| | SNGIH |
| 6 | **3G5 - VH CDR1 (CHOTHIA)** |
| | GFTFSSN |
| 7 | **3G5 - VH CDR2 (KABAT)** |
| | VIWSDGSNKFYADSVKG |
| 8 | **3G5 - VH CDR2 (CHOTHIA)** |
| | WSDGSN |
| 9 | **3G5 - VH CDR3 (KABAT)** |
| | ASGSGSYYNFFDY |
| 10 | **3G5 - VH CDR3 (CHOTHIA)** |
| | ASGSGSYYNFFDY |
| 11 | **3G5 - VL CDR1 (KABAT)** |
| | RASQSVRSNLA |
| 12 | **3G5 - VL CDR1 (CHOTHIA)** |
| | RASQSVRSNLA |
| 13 | **3G5 - VL CDR2 (KABAT)** |
| | GASTRAT |
| 14 | **3G5 - VL CDR2 (CHOTHIA)** |
| | GASTRAT |
| 15 | **3G5 - VL CDR3 (KABAT)** |
| | QQHNKWIT |
| 16 | **3G5 - VL CDR3 (CHOTHIA)** |
| | QQHNKWIT |
| 17 | **3C3 - VH** |
| | QVQLVESGGGVVQPGRSLRLSCAGSGFIFSRYGMYWVRQAPGKGLEWVAV IWYDGSYKYYADSVKGRFTISRDNSKNTLYLQMNSLRAEDTAVYYCARES PWYYFDYWGQGTLVTVSS |
| 18 | **3C3 - VL** |
| | DIQMTQSPSSLSASVGDRVTITCRASQGISNYLAWYQQKPGKVPKLLIYAAS TLQSGVPSRFSGSGSGTDFTLTISSLQPEDVATYYCQKYKSAPFTFGPGTKVD IK |
| 19 | **3C3 - VH CDR1 (KABAT)** |
| | RYGMY |
| 20 | **3C3 - VH CDR1 (CHOTHIA)** |
| | GFIFSRY |
| 21 | **3C3 - VH CDR2 (KABAT)** |
| | VIWYDGSYKYYADSVKG |
| 22 | **3C3 - VH CDR2 (CHOTHIA)** |
| | WYDGSY |
| 23 | **3C3 - VH CDR3 (KABAT)** |
| | ESPWYYFDY |
| 24 | **3C3 - VH CDR3 (CHOTHIA)** |
| | ESPWYYFDY |
| 25 | **3C3 - VL CDR1 (KABAT)** |
| | RASQGISNYLA |
| 26 | **3C3 - VL CDR1 (CHOTHIA)** |
| | RASQGISNYLA |
| 27 | **3C3 - VL CDR2 (KABAT)** |
| | AASTLQS |
| 28 | **3C3 - VL CDR2 (CHOTHIA)** |
| | AASTLQS |
| 29 | **3C3 - VL CDR3 (KABAT)** |
| | QKYKSAPFT |
| 30 | **3C3 - VL CDR3 (CHOTHIA)** |
| | QKYKSAPFT |
| 31 | **3B6 - VH** |
| | EVQLLESGGGLVQPGGSLRLSCAASGFTFSSYAMSWVRQAPGKGLEWVSGI TGTGGSTYYADSVKGRFTISRDNSKNTLYVQMNSLRAEDTAVYYCAKRAG GSFYYYYGMDVWGQGTTVTVSS |
| 32 | **3B6 - VL** |
| | DIVMTQSPLSLPVTPGEPASISCRSSQSLLHSTGYNYLDWYLQKPGQSPQLLI YLGSNRASGVPDRFNGSGSGTDFTLKISRVEAEDFGVYYCMQALQTPWTFG HGTKVEIK |
| 33 | **3B6 - VH CDR1 (KABAT)** |
| | SYAMS |
| 34 | **3B6** - **VH CDR1 (CHOTHIA)** |
| | GFTFSSY |
| 35 | **3B6 - VH CDR2 (KABAT)** |
| | GITGTGGSTYYAD SVKG |
| 36 | **3B6** - **VH CDR2 (CHOTHIA)** |
| | TGTGGS |
| 37 | **3B6 - VH CDR3 (KABAT)** |
| | RAGGSFYYYYGMDV |
| 38 | **3B6** - **VH CDR3 (CHOTHIA)** |
| | RAGGSFYYYYGMDV |
| 39 | **3B6 - VL CDR1 (KABAT)** |
| | RSSQSLLHSTGYNYLD |
| 40 | **3B6 - VL CDR1 (CHOTHIA)** |
| | RSSQSLLHSTGYNYLD |
| 41 | **3B6 - VL CDR2 (KABAT)** |
| | LGSNRAS |
| 42 | **3B6 - VL CDR2 (CHOTHIA)** |
| | LGSNRAS |
| 43 | **3B6 - VL CDR3 (KABAT)** |
| | MQALQTPWT |
| 44 | **3B6** - **VL CDR3 (CHOTHIA)** |
| | MQALQTPWT |
| 45 | **6H6 - VH** |
| | QVQLVESGGGVVQPGRSLRFSCAASGFTLSSYGMHWVRQAPGKGLEWVA VIWDDGSNKYYADSVKGRFTISRDNSKNTLYLQMNSLRAEDTAVYYCARA GGSGRYYNYFDYWGQGTLVTVSS |
| 46 | **6H6 - VL** |
| | EIVMTQSPATLSVSPGERATLSCRASQSVRSNLAWYQQKPGQAPRLLIYGAS TRATGIPARFSGSGSGTDFTLTISSLQSEDFAVYYCQQHNNWLTFGGGTKVE IK |
| 47 | **6H6 - VH CDR1 (KABAT)** |
| | SYGMH |
| 48 | **6H6 - VH CDR1 (CHOTHIA)** |
| | GFTLSSY |
| 49 | **6H6 - VH CDR2 (KABAT)** |
| | VIWDDGSNKYYADSVKG |
| 50 | **6H6 - VH CDR2 (CHOTHIA)** |
| | WDDGSN |
| 51 | **6H6** - **VH CDR3 (KABAT)** |
| | AGGSGRYYNYFDY |
| 52 | **6H6** - **VH CDR3 (CHOTHIA)** |
| | AGGSGRYYNYFDY |
| 53 | **6H6 - VL CDR1 (KABAT)** |
| | RASQSVRSNLA |
| 54 | **6H6 - VL CDR1 (CHOTHIA)** |
| | RASQSVRSNLA |
| 55 | **6H6** - **VL CDR2 (KABAT)** |
| | GASTRAT |
| 56 | **6H6** - **VL CDR2 (CHOTHIA)** |
| | GASTRAT |
| 57 | **6H6** - **VL CDR3 (KABAT)** |
| | QQHNNWLT |
| 58 | **6H6** - **VL CDR3 (CHOTHIA)** |
| | QQHNNWLT |
| 59 | **1B4 - VH** |
| | EVQLLESGGGLVQPGGSLRLSCAASGFTFSSYAMTWVRQVPGKGLEWVSGI TGSGANTFYTDSVKGRFTISRDNSNNSLYLQMNSLRADDTAVYYCAKRNG GSYYYYYGMDVWGQGTTVTVSS |
| 60 | **1B4** - **VL** |
| | DIVMTQSPLSLPVTPGEPASISCRSSQSLLHSSGYNYLDWYLQKPGQSPQLLI YLGSNRASGVPDRFSGSGSGTDFTLKISRVEAEDVGVYYCMQALQIPWTFG QGTKVEIK |
| 61 | **1B4** - **VH CDR1 (KABAT)** |
| | SYAMT |
| 62 | **1B4** - **VH CDR1 (CHOTHIA)** |
| | GFTFSSY |
| 63 | **1B4 - VH CDR2 (KABAT)** |
| | GITGSGANTFYTDSVKG |
| 64 | **1B4 - VH CDR2 (CHOTHIA)** |
| | TGSGAN |
| 65 | **1B4 - VH CDR3 (KABAT)** |
| | RNGGSYYYYYGMDV |
| 66 | **1B4 - VH CDR3 (CHOTHIA)** |
| | RNGGSYYYYYGMDV |
| 67 | **1B4 - VL CDR1 (KABAT)** |
| | RSSQSLLHSSGYNYLD |
| 68 | **1B4** - **VL CDR1 (CHOTHIA)** |
| | RSSQSLLHSSGYNYLD |
| 69 | **1B4 - VL CDR2 (KABAT)** |
| | LGSNRAS |
| 70 | **1B4 - VL CDR2 (CHOTHIA)** |
| | LGSNRAS |
| 71 | **1B4 - VL CDR3 (KABAT)** |
| | MQALQIPWT |
| 72 | **1B4** - **VL CDR3 (CHOTHIA)** |
| | MQALQIPWT |
| 73 | **3B6-NS - VH** |
| | EVQLLESGGGLVQPGGSLRLSCAASGFTFSSYAMSWVRQAPGKGLEWVSGI TGTGGSTYYADSVKGRFTISRDNSKNTLYVQMNSLRAEDTAVYYCAKRAG GSFYYYYGMDVWGQGTTVTVSS |
| 74 | **3B6-NS - VL** |
| | DIVMTQSPLSLPVTPGEPASISCRSSQSLLHSTGYNYLDWYLQKPGQSPQLLI YLGSNRASGVPDRFSGSGSGTDFTLKISRVEAEDFGVYYCMQALQTPWTFG HGTKVEIK |
| 75 | **3B6-NS** - **VH CDR1 (KABAT)** |
| | SYAMS |
| 76 | **3B6-NS** - **VH CDR1 (CHOTHIA)** |
| | GFTFSSY |
| 77 | **3B6-NS** - **VH CDR2 (KABAT)** |
| | GITGTGGSTYYADSVKG |
| 78 | **3B6-NS - VH CDR2 (CHOTHIA)** |
| | TGTGGS |
| 79 | **3B6-NS - VH CDR3 (KABAT)** |
| | RAGGSFYYYYGMDV |
| 80 | **3B6-NS - VH CDR3 (CHOTHIA)** |
| | RAGGSFYYYYGMDV |
| 81 | **3B6-NS - VL CDR1 (KABAT)** |
| | RSSQSLLHSTGYNYLD |
| 82 | **3B6-NS - VL CDR1 (CHOTHIA)** |
| | RSSQSLLHSTGYNYLD |
| 83 | **3B6-NS** - **VL CDR2 (KABAT)** |
| | LGSNRAS |
| 84 | **3B6-NS** - VL **CDR2 (CHOTHIA)** |
| | LGSNRAS |
| 85 | **3B6-NS - VL CDR3 (KABAT)** |
| | MQALQTPWT |
| 86 | **3B6-NS - VL CDR3 (CHOTHIA)** |
| | MQALQTPWT |
| | |
| 87 | **2E1.2 - VH** |
| | QVQLVESGGGVVQPGRSLRLSCAASGFTFSSYGMHWVRQAPGKGLEWVA VIWDDGSNKYYADSVKGRFTISRDNSKNTLYLQMNSLRAEDTAVYYCARA GSSGRYYNYFDYWGQGTLVTVSS |
| 88 | **2E1.2 - VL2** |
| | EIVMTQSPATLSVSPGERATLSCRASQSVRSNLAWYQQKPGQAPRLLIYGAS TRATGIPDRFSGSGSGTEFTLTISSLQSEDFAVYHCQQYNKWLIFGGGTKVEI K |
| 89 | **2E1.2** - **VH CDR1 (KABAT)** |
| | SYGMH |
| 90 | **2E1.2 - VH CDR1 (CHOTHIA)** |
| | GFTFSSY |
| 91 | **2E1.2 - VH CDR2 (KABAT)** |
| | VIWDDGSNKYYADSVKG |
| 92 | **2E1.2 - VH CDR2 (CHOTHIA)** |
| | WDDGSN |
| 93 | **2E1.2 - VH CDR3 (KABAT)** |
| | AGSSGRYYNYFDY |
| 94 | **2E1.2** - **VH CDR3 (CHOTHIA)** |
| | AGSSGRYYNYFDY |
| 95 | **2E1.2** - **VL2 CDR1 (KABAT)** |
| | RASQSVRSNLA |
| 96 | **2E1.2** - **VL2 CDR1 (CHOTHIA)** |
| | RASQSVRSNLA |
| 97 | **2E1.2** - **VL2 CDR2 (KABAT)** |
| | GASTRAT |
| 98 | **2E1.2** - **VL2 CDR2 (CHOTHIA)** |
| | GASTRAT |
| 99 | **2E1.2 -VL2 CDR3 (KABAT)** |
| | QQYNKWLI |
| 100 | **2E1.2** - **VL2 CDR3 (CHOTHIA)** |
| | QQYNKWLI |
| | |
| 101 | **1B5-NK - VH** |
| | QVQLVESGGGVVQPGRSLRLSCAASGFTFSSFGMHWVRQAPGKGLEWVTL IWFDGSSKYYADSVKGRFTISRDNSKNTLYLQMNSLRAEDTAVYYCVRGFA AVAGWYFDFWGRGTLVTVSS |
| 102 | **1B5-NK - VL** |
| | DIQMTQSPSSLSASVGDRVTITCRASQGVRKYLAWYQQKPGKVPKLLIYAA STLQSGVPSRFSGSGSGTDFTLTISSLQPEDVATYYCQKYFSAPYTFGQGTKL EIK |
| 103 | **1B5-NK - VH CDR1 (KABAT)** |
| | SFGMH |
| 104 | **1B5-NK** - **VH CDR1 (CHOTHIA)** |
| | GFTFSSF |
| 105 | **1B5-NK** - **VH CDR2 (KABAT)** |
| | LIWFDGSSKYYADSVKG |
| 106 | **1B5-NK** - **VH CDR2 (CHOTHIA)** |
| | WFDGSS |
| 107 | **1B5-NK** - **VH CDR3 (KABAT)** |
| | GFAAVAGWYFDF |
| 108 | **1B5-NK** - **VH CDR3 (CHOTHIA)** |
| | GFAAVAGWYFDF |
| 109 | **1B5-NK** - **VL CDR1 (KABAT)** |
| | RASQGVRKYLA |
| 110 | **1B5-NK** - **VL CDR1 (CHOTHIA)** |
| | RASQGVRKYLA |
| 111 | **1B5-NK** - **VL CDR2 (KABAT)** |
| | AASTLQS |
| 112 | **1B5-NK** - **VL CDR2 (CHOTHIA)** |
| | AASTLQS |
| 113 | **1B5-NK -VL CDR3 (KABAT)** |
| | QKYFSAPYT |
| 114 | **1B5-NK** - **VL CDR3 (CHOTHIA)** |
| | QKYFSAPYT |
| | |
| 115 | **3G5 VH with leader sequence underlined** |
| | Atggagtttgggctgacctgggttttcctcgttgctcttttaagaggtgtccagtgtcaggtgcagttggtggaatct Gggggaggcgtggtccagcctgggaagtccctgagactctcctgtgcagcgtctggattcaccttcagtagcaatg Gcattcactgggtccgccaggctccaggcaaggggctggagtgggtggcagttatctggtctgatggaagtaataa Attctatgcagactccgtgaagggccgattcaccatctccagagacaattccaagaacacgctatatctgcaaatga Acagcctgagagccgaggacacggctgtatattactgtgcgagagcctctggttcggggagttattataacttctttg actactggggccagggaaccctggtcaccgtctcctca |
| 116 | **3G5 VL with leader sequence underline** |
| | Atggaagccccagcgcagcttctcttcctcctgctactctggctcccagatagcactggagaaatagtgatgacgcag Tctccagccaccctgtctgtgtctccaggggaaagagccaccctctcctgcagggccagtcagagtgttagaagtaac Ttagcctggtaccagcagaaacctggccaggctcccaggctcctcatctatggtgcatccaccagggccactggtatcc Cagccaggttcagtggcagtgggtctgggacagagttcactctcaccatcaacagcctgcagtctgaagattttgcagt ttattactgtcagcagcataataagtggatcaccttcggccaagggacacgactggagattaaa |
| 117 | **3C3 VH with leader sequence underlined** |
| | Atggagtttgggctgagctgggttttcctcgttgctcttttaagaggtgtccagtgtcaggtgcagctggtggagtctgg Gggaggcgtggtccagcctgggaggtccctgagactctcctgtgcagggtctggattcattttcagtcgctatggcatg Tactgggtccgccaggctccaggcaaggggctggagtgggtggcagttatatggtatgatggaagttataaatactat Gcagactccgtgaagggccgattcaccatctccagagacaattccaagaacacgctgtatctgcaaatgaacagcctg Agagccgaggacacggctgtgtattactgtgcgagagaatcaccatggtactactttgactactggggccagggaacc ctggtcaccgtctcctct |
| 118 | **3C3 VL with leader sequence underlined** |
| | Atggacatgagggtccctgctcagctcctgggactcctgctctggctcccagataccagatgtgacatccagatgac Ccagtctccatcctccctgtctgcatctgtaggagacagagtcaccatcacttgccgggcgagtcagggcattagcaatta Tttagcctggtatcagcagaaaccagggaaagttcctaagctcctgatctatgctgcatccactttgcaatcaggggtccc Atctcggttcagtggcagtggatctgggacagatttcactctcaccatcagcagcctgcagcctgaagatgttgcaactta ttactgtcaaaagtataagagtgccccattcactttcggccctgggaccaaagtggatatcaaa |
| 119 | **3B6 VH with leader sequence underlined** |
| | Atggagtttgggctgagctggctttttcttgtggctattttaaaaggtgtccagtgtgaggtgcagctgttggagtctgggg Gaggcttggtacagcctggggggtccctgagactctcctgtgcagcctctggattcacctttagcagctatgccatgagct Gggtccgccaggctccagggaaggggctggagtgggtctcaggtataactggtactggtggtagcacatactacgcag Actccgtgaagggccggttcaccatctccagagacaattccaagaacacgctgtatgtgcaaatgaacagcctgagagc Cgaggacacggccgtatattactgtgcgaaaagggctggtgggagcttctactactactacggtatggacgtctggggcc aagggaccacggtcaccgtctcctca |
| 120 | **3B6 VL with leader sequence underlined** |
| | Atgaggctccctgctcagctcctggggctgctaatgctctgggtctctggatccagtggggatattgtgatgactcagtctc Cactctccctgcccgtcacccctggagagccggcctccatctcctgcaggtctagtcagagcctcctgcatagtactggata Caactatttggattggtacctgcagaagccagggcagtctccacagctcctgatctatttgggttctaatcgggcctccggg Gtccctgacaggttcaatggcagtggatcaggcacagattttacactgaaaatcagcagagtggaggctgaggattttgg ggtttattactgcatgcaagctctacaaactccgtggacgttcggccacgggaccaaggtggaaatcaaa |
| 121 | **6H6 VH with leader sequence underlined** |
| | Atggagtttgggctgagctgggtattcctcgttgctcttttaagaggtgtccagtgtcaggtgcagctggtggagtctgggg Gaggcgtggtccagcctgggaggtccctgagattctcctgtgcagcgtctggattcaccctcagtagctatggcatgcactg Ggtccgccaggctccaggcaaggggctggagtgggtggcagttatatggatgatggaagtaataaatactatgcagact Ccgtgaagggccgattcaccatctccagagacaattccaagaacacgctgtatctgcaaatgaacagcctgagagccgagg Acacggctgtctattactgtgcgagagcgggggggttcggggaggtattataactactttgactactggggccagggaaccct |
| | ggtcaccgtctcctca |
| 122 | **6H6 VL with leader sequence underlined** |
| | Atggaagccccagcgcagcttctcttcctcctgctactctggctcccagataccactggagaaatagtgatgacgcagtctcc Agccaccctgtctgtgtctccaggggaaagagccaccctctcctgcagggccagtcagagtgttagaagcaacttagcctgg Taccagcagaaacctggccaggctcccaggctcctcatctatggtgcatccaccagggccactggtatcccagccaggttcag Tggcagtgggtctgggacagacttcactctcaccatcagcagcctgcagtctgaagattttgcagtttattactgtcagcagca Taataactggctcactttcggcggagggaccaaggtggagatcaaa |
| 123 | **1B4 VH with leader sequence underlined** |
| | Atggagtttgggctgagctggctttttcttgtggctattttaaaaggtgtccaatgtgaggtgcagctgttggaatctggggga Ggcttggtacagcctggggggtccctgagactctcctgtgcggcctctgggttcacctttagcagctatgccatgacctgggtc Cgccaggttccagggaagggcctggagtgggtctcaggtattactggtagtggtgctaacacattctacacagactccgtga Agggccggttcaccatttccagagacaattccaataattcgctgtatctgcaaatgaacagcctgagagccgatgacacggc Cgtatactactgtgcgaaaagaaatggtgggagttactactactactacggcatggacgtctggggccaagggaccacggtc accgtgtcctca |
| 124 | **1B4 VL with leader sequence underlined** |
| | Atgaggctccctgctcagctcctggggctgctaatgctctgggtctctggatccagtggggatattgtgatgactcagtctccac Tctccctgcccgtcacccctggagagccggcctccatctcctgcaggtcaagtcagagcctcctgcatagtagtggatacaacta Tttggattggtacctgcagaagccagggcagtctccacaactcctgatctatttgggttctaatcgggcctccggggtccctgac Aggttcagtggcagtggatcaggcacagattttacactgaaaatcagcagagtggaggctgaggatgttggggtttattactg catgcaagctctacaaattccgtggacgttcggccaagggaccaaggtggaaatcaaa |
| 125 | **2E1.2 VH with leader sequence underlined** |
| | Atggagtttgggctgagctgggttttcctcgttgctcttttaagaggtgtccagtgtcaggtgcagctggtggagtctgggggaggc gtggtCcagcctgggaggtccctgagactctcctgtgcagcgtctggattcaccttcagtagctatggcatgcactgggtccgcca ggctccaggcaAggggctggagtgggtggcagttatatggatgatggaagtaataaatactatgcagactccgtgaagggccg attcaccatctccagagAcaattccaagaacacgctgtatctgcaaatgaacagcctgagagccgaggacacggctgtgtattac tgtgcgagagcgggaagttcggggaggtattataactactttgactactggggccagggaaccctggtcaccgtctcctca |
| 126 | **2E1.2 VL2 with leader sequence underlined** |
| | Atggaagccccagcgcagcttctcttcctcctgctactctggctcccagataccactggagaaatagtgatgacgcagt ctccagccaccctgtctgtgtctccaggggaaagagccaccctctcctgcagggccagtcagagtgttaggagcaactt agcctggtatcagcagaaacctggccaggctcccaggctcctcatctatggtgcatccaccagggccactggtatccc agacaggttcagtggcagtgggtctgggacagagttcactctcaccatcagcagcctgcagtctgaagattttgcagtt tatcactgtcagcagtataataagtggctcattttcggcggagggaccaaggtggagatcaaa |
| 127 | **1B5 VH with leader sequence underlined** |
| | Atggagtttgggctgagctgggttttcctcgttgctcttttaagaggtgtccagtgtcaggtgcagctggtggagtctgggggaggc gtggtccagcCtgggaggtccctgagactctcctgtgcagcgtctggattcaccttcagtagctttggcatgcactgggtccgccag gctccaggcaaggggctggaGtgggtgacacttatatggtttgatggaagttctaaatactatgcagactccgtgaagggccgat tcaccatctccagagacaactccaacaacacGctgtatctgcaaatgaacagcctgagagccgaggacacggctgtatattact gtgtgagaggttttgcagcagtggctgggtggtacttcgatttctggggccgtggcaccctggtcactgtctcctca |
| 128 | **1B5 VL with leader sequence underlined** |
| | Atggacatgagggtccctgctcagctcctgggactcctgctgctctggctcccagataccagatgtgacatccagatgacccagtc tccatcctcccTgtctgcatctgtaggagacagagtcaccatcacttgccgggcgagtcagggcgttagaaagtatttagcctggt atcagcagaaaccagggaaAgttcctaagctcctgatctatgctgcatccactttgcaatcaggggtcccatctcggttcagtggc agtggatctgggacagatttcactctcaccaTcagcagcctgcagcctgaagatgttgcaacttattactgtcaaaagtatttcagt gccccgtacacttttggccaggggaccaaactggagatcaaa |
| 129 | **3B6-NS VH with leader sequence underlined** |
| | Atggagtttgggctgagctggctttttcttgtggctattttaaaaggtgtccagtgtgaggtgcagctgttggagtctggg ggaggcttggtacagcctggggggtccctgagactctcctgtgcagcctctggattcacctttagcagctatgccatga |
| | gctgggtccgccaggctccagggaaggggctggagtgggtctcaggtataactggtactggtggtagcacatactacg cagactccgtgaagggccggttcaccatctccagagacaattccaagaacacgctgtatgtgcaaatgaacagcctg agagccgaggacacggccgtatattactgtgcgaaaagggctggtgggagcttctactactactacggtatggacgtc tggggccaagggaccacggtcaccgtctcctca |
| 130 | **3B6-NS VL with leader sequence underlined** |
| | atgaggctccctgctcagctcctggggctgctaatgctctgggtctctggatccagtggggatattgtgatgactcagtc tccactctccctgcccgtcacccctggagagccggcctccatctcctgcaggtctagtcagagcctcctgcatagtactg gatacaactatttggattggtacctgcagaagccagggcagtctccacagctcctgatctatttgggttctaatcgggcc tccggggtccctgacaggttcagtggcagtggatcaggcacagattttacactgaaaatcagcagagtggaggctga ggattttggggtttattactgcatgcaagctctacaaactccgtggacgttcggccacgggaccaaggtggaaatcaa a |
| 131 | **1B5-NK VH with leader sequence underlined** |
| | atggagtttgggctgagctgggttttcctcgttgctcttttaagaggtgtccagtgtcaggtgcagctggtggagtctgg gggaggcgtggtccagcctgggaggtccctgagactctcctgtgcagcgtctggattcaccttcagtagctttggcatg cactgggtccgccaggctccaggcaaggggctggagtgggtgacacttatatggtttgatggaagttctaaatactatg cagactccgtgaagggccgattcaccatctccagagacaactccaagaacacgctgtatctgcaaatgaacagcctg agagccgaggacacggctgtatattactgtgtgagaggttttgcagcagtggctgggtggtacttcgatttctggggcc gtggcaccctggtcactgtctcctca |
| 132 | **1B5-NK VL with leader sequence underlined** |
| | Atggacatgagggtccctgctcagctcctgggactcctgctgctctggctcccagataccagatgtgacatccagatga cccagtctccatcctccctgtctgcatctgtaggagacagagtcaccatcacttgccgggcgagtcagggcgttagaaa gtatttagcctggtatcagcagaaaccagggaaagttcctaagctcctgatctatgctgcatccactttgcaatcaggg gtcccatctcggttcagtggcagtggatctgggacagatttcactctcaccatcagcagcctgcagcctgaagatgttg caacttattactgtcaaaagtatttcagtgccccgtacacttttggccaggggaccaaactggagatcaaa |
| 133 | **Human CD40 Extracellular Domain** |
| | EPPTACREKQYLINSQCCSLCQPGQKLVSDCTEFTETECLPCGESEFLDTWN RETHCHQHKYCDPNLGLRVQQKGTSETDTICTCEEGWHCTSEACESCVLHR SCSPGFGVKQIATGVSDTICEPCPVGFFSNVSSAFEKCHPWTSCETKDLVVQ QAGTNKTDVVCGPQDRLR |
| 134 | **Immunoglobulin heavy constant gamma 2 (IgHG2) (Uniprot P01859)** |
| | ASTKGPSVFPLAPCSRSTSESTAALGCLVKDYFPEPVTVSWNSGALTSGV HTFPAVLQSSGLYSLSSVVTVPSSNFGTQTYTCNVDHKPSNTKVDKTVER KCCVECPPCPAPPVAGPSVFLFPPKPKDTLMISRTPEVTCVVVDVSHEDP EVQFNWYVDGVEVHNAKTKPREEQFNSTFRVVSVLTVVHQDWLNGKEYK CKVSNKGLPAPIEKTISKTKGQPREPQVYTLPPSREEMTKNQVSLTCLVKG FYPSDISVEWESNGQPENNYKTTPPMLDSDGSFFLYSKLTVDKSRWQQGN VFSCSVMHEALHNHYTQKSLSLSPGK |
| 135 | **3C3 heavy chain with variable region in italics and constant domain in bold** |
| | QVQLVESGGGVVQPGRSLRLSCAGSGFIFSRYGMYWVRQAPGKGLEWVAVIWY DGSYKYYADSVKGRFTISRDNSKNTLYLQMNSLRAEDTAVYYCARESPWYYFDYW GQGTLVTVSSASTKGPSVFPLAPCSRSTSESTAALGCLVKDYFPEPVTVSW NSGALTSGVHTFPAVLQSSGLYSLSSVVTVPSSNFGTQTYTCNVDHKPSN TKVDKTVERKCCVECPPCPAPPVAGPSVFLFPPKPKDTLMISRTPEVTC VVVDVSHEDPEVQFNWYVDGVEVHNAKTKPREEQFNSTFRVVSVLTVV |
| | HQDWLNGKEYKCKVSNKGLPAPIEKTISKTKGQPREPQVYTLPPSREE MTKNQVSLTCLVKGFYPSDIAVEWESNGQPENNYKTTPPMLDSDGSFF LYSKLTVDKSRWQQGNVFSCSVMHEALHNHYTQKSLSLSPG |
| 136 | **3C3 light chain with variable region in italics and constant domain in bold** |
| | DIQMTQSPSSLSASVGDRVTITCRASQGISNYLAWYQQKPGKVPKLLIYAASTLQS GVPSRFSGSGSGTDFTLTISSLQPEDVATYYCQKYKSAPFTFGPGTKVDIKRTVA APSVFIFPPSDEQLKSGTASVVCLLNNFYPREAKVQWKVDNALQSGNSQ ESVTEQDSKDSTYSLSSTLTLSKADYEKHKVYACEVTHQGLSSPVTKSF NRGEC |
| 136 | **3C3 heavy chain with leader sequence underlined, variable region in italics and constant domain in bold** |
| | MEFGLSWVFLVALLRGVQCQVQLVESGGGVVQPGRSLRLSCAGSGFIFSRYG MYWVRQAPGKGLEWVAVIWYDGSYKYYADSVKGRFTISRDNSKNTLYLQMNSLR AEDTAVYYCARESPWYYFDYWGQGTLVTVSSASTKGPSVFPLAPCSRSTSEST AALGCLVKDYFPEPVTVSWNSGALTSGVHTFPAVLQSSGLYSLSSVVTV PSSNFGTQTYTCNVDHKPSNTKVDKTVERKCCVECPPCPAPPVAGPSVF LFPPKPKDTLMISRTPEVTCVVVDVSHEDPEVQFNWYVDGVEVHNAKT KPREEQFNSTFRVVSVLTVVHQDWLNGKEYKCKVSNKGLPAPIEKTISK TKGQPREPQVYTLPPSREEMTKNQVSLTCLVKGFYPSDIAVEWESNGQ PENNYKTTPPMLDSDGSFFLYSKLTVDKSRWQQGNVFSCSVMHEALHN HYTQKSLSLSPG |
| 137 | **3C3 light chain with leader sequence underlined, variable region in italics and constant domain in bold** |
| | MGWSCIILFLVATATGVHSDIQMTQSPSSLSASVGDRVTITCRASQGISNYLAWY QQKPGKVPKLLIYAASTLQSGVPSRFSGSGSGTDFTLTISSLQPEDVATYYCQKYK SAPFTFGPGTKVDIKRTVAAPSVFIFPPSDEQLKSGTASVVCLLNNFYPREA KVQWKVDNALQSGNSQESVTEQDSKDSTYSLSSTLTLSKADYEKHKVY ACEVTHQGLSSPVTKSFNRGEC |

## Claims

1. An isolated antibody which binds to human CD40, and comprises heavy chain CDR1, CDR2, and CDR3 sequences comprising SEQ ID NOs: 19, 21, 23, respectively, and light chain CDR1, CDR2, and CDR3 sequences comprising SEQ ID NOs: 25, 27, 29, respectively.

2. The antibody of claim 1, which comprises heavy and light chain variable regions having amino acid sequences at least 95% identical to SEQ ID Nos: 17 and 18.

3. The antibody of claim 1, which comprises heavy and light chain variable regions having amino acid sequences at least 98% identical to SEQ ID Nos: 17 and 18.

4. The antibody of claim 1, which comprises heavy and light chain variable regions having the amino acid sequences as set forth in SEQ ID Nos: 17 and 18, respectively.

5. The antibody of any one of claims 1 to 4, wherein:
(a) the antibody is a human antibody; and/or
(b) the antibody comprises a human constant region.

6. The antibody of any one of claims 1 to 4, wherein the antibody is an antigen binding fragment, a Fab, Fab', (Fab')2, Fv, or scFv fragment.

7. A molecular conjugate comprising the antibody of any one of claims 1 to 6, linked to an antigen.

8. A bispecific molecule comprising the antibody of any one of claims 1 to 6 linked to a second molecule having a binding specificity which is different from the antibody of any one of claims 1 to 6.

9. A composition comprising the antibody, molecular conjugate, or bispecific molecule of any one of claims 1 to 9 and a carrier, optionally further comprising an adjuvant, and/or one or more other antibodies.

10. The composition of claim 9, wherein the one or more other antibodies bind to CTLA-4, PD-1, PD-L1, LAG-3, TIM-3, Galectin 9, CEACAM-1, BTLA, CD69, Galectin 1, TIGIT, CD113, GPR56, VISTA, B7-H3, B7-H4, 2B4, CD48, GARP, PD1H, LAIR1, TIM-1, TIM-4, B7-1, B7-2, CD28, 4-1BB (CD137), 4-1BBL, ICOS, ICOS-L, OX40, OX40L, CD70, CD27, DR3 or CD28H.

11. The antibody, molecular conjugate, bispecific antibody or composition of any one of claims 1 to 10 for use in a method for inducing or enhancing an immune response against an antigen in a subject, optionally wherein the method further comprises administering the antigen simultaneously, separately or sequentially from the antibody, molecular conjugate, composition, or bispecific molecule.

12. An *ex vivo* method of inhibiting growth of CD40 expressing cells comprising contacting the cells with the antibody, composition or bispecific molecule of any one of claims 1 to 10, in an amount effective to inhibit growth of CD40 expressing cells.

13. The antibody, molecular conjugate, bispecific antibody or composition of any one of claims 1 to 10 for use in a method of treating a subject by therapy.

14. The antibody, molecular conjugate, bispecific antibody or composition of any one of claims 1 to 10, for use in a method of treating a cancer selected from the group consisting of chronic lymphocytic leukemia, mantle cell lymphoma, primary central nervous system lymphoma, Burkitt's lymphoma and marginal zone B cell lymphoma.

15. The antibody, molecular conjugate, bispecific antibody or composition for use according to claim 13 or 14, wherein the method further comprises administering one or more therapeutic agents to the subject, optionally wherein the therapeutic agent is another antibody, such as an anti-PD-1 antibody, an anti-PD-L1, and/or an anti-CTLA-4 antibody, and/or wherein the first and second antibodies are administered concurrently, or sequentially.

## Patentansprüche

1. Isolierter Antikörper, der an humanes CD40 bindet und CDR1-, CDR2- und CDR3-Sequenzen der schweren Kette, umfassend SEQ ID NO: 19, 21 bzw. 23, und CDR1-, CDR2- und CDR3-Sequenzen der leichten Kette umfasst, umfassend SEQ ID NO: 25, 27 bzw. 29.

2. Antikörper nach Anspruch 1, der variable Regionen der schweren und leichten Kette umfasst, mit Aminosäuresequenzen, die mindestens 95 % identisch zu SEQ ID NO: 17 und 18 sind.

3. Antikörper nach Anspruch 1, der variable Regionen der schweren und leichten Kette umfasst, mit Aminosäuresequenzen, die mindestens 98 % identisch zu SEQ ID NO: 17 und 18 sind.

4. Antikörper nach Anspruch 1, der variable Regionen der schweren und leichten Kette umfasst, mit den Aminosäuresequenzen gemäß SEQ ID NO: 17 bzw. 18.

5. Antikörper nach einem der Ansprüche 1 bis 4, wobei:
(a) der Antikörper ein humaner Antikörper ist; und/oder
(b) der Antikörper eine humane konstante Region umfasst.

6. Antikörper nach einem der Ansprüche 1 bis 4, wobei der Antikörper ein antigenbindendes Fragment, ein Fab-, Fab'-, (Fab')2-, Fv- oder scFv-Fragment ist.

7. Molekularkonjugat, umfassend den Antikörper nach einem der Ansprüche 1 bis 6, verknüpft mit einem Antigen.

8. Bispezifisches Molekül, umfassend den Antikörper nach einem der Ansprüche 1 bis 6, verknüpft mit einem zweiten Molekül, das eine Bindungsspezifität aufweist, die anders als der Antikörper nach einem der Ansprüche 1 bis 6 ist.

9. Zusammensetzung, umfassend den Antikörper, das Molekularkonjugat oder das bispezifische Molekül nach einem der Ansprüche 1 bis 9 und einen Träger, optional ferner umfassend einen Hilfsstoff und/oder einen oder mehrere andere Antikörper.

10. Zusammensetzung nach Anspruch 9, wobei der eine oder die mehreren anderen Antikörper an CTLA-4, PD-1, PD-L1, LAG-3, TIM-3, Galectin 9, CEACAM-1, BTLA, CD69, Galectin 1, TIGIT, GD113, GPR56, VISTA, B7-H3, B7-H4, 2B4, CD48, GARP, PD1H, LAIR1, TIM-1, TIM-4, B7-1, B7-2, CD28, 4-1BB (CD137), 4-1BBL, ICOS, ICOS-L, OX40, OX40L, CD70, CD27, DR3 oder CD28H binden.

11. Antikörper, Molekularkonjugat, bispezifischer Antikörper oder Zusammensetzung nach einem der Ansprüche 1 bis 10 zur Verwendung in einem Verfahren zum Induzieren oder Verstärken einer Immunantwort gegen ein Antigen in einem Subjekt, wobei optional das Verfahren ferner das Verabreichen des Antigens simultan, separat oder sequenziell von dem Antikörper, dem Molekularkonjugat, der Zusammensetzung oder dem bispezifischen Molekül umfasst.

12. *Ex-vivo*-Verfahren des Hemmens des Wachstums von CD40-exprimierenden Zellen, umfassend das Kontaktieren der Zellen mit dem Antikörper, der Zusammensetzung oder dem bispezifischen Molekül nach einem der Ansprüche 1 bis 10, in einer Menge, die wirksam ist, um das Wachstum von CD40-exprimierenden Zellen zu hemmen.

13. Antikörper, Molekularkonjugat, bispezifischer Antikörper oder Zusammensetzung nach einem der Ansprüche 1 bis 10 zur Verwendung in einem Verfahren des Behandelns eines Subjekts durch Therapie.

14. Antikörper, Molekularkonjugat, bispezifischer Antikörper oder Zusammensetzung nach einem der Ansprüche 1 bis 10 zur Verwendung in einem Verfahren des Behandelns eines Krebses, ausgewählt aus der Gruppe bestehend aus chronisch lymphatischer Leukämie, Mantelzelllymphom, primärem Lymphom des zentralen Nervensystems, Burkitt-Lymphom und Marginalzonen-B-Zell-Lymphom.

15. Antikörper, Molekularkonjugat, bispezifischer Antikörper oder Zusammensetzung zur Verwendung nach Anspruch 13 oder 14, wobei das Verfahren ferner das Verabreichen von einem oder mehreren Therapeutika an das Subjekt umfasst, wobei optional das Therapeutikum ein anderer Antikörper ist, wie z. B. ein Anti-PD-1-Antikörper, ein Anti-PD-L1- und/oder ein Anti-CTLA-4-Antikörper, und/oder wobei der erste und zweite Antikörper gleichzeitig oder sequenziell verabreicht werden.

## Revendications

1. Anticorps isolé qui se lie au CD40 humain et comprend des séquences CDR1, CDR2 et CDR3 de chaînes lourdes comprenant respectivement les SEQ ID No : 19, 21, 23, et des séquences CDR1, CDR2 et CDR3 de chaînes légères comprenant respectivement les SEQ ID No : 25, 27, 29.

2. Anticorps selon la revendication 1, lequel comprend des régions variables de chaînes lourdes et légères ayant des séquences d'acides aminés présentant au moins 95 % d'identité avec les SEQ ID No : 17 et 18.

3. Anticorps selon la revendication 1, lequel comprend des régions variables de chaînes lourdes et légères ayant des séquences d'acides aminés présentant au moins 95 % d'identité avec les SEQ ID No : 17 et 18.

4. Anticorps selon la revendication 1, lequel comprend des régions variables de chaînes lourdes et légères ayant les séquences d'acide aminés présentées respectivement dans les SEQ ID No : 17 et 18.

5. Anticorps selon l'une quelconque des revendications 1 à 4, lequel anticorps :
(a) est un anticorps humain, et/ou
(b) comprend une région constante humaine.

6. Anticorps selon l'une quelconque des revendications 1 à 4, lequel anticorps est un fragment de liaison à l'antigène, un fragment Fab, Fab', (Fab')2, Fv ou scFv.

7. Conjugué moléculaire comprenant l'anticorps selon l'une quelconque des revendications 1 à 6, lié à un antigène.

8. Molécule bispécifique comprenant l'anticorps selon l'une quelconque des revendications 1 à 6, lié à une deuxième molécule ayant une spécificité de liaison différente de celle de l'anticorps selon l'une quelconque des revendications 1 à 6.

9. Composition comprenant l'anticorps, le conjugué moléculaire ou la molécule bispécifique selon l'une quelconque des revendications 1 à 9, ainsi qu'un support, comprenant éventuellement en outre un adjuvant et/ou un ou plusieurs autres anticorps.

10. Composition selon la revendication 9, dans laquelle le ou les autres anticorps se lient à CTLA-4, PD-1, PD-L1, LAG-3, TIM-3, Galectine 9, CEACAM-1, BTLA, CD69, Galectine 1, TIGIT, CD113, GPR56, VISTA, B7-H3, B7-H4, 2B4, CD48, GARP, PD1H, LAIR1, TIM-1, TIM-4, B7-1, B7-2, CD28, 4-1BB (CD137), 4-1BBL, ICOS, ICOS-L, OX40, OX40L, CD70, CD27, DR3 ou CD28H.

11. Anticorps, conjugué moléculaire, anticorps bispécifique ou composition selon l'une quelconque des revendications 1 à 10, destinés à être utilisés dans un procédé d'induction ou d'amélioration d'une réponse immunitaire contre un antigène chez un sujet, ledit procédé comprenant éventuellement en outre l'administration de l'antigène simultanément, séparément ou à la suite de l'anticorps, du conjugué moléculaire, de la composition ou de la molécule bispécifique.

12. Procédé *ex vivo* d'inhibition de la croissance de cellules exprimant CD40, comprenant la mise en contact desdites cellules avec l'anticorps, la composition ou la molécule bispécifique selon l'une quelconque des revendication 1 à 10, en une quantité efficace pour inhiber la croissance des cellules exprimant CD40.

13. Anticorps, conjugué moléculaire, anticorps bispécifique ou composition selon l'une quelconque des revendications 1 à 10, destinés à être utilisés dans un procédé de traitement d'un sujet par thérapie.

14. Anticorps, conjugué moléculaire, anticorps bispécifique ou composition selon l'une quelconque des revendications 1 à 10, destinés à être utilisés dans un procédé de traitement d'un cancer appartenant au groupe constitué de la leucémie lymphoïde chronique, du lymphome à cellules du manteau, du lymphome primitif du système nerveux central, du lymphome de Burkitt et du lymphome B de la zone marginale.

15. Anticorps, conjugué moléculaire, anticorps bispécifique ou composition destinés à être utilisés selon la revendication 13 ou 14, ledit procédé comprenant en outre l'administration d'un ou plusieurs agents thérapeutiques au sujet, ledit agent thérapeutique étant éventuellement un autre anticorps, tel qu'un anticorps anti-PD-1, un anticorps anti-PD-L1 et/ou un anticorps anti-CTLA-4, et/ou lesdits premier et deuxième anticorps étant administrés de manière concomitante ou séquentielle.
